(19) Europäisches Patentamt
European Patent Office
Office européen des brevets

(11) EP 1 254 118 B1

(12) # EUROPÄISCHE PATENTSCHRIFT

(45) Veröffentlichungstag und Bekanntmachung des Hinweises auf die Patenterteilung:
**09.11.2005 Patentblatt 2005/45**

(21) Anmeldenummer: **01901176.6**

(22) Anmeldetag: **19.01.2001**

(51) Int Cl.[7]: **C07D 215/48**, A61K 31/47, A61P 25/32, C07D 221/16, C07D 221/22, C07D 491/04, C07D 495/04

(86) Internationale Anmeldenummer:
**PCT/EP2001/000588**

(87) Internationale Veröffentlichungsnummer:
**WO 2001/058875 (16.08.2001 Gazette 2001/33)**

(54) **SUBSTITUIERTE 1,2,3,4-TETRAHYDROCHINOLIN-2-CARBONSÄUREDERIVATE**

SUBSTITUTED 1,2,3,4- TETRAHYDROQUINOLINE-2-CARBOXYLIC ACID DERIVATIVES

DERIVES SUBSTITUES D'ACIDE 1,2,3,4-TETRAHYDROQUINOLINE-2-CARBOXYLIQUE

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **07.02.2000 DE 10005302**

(43) Veröffentlichungstag der Anmeldung:
**06.11.2002 Patentblatt 2002/45**

(73) Patentinhaber: **Grünenthal GmbH**
**52078 Aachen (DE)**

(72) Erfinder:
- **GERLACH, Matthias**
**63636 Brachttal (DE)**
- **PRZEWOSNY, Michael**
**52062 Aachen (DE)**
- **ENGLBERGER, Werner**
**52223 Stolberg (DE)**
- **REISSMÜLLER, Elke**
**33617 Bielefeld (DE)**
- **BLOMS-FUNKE, Petra**
**52146 Würselen (DE)**
- **MAUL, Corinna**
**52066 Aachen (DE)**
- **JAGUSCH, Utz-Peter**
**52066 Aachen (DE)**

(56) Entgegenhaltungen:
**EP-A- 0 386 839** **WO-A-99/64411**

- **BORRIONE E. ET AL.: "Synthesis and cycloaddition reactions of ethyl glyoxylate imines. Synthesis of substituted furo[3,2-c]quinolines and 7H-indeno[2,1-c]quinolines" JOURNAL OF HETEROCYCLIC CHEMISTRY, Bd. 25, Nr. 1831, 1988, Seiten 1831-1835, XP000670083 PROVO, UT, US ISSN: 0022-152X in der Anmeldung erwähnt**
- **BORRIONE, E. ET AL.: "Diastereofacial selectivity in the cycloaddition of chiral glyoxylateimines to cyclopentadiene and indene. Synthesis of optically active tetrahydroquinolines" JOURNAL OF THE CHEMICAL SOCIETY, PERKIN TRANSACTIONS 1., Bd. 12, Nr. 9, 1989, Seiten 2245-2250, XP000926476 CHEMICAL SOCIETY. LETCHWORTH., GB ISSN: 1472-7781 in der Anmeldung erwähnt**
- **R. W. CARLING ET AL.: "2-Carboxytetrahydroquinolines. Conformational and stereochemical requirements for antagonism of the glycine site on the N-methyl-D-aspartate (NMDA) receptor" JOURNAL OF MEDICINAL CHEMISTRY., Bd. 35, Nr. 11, 1992, Seiten 1942-1953, XP001002472 AMERICAN CHEMICAL SOCIETY. WASHINGTON., US ISSN: 0022-2623 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 254 118 B1

## Beschreibung

Die vorliegende Erfindung betrifft substituierte 1,2,3,4-Tetrahydrochinolin-2-carbonsäurederivate, sowie Verfahren zu deren Herstellung, deren Verwendung zur Herstellung von Arzneimitteln und Arzneimittel enthaltend diese Verbindungen.

Die Behandlung chronischer und nicht chronischer Schmerzzustände hat in der Medizin eine große Bedeutung. Es besteht ein weltweiter Bedarf an gut wirksamen Schmerztherapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist. Dies zeigt sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Proteingekoppelten Rezeptoren gehören. Die biochemische und pharmakologische Charakterisierung von Subtypen dieser Rezeptoren hat nun die Hoffnung geweckt, daß subtypenspezifische Opioide über ein anderes Wirkungs/Nebenwirkungsprofil als z.B. Morphin verfügen. Weitere pharmakologische Untersuchungen haben inzwischen die Existenz mehrerer Subtypen dieser Opioidrezeptoren ($\mu_1$, $\mu_2$, $\kappa_1$, $\kappa_2$, $\kappa_3$, $\delta_1$ und $\delta_2$) wahrscheinlich gemacht.

Daneben gibt es weitere Rezeptoren und Ionenkanäle, die wesentlich an dem System der Schmerzentstehung und Schmerzweiterleitung beteiligt sind. Besonders wichtig ist dabei der NMDA-Ionenkanal: Über ihn läuft ein wesentlicher Teil der Komunikation von Synapsen ab. Durch diesen Kanal wird der Calcium-Ionenaustausch zwischen neuronaler Zelle und seiner Umgebung gesteuert.

Kenntnisse über die physiologische Bedeutung von Ionenkanal-selektiven Substanzen sind durch die Entwicklung der patch-clamp-Technik gewonnen worden. So läßt sich eindeutig die Wirkung von NMDA-Antagonisten auf den Einfluß von Calcium-Ionen in das Zellinnere nachweisen. Es stellte sich auch dabei heraus, daß diese Substanzen über ein eigenständiges antinociceptives Potential verfügen (z.B. Ketamin). Wichtig dabei ist, daß der Wirkmechanismus ein ganz anderer ist, wie beispielsweise bei den Opiaten, denn durch NMDA-Antagonisten wird direkt in den entscheidenden Calciumhaushalt der Zellen bei der Schmerzweiterleitung eingegriffen. Daher besteht erstmalig die Möglichkeit, die Behandlung von neuropathischen Schmerzformen erfolgreich durchzuführen.

Verschiedene NMDA-Antagonisten, wobei es sich in diesem Falle um Tetrahydrochinolinderivate handelte, wurden bereits in den Artikeln J. Med. Chem. (1992) 35, 1954-1968, J. Med. Chem. (1992) 35, 1942-1953 und Med. Chem. Res. (1991) 1; 64-73 sowie den Patentanmeldungen EP 386 839, WO 97/12870 A1, WO 98/07704 A1 und WO 98/42673 A1 beschrieben. Dabei wurde insbesondere in den Patentanmeldungen eine Vielzahl von möglichen Indikationen angegeben, unter anderen auch die Schmerztherapie. Die Wirksamkeit und Verwendbarkeit dieser Substanzen ist allerdings weiter offen, so daß hier ein Bedarf nach weiteren Substanzen besteht.

Eine der Erfindung zugrundeliegende Aufgabe bestand darin, analgetisch wirksame Substanzen, insbesondere NMDA-Antagonisten, zur Verfügung zu stellen, die sich zur Schmerztherapie - insbesondere auch chronischer und neuropathischer Schmerzen - eignen. Darüber hinaus sollten diese Substanzen möglichst wenig Nebenwirkungen wie z.B. Übelkeit, Erbrechen, Abhängigkeit, Atemdepression oder Obstipation aufweisen.

Entsprechend sind Gegenstand der Erfindung substituierte 1,2,3,4-Tetrahydrochinolin-2-carbonsäurederivate der allgemeinen Formel I, in Form ihrer Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren,

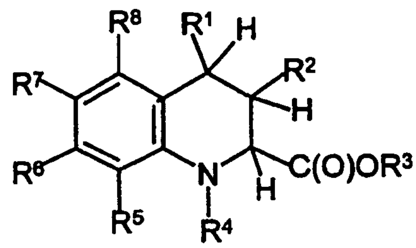

I

worin
**$R^1$** und **$R^2$** zusammen

$-(CH_2)_n-$ mit n= 3-10,
$-CH=CH-CH_2-$,
$-CH=CH-CH_2-CH_2-$,
$-CH_2-CH=CH-CH_2-$,
$-CH_2-CH=CH-CH_2-CH_2-$,
$-CH_2-CH_2-CH=CH-CH_2-CH_2-$,
$-O-CH_2-CH_2-$,
$-O-CH_2-CH_2-CH_2-$,
$-CH_2-O-CH_2-$,
$-CH_2-CH_2-O-CH_2-$,

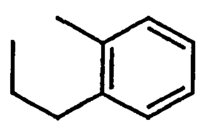

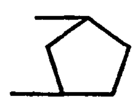

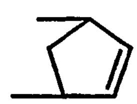

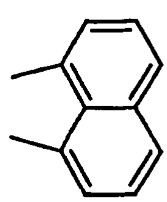

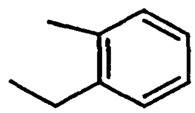

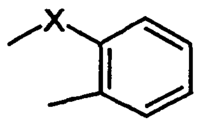

X= O, S.

bilden,

**$R^3$** ausgewählt ist aus

H; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring durch N, S oder O ersetzt ist; Alkylaryl oder Alkylheteroaryl, jeweils einfach oder mehrfach substituiert oder unsubstituiert; Aryl oder Heteroaryl, jeweils einfach oder mehrfach substituiert oder unsubstituiert;

**$R^4$** ausgewählt ist aus

$R^{4a}$ oder $ZR^{4a}$ mit Z = $C_1$-$C_6$-Alkyl, $C_2$-$C_6$-Alkenyl oder $C_2$-$C_6$-Alkinyl, jeweils verzweigt oder unverzweigt, einfach oder mehrfach substituiert oder unsubstituiert, mit $R^{4a}$ ausgewählt aus

H; $C_1$-$C_{12}$-Alkyl, $C_2$-$C_{12}$-Alkenyl oder $C_2$-$C_{12}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring durch S, O oder N ersetzt ist; Aryl oder Heteroaryl, jeweils einfach oder mehrfach substituiert oder unsubstituiert;

$C(O)R^9$, $C(O)OR^9$, $C(S)R^9$, $C(S)OR^9$ bzw. $S(O_2)R^9$ mit $R^9$ ausgewählt aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring durch S, O oder N ersetzt ist; Alkylaryl oder Alkylheteroaryl, jeweils einfach oder mehrfach substituiert oder unsubstituiert; Aryl oder Heteroaryl, jeweils einfach oder mehrfach substituiert oder unsubstituiert, insbesondere Phenethyl, 1-Adamantyl, 2-Adamantyl, 1-Naphthyl oder 2-Napthyl 2-,3- oder 4-Pyridyl; Thiazolyl;

$SR^{10}$ mit $R^{10}$ ausgewählt aus

Aryl oder Heteroaryl, jeweils einfach oder mehrfach substituiert oder unsubstituiert,

$C(O)NR^{11}R^{12}$, $C(O)NR^{11}NR^{12}R^{13}$, $C(NR^{11})NR^{12}R^{13}$, $C(S)NR^{11}R^{12}$ oder $C(S)NR^{11}NR^{12}R^{13}$, wobei $R^{11}$, $R^{12}$ und $R^{13}$ unabhängig voneinander ausgewählt sind aus

H; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring durch S, O oder N ersetzt ist, Alkylaryl oder Alkylheteroaryl, jeweils einfach oder mehrfach substituiert oder unsubstituiert; Aryl oder Heteroaryl, jeweils einfach oder mehrfach substituiert oder unsubstituiert;

**$R^5$, $R^6$, $R^7$** und **$R^8$** unabhängig voneinander ausgewählt sind aus

H, F, Cl, Br, I, CN, $NO_2$; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

$OR^{14}$, $OC(O)R^{14}$, $OC(S)R^{14}$, $C(O)R^{14}$, $C(O)OR^{14}$, $C(S)R^{14}$, $C(S)OR^{14}$, $SR^{14}$, $S(O)R^{14}$ bzw. $S(O_2)R^{14}$, wobei $R^{14}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring durch S, O oder N ersetzt ist; Alkylaryl oder Alkylheteroaryl, jeweils einfach oder mehrfach substituiert oder unsubstituiert; Aryl oder Heteroaryl, jeweils einfach oder mehrfach substituiert oder unsubstituiert;

$NR^{15}R^{16}$, $NR^{15}C(O)R^{16}$, $C(NR^{15})NR^{16}R^{17}$ $NR^{15}C(S)R^{16}$, $C(S)NR^{15}R^{16}$ oder $C(S)NR^{15}NR^{16}R^{17}$ oder $S(O_2)NR^{15}R^{16}$, wobei $R^{15}$, $R^{16}$ und $R^{17}$ unabhängig voneinander ausgewählt sind aus

H, O; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring

durch S, O oder N ersetzt ist, Alkylaryl oder Alkylheteroaryl, jeweils einfach oder mehrfach substituiert oder unsubstituiert; Aryl oder Heteroaryl, jeweils einfach oder mehrfach substituiert oder unsubstituiert;

oder

$R^{15}$ und $R^{16}$ oder $R^{16}$ und $R^{17}$ zusammen ein $C_3$-$C_8$-Cycloalkyl, gesättigt oder ungesättigt, einfach oder mehrfach substituiert oder unsubstituiert,bilden, bzw. einen entsprechenden Heterocyclus, bei dem mindestens ein C-Atom im Ring durch S, O oder N ersetzt ist; oder

**$R^5$** und **$R^6$, $R^6$** und **$R^7$** oder **$R^7$** und **$R^8$** gemeinsam

=$CR^{18}$-CH=CH-CH= oder =CH-$CR^{18}$=CH-CH= bilden, mit $R^{18}$ ausgewählt aus

H, F, Cl, Br, I, OH oder $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

, mit der Maßgabe, daß,

wenn $R^1$ und $R^2$ zusammen -CH=CH-$CH_2$- oder

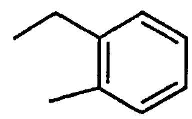

bilden und $R^3$ (-)p-Menthan-3-ol, insbesondere Menthol oder Borneol, entspricht, nicht gleichzeitig $R^7$ = Cl und $R^5$, $R^6$ und $R^8$ = H sind,

wenn $R^1$ und $R^2$ zusammen -CH=CH-$CH_2$- bilden und $R^3$ $CH_3$ entspricht, nicht gleichzeitig $R^7$ = H, Cl oder $OCH_3$ und $R^5$, $R^6$ und $R^8$ = H sind,

wenn $R^{1b}$ und $R^{2a}$ zusammen -CH=CH-$CH_2$- bilden und $R^3$ H entspricht, nicht gleichzeitig $R^7$ = $OCH_3$ oder $C(O)NH_2$ und $R^5$, $R^6$ und $R^8$ = H, $R^5$ und $R^7$ = $CH_3$ und $R^6$ und $R^8$ = H oder $R^5$ = $OCH_3$ und $R^6$, $R^7$ und $R^8$ = H sind,

wenn $R^{1b}$ und $R^{2a}$ zusammen

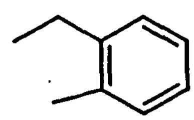

oder -O-$CH_2$-$CH_2$- bilden und $R^3$ $C_2H_5$ entspricht, nicht gleichzeitig $R^7$ = H, Cl, $CH_3$, $OCH_3$ oder $NO_2$ und $R^5$, $R^6$ und $R^8$ = H oder $R^5$ = $NO_2$ und $R^6$, $R^7$ und $R^8$ = H sind;

Die erfindungsgemäßen 1,2,3,4-Tetrahydrochinolin-2-carbonsäurederivate sind NMDA-Antagonisten, die selektiv an der Glycin-Bindungsstelle angreifen, und zeigen auch eine deutliche analgetische Wirkung.

Dabei versteht man im Zusammenhang mit Alkyl, Alkenyl, Alkinyl und Cycloalkyl bzw. dem "entsprechenden Heterocyclus" unter dem Begriff substituiert im Sinne dieser Erfindung die Substitution eines Wasserstoffrestes durch F, Cl, Br, I, $NH_2$, SH oder OH, wobei unter mehrfach substituierten Resten Reste zu verstehen sind, die sowohl an verschiedenen als auch an gleichen Atomen mehrfach substituiert sind, beispielsweise dreifach am gleichen C-Atom wie im Falle von $CF_3$ oder an verschiedenen Stellen wie im Falle von -CH(OH)-CH=CH-$CHCl_2$.

Weiter bedeutet -C(O)-

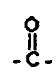

, was auch für -C(S)- oder -S(O)- bzw. -$S(O_2)$- gilt.

Der Ausdruck "$C_1$-$C_8$-Alkyl" bzw. "$C_1$-$C_{10}$-Alkyl" bedeutet im Sinne dieser Erfindung Kohlenwasserstoffe mit 1 bis 8 bzw. 10 Kohlenstoffatomen. Bespielhaft seien Methyl, Ethyl, Propyl, Isopropyl, n-Butan, sek-Butyl, tert-Butyl, n-Pentan, Neopentyl, n-Hexan, n-Heptan, n-Octan, n-Nonan oder n-Decan genannt

Der Ausdruck "$C_1$-$C_{18}$-Alkyl" bedeutet im Sinne dieser Erfindung Kohlenwasserstoffe mit 1 bis 18 Kohlenstoffatomen. Beispielhaft seien Methyl, Ethyl, Propyl, isopropyl, n-Butan, sek-Butyl, tert-Butyl, n-Pentan, Neopentyl, n-Butan, sek-Butyl, tert-Butyl, n-Hexan, n-Heptan, n-Octan, n-Nonan, n-Decan, n-Undecan, n-Dodecan, n-Dodecan, n-Tridecan, n-Tetradecan, n-Pentadecan, n-Hexadecan, n-Heptadecan oder n-Octadecan unsubstituiert oder ein oder mehrfach substituiert genannt.

Der Ausdruck "$C_2$-$C_{10}$-Alkenyl" bzw. "$C_2$-$C_{10}$-Alkinyl" oder "$C_2$-$C_{18}$-Alkenyl" bzw. "$C_2$-$C_{18}$-Alkinyl" bedeutet

im Sinne dieser Erfindung Kohlenwasserstoffe mit 2 bis 8 bzw. 2 bis 18 Kohlenstoffatomen. Beispielhaft genannt seien Methenyl, Propenyl, Butenyl, Pentenyl, Hexenyl, Heptenyl, Octenyl unsubstituiert oder ein oder mehrfach substituiert bzw. Methinyl, Propinyl, Butinyl, Pentinyl, Hexinyl, Heptinyl, Octinyl unsubstituiert oder ein oder mehrfach substituiert.

Der Ausdruck $C_3$-$C_7$-Cycloalkyl bedeutet im Sinne dieser Erfindung cyclische Kohlenwasserstoffe mit 3 bis 7 Kohlenstoffatomen. Beispielhaft seien Cyclopropyl, Cyclobutyl, Cyclopentyl, Cyclohexyl, Cycloheptyl, Cyclopentenyl, Cyclohexenyl oder Cycloheptenyl gesättigt oder ungesättigt, unsubstituiert oder ein oder mehrfach substituiert genannt. Dabei versteht man im Sinne der Erfindung unter einem einem "entsprechenden Heterocyclus" ein $C_3$-$C_7$-Cycloalkyl, bei dem mindestens ein C-Atom im Ring durch S, O oder N ersetzt ist. Beispielhaft seien hierfür Pyrrolidin, Pyran, Thiolan, Piperidin oder Tetrahydrofuran aufgeführt.

Der Ausdruck "Aryl" bedeutet im Sinne dieser Erfindung Phenyle, Naphthyle oder Anthracenyle. Die Aryl-Reste können auch mit weiteren Ringen kondensiert sein.

Der Ausdruck "Heteroaryl" bedeutet im Sinne dieser Erfindung gegebenenfalls mit einem ankondensierten Ringsystem versehene, aromatische Verbindungen, die wenigstens ein Heteroatom aus der Gruppe Stickstoff, Sauerstoff und/oder Schwefel enthalten. In dieser Gruppe seien beispielhaft Thiophen, Furan, Pyrrol, Pyridin, Pyrimidin, Chinolin, Isochinolin, Phtlalazin oder Chinazolin aufgeführt.

Der Ausdruck "Alkylaryl" bzw. "Alkylheteroaryl" bedeutet im Sinne dieser Erfindung mindestens mit $C_1$-$C_6$-Alkylen substituierte Aryle bzw. Heteroaryle, wobei die Ausdrücke Aryl, Heteroaryl und Alkyl die gleiche Bedeutung haben wie oben, in denen die Bindung über den Alkylrest erfolgt.

In Bezug auf "Aryl", "Alkylaryl", "Heteroaryl" oder "Alkylheteroaryl" versteht man im Sinne dieser Erfindung unter ein- oder mehrfach substituiert die Substitution des Ringsystems mit F, Cl, Br, I, $NH_2$, SH, OH, $CF_3$; =O oder =S; ein- oder mehrfach substituiertem oder unsubstituiertem $C_1$-$C_6$-Alkyl, $C_1$-$C_6$-Alkoxy, $C_2$-$C_8$-Alkenyl, $C_2$-$C_8$-Alkinyl; Phenyl oder Benzyl; oder Dioxolyl; an einem oder verschiedenen Atomen.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid.

Ein besonders bevorzugter Gegenstand der Anmeldung sind erfindungsgemäße substituierte 1,2,3,4-Tetrahydrochinolin-2-carbonsäurederivate gemäß Formel I, in denen $R^4$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert;

C(O)$R^8$ mit $R^9$ ausgewählt aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert; Aryl oder Heteroaryl, jeweils einfach oder mehrfach substituiert oder unsubstituiert, insbesondere Phenethyl, 1-Adamantyl, 2-Adamantyl, 1-Naphthyl oder 2-Napthyl 2-,3- oder 4-Pyridyl; Thiazolyl.

Dabei sind besonders bevorzugt erfindungsgemäße substituierte 1,2,3,4-Tetrahydrochinolin-2-carbonsäurederivate gemäß Formel I, in denen $R^4$ ausgewählt ist aus

H; $C_1$-$C_{10}$- Alkyl, unsubstituiert oder einfach oder mehrfach substituiert; Phenyl, unsubstituiert oder einfach oder mehrfach substituiert; vorzugsweise H, $CH_3$ oder $C_2H_5$, insbesondere H.

Ein bevorzugter Gegenstand der Anmeldung sind erfindungsgemäße substituierte 1,2,3,4-Tetrahydrochinolin-2-carbonsäurederivate gemäß Formel I, in denen $R^3$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring durch N oder O ersetzt ist; Alkylaryl, einfach oder mehrfach substituiert oder unsubstituiert; Aryl oder Heteroaryl, jeweils einfach oder mehrfach substituiert oder unsubstituiert

Dabei sind besonders bevorzugt erfindungsgemäße substituierte 1,2,3,4-Tetrahydrochinolin-2-carbonsäurederivate gemäß Formel I, in denen $R^3$ ausgewäht ist aus

H; $C_1$-$C_4$-Alkyl, verzweigt oder unverzweigt, einfach oder mehrfach substituiert oder unsubstituiert; Phenyl, Benzyl, oder Phenethyl, einfach oder mehrfach substituiert oder unsubstituiert, vorzugsweise H, $CH_3$ oder $C_2H_5$, insbesondere H.

Besonders bevorzugt sind erfindungsgemäße substituierte 1,2,3,4-Tetrahydrochinolin-2-carbonsäurederivate gemäß Formel I, in denen $R^1$ und $R^2$ zusammen

$-O-CH_2-CH_2-$, $(-CH_2-)_n$ mit n=3-6, vorzugsweise 3 oder 6, $-CH=CH-CH_2-$, $-CH=CH-CH_2-CH_2-$,

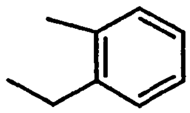, 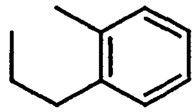, 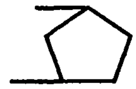,

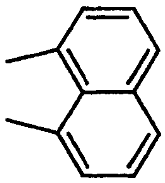, 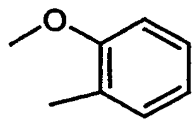 oder 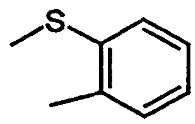,

vorzugsweise $-CH{=}CH{-}CH_2-$ oder $-CH{=}CH{-}CH_2{-}CH_2-$, insbesondere $-CH{=}CH{-}CH_2-$, bilden.

Ein bevorzugter Gegenstand der Anmeldung sind erfindungsgemäße substituierte 1,2,3,4-Tetrahydrochinolin-2-carbonsäurederivate gemäß Formel 1, in denen $R^5$, $R^6$, $R^7$ und $R^8$ unabhängig voneinander ausgewählt sind aus

H, F, Cl, Br, I, CN, $NO_2$; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

$OR^{14}$, $C(O)R^{14}$, $C(O)OR^{14}$ oder $SR^{14}$, wobei $R^{14}$ ausgewählt ist aus H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_8$-Cycloalkyl, gesättigt oder ungesättigt, einfach oder mehrfach substituiert oder unsubstituiert, bzw. einem entsprechenden Heterocyclus, bei dem mindestens ein C-Atom im Ring durch S, O oder N ersetzt ist; Alkylaryl oder Alkylheteroaryl, jeweils einfach oder mehrfach substituiert oder unsubstituiert; Aryl oder Heteroaryl, jeweils einfach oder mehrfach substituiert oder unsubstituiert;

$NR^{15}R^{16}$, $NR^{15}C(O)R^{16}$, wobei $R^{15}$ und $R^{16}$ unabhängig voneinander ausgewählt sind aus

H, O; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert.

Dabei sind besonders bevorzugt erfindungsgemäße substituierte 1,2,3,4-Tetrahydrochinolin-2-carbonsäurederrvate gemäß Formel I, in denen $R^5$, $R^6$, $R^7$ und $R^8$ unabhängig voneinander ausgewählt sind aus

H, F, Cl, Br, I, CN, $NO_2$; $C_1$-$C_6$-Alkyl, $C_2$-$C_6$-Alkenyl oder $C_2$-$C_6$-Alkinyl, jeweils verzweigt oder unverzweigt, einfach oder mehrfach substituiert oder unsubstituiert;

$OR^{14}$, $C(O)R^{14}$, $C(O)OR^{14}$ oder $SR^{14}$, wobei $R^{14}$ ausgewählt ist aus

H; $C_1$-$C_4$-Alkyl, verzweigt oder unverzweigt, einfach oder mehrfach substituiert oder unsubstituiert; Aryl, einfach oder mehrfach substituiert oder unsubstituiert,

vorzugsweise $R^5$, $R^6$, $R^7$ und $R^8$ unabhängig voneinander ausgewählt sind aus

H, F, Cl, Br, I, CN; $C_1$-$C_4$-Alkyl, verzweigt oder unverzweigt, einfach oder mehrfach substituiert oder unsubstituiert;

$OR^{14}$ oder $SR^{14}$, mit $R^{14}$ ausgewählt aus

$C_1$-$C_4$-Alkyl, verzweigt oder unverzweigt, einfach oder mehrfach substituiert oder unsubstituiert; Aryl, einfach oder mehrfach substituiert oder unsubstituiert,

insbesondere $R^5$, $R^9$, $R^7$ und $R^8$ unabhängig voneinander ausgewählt sind aus

H, F, Cl, Br, I, CN; $CH_3$, $CF_3$, t-Butyl, i-Butyl, $-OCH_3$, $-OCF_3$, $-SCH_3$, -O-Phenyl.

Dabei sind ganz besonders erfindungsgemäße substituierte 1,2,3,4-Tetrahydrochinolin-2-carbonsäurederivate gemäß Formel I bevorzugt, in denen

$R^5$, $R^6$ und $R^8$ H sowie $R^7$ Cl oder

$R^5$ und $R^7$ H sowie $R^6$ und $R^8$ Cl bedeuten.

Bevorzugte Gegenstände sind insbesondere die folgenden erfindungsgemäßen substituierten 1,2,3,4-Tetrahydrochinolin-2-carbonsäurederivate:

7,9-Dichlor-3a,4,5,9b-tetrahydro-3H-cyclopenta[c]chinolin-4-carbonsäureethylester,

7,9-Dichlor-3a,4,5,9b-tetrahydro-3H-cyclopenta[c]chinolin-4-carbonsäure,

8-Chlor-3a,4,5,9b-tetrahydro-3H-cyclopenta[c]chinolin-4-carbonsäureethylester,

8-Chlor-3a,4,5,9b-tetrahydro-3H-cyclo-penta[c]chinolin-4-carbonsäure,

2-Phenoxy-5,6a,11,11a-tetrahydro-6H-inden[1,2-c]chinolin-6-carbonsäure-ethylester,

1,3-Dichlor-5,6,6a,7,8,12b-hexahydrobenzo[k]phenanthridin-6-carbonsäure-ethylester,

7,9-Dichlor-2,3,3a,4,5,9b-hexahydro-1*H*-cyclopenta[*c*]chinolin-4-carbonsäure-ethylester,

1,3-Dichlor-7,10-methano-5,6,6a,7,8,9,10,10a-octahydrophenanthridin-6-carbon-säureethylester,

5,6a,7,11b-Tetrahydro-6*H*-indeno-[2,1-*c*]chinolin-6-carbonsäureethylester

10,12-Dichlor-6b,7,8,12b-tetrahydro-8-azabenzo[*j*]fluoranthren-7-carbonsäureethyl-ester,

1,3-Dichlor-5,6,6a,11a-tetrahydro-11-oxa-5-aza-benzo[*a*]fluoren-6-carbonsäureethyl-ester,

1,3-Dichlor-5,6,6a,11a-tetrahydro-11-thia-5-aza-benzo[*a*]fluoren-6-carbonsäureethyl-ester,

6,8,9-Trichlor-2,3,3a,4,5,9b-hexahydro-furo[3,2-c]chinolin-4-carbonsäure

2-Trifluormethoxy-5,6,6a,7,8,9,10,11,12,12a-decahydro-5-aza-cycloocta[a]naphthalen-6-carbonsäure ethyl ester

2-Cyano-5,6a,7,11b-tetrahydro-6H-indeno[2,1-c]chinolin-6-carbonsäureethy ester

1,3-Dichlor-5,6,6a,7,8,12b-hexahydro-benzo[k]phenanthridine-6-carbonsäure

1,3-Dichlor-5,6a,7,11b-tetrahydro-6H-indeno[2,1-c]chinolin-6-carbonsäure

Besonders bevorzugt sind erfindungsgemäße substituierte 1,2,3,4-Tetrahydrochinolin-2-carbonsäurederivate in Form ihrer Hydrochlorid-Salze.

Ein weiterer Gegenstand der Erfindung sind auch Verfahren zur Herstellung erfindungsgemäßer substituierter 1,2,3,4-Tetrahydrochinolin-2-carbonsäurederivate.

In der Literatur sind verschiedene Verfahren zur Darstellung von Tetrahydrochinolinen beschrieben:

- ein Festphasen-Ansatz (WO 98/34111),
- mehrstufige Prozessführungen (WO 98/42673; Bioorganic and Medicinal Chemistry Lettetters Vol. 2, S. 371, 1992; Journal of Heterocyclic Chemistry Vol. 25, S. 1831, 1988; Journal of the Chemical Society, Perkin Transactions I (1989), Seite 2245) oder
- ein Lewis-Säure-katalysiertes "Eintopf"-Verfahren (Journal of the Chemical Society, Chemical Communications, 1999, S. 651; Journal of the American Chemical Society, Vol. 118, S. 8977, 1996).

Alle diese Verfahren weisen aber klar einige Nachteile auf.

Abweichend von diesen ist das hier beschriebene sog. Grundverfahren ein Trifluoressigsäure vermitteltes - vorzugsweise "Eintopf"- Verfahren, bei dem je eine aromatische Amin-, Aldehyd- und elektronenreiche Olefinkomponente miteinander reagieren.

Zunächst werden in dem Grundverfahren substituierte 1,2,3,4-Tetrahydrochinolin-2-carbonsäurederivate gemäß Formel I mit $R^4$ = H, während die anderen Reste eine der bereits genannten Bedeutungen haben, hergestellt. Dabei werden Aniline gemäß Formel II, in denen $R^5$, $R^6$, $R^7$ und $R^8$ eine der bereits angegebenen Bedeutungen haben,

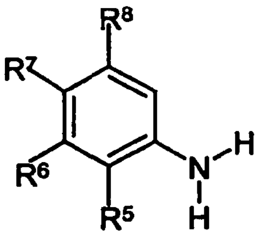

+

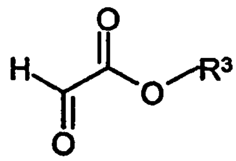

+

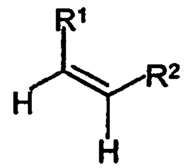

II III IV

mit Glyoxalsäureester gemäß Formel III und Olefinen gemäß IV, in denen $R^1$ und $R^2$ eine der bereits angegebenen Bedeutungen haben, mit Trifluoressigsäure zwischen 0°C und 100°C umgesetzt werden. Dabei ist es bevorzugt, daß die Reaktionsdauer 0.25 - 12 h, vorzugsweise maximal 2h, beträgt, die Reaktion bevorzugt bei Temperatur zwischen 20 und 40°C, vorzugsweise Raumtemperatur, erfolgt und/oder die Reaktion eine Eintopfreaktion ist.

Ein entscheidender Vorteil des erfindungsgemäßen Verfahrens ist, daß das Verfahren gemäß einer Dominoreaktion (Iminbildung und nachgeschaltete Aza-Diels-Alder-Reaktion) sehr selektiv bei zudem guten Ausbeuten zu den gewünschten Systemen führt.

Ohne einen Knüpfungs- bzw. Abspaltungsschritt durchführen zu müssen, wie im Falle des solid phase-Ansatzes, ferner ohne Aufreinigung der Zwischenstufen - wie im Falle der beschriebenen Lösungschemie - unterscheidet sich das erfindungsgemäße Verfahren neben seiner einfachen Durchführbarkeit ferner durch seine Aufreinigungmethode. Durch mehrmaliges Waschen mit unpolaren Lösungsmitteln, wie beispielsweise nHexan lassen sich größtenteils die Produkte in hoher Reinheit erhalten. Anderenfalls gelingt ihre Aufreinigung mittels Säulenchromatographie. Insbesondere lassen sich Verbindungen der Formel **I** durch die Waschprozesse mit unpolaren Lösungsmitteln - wie beispielsweise *n*Hexan - oder durch Kristallisation ihrer Salze, beispielsweise der Hydrochloride, diastereomerenrein erhalten.

Die meisten der hier eingesetzten Reagenzien, insbesondere nach Formel II, III und IV sind käuflich zu erwerben oder können durch einfache, dem Fachmann bekannte Syntheseschritte hergestellt werden.

Im Anschluß an das Grundverfahren können in Folgereaktionen die gemäß dem Grundverfahren entstandenen Produkte gemäß dem Fachmann bekannter Vorgehensweise zu erfindungsgemäßen Folgeprodukten gemäß Formel I umgesetzt werden, wobei zunächst der Wasserstoff an $R^4$ substituiert wird.

So kann, wenn das Produkt substituierte 1,2,3,4-Tetrahydrochinolin-2-carbonsäurederivate gemäß Formel I mit $R^4$ = Alkylformyl, Acyl, Sulfenyl und Sulfonyl sein sollen, nach Ablauf der Grundreaktion das Reaktionsprodukt mit entsprechenden Chlor- oder Fluorformiaten, Säurechloriden, Sulfenylchloriden und Sulfonylchloriden in Gegenwart einer Base, vorzugsweise Triethylamin, Pyridin oder NaOH in Wasser, Dioxan-Wasser- oder THF-Wasser-Gemischen bei einer Temperatur zwischen 0-20°C umgesetzt werden (J. Org. Chem. 1989, 54, 5574-5580).

Ebenso kann, wenn das Produkt substituierte 1,2,3,4-Tetrahydrochinolin-2-carbonsäurederivate gemäß Formel I mit $R^4 = CSNR^{17}$ sein sollen, nach Ablauf der Grundreaktion das Reaktionsprodukt mit einem Thionierungsreagenz, vorzugsweise Lawessons Reagenz (2,4-Bis(4-mathoxyphenyl)-2,4-dithioxo-1,3,2,4-dithiaphosph-etan), in organischen Lösungsmitteln, vorzugsweise THF oder Toluol bei einer Temperatur von 30-50°C umgesetzt werden.

Oder es kann, wenn das Produkt substituierte 1,2,3,4-Tetrahydrochinolin-2-carbonsäurederivate gemäß Formel I mit $R^4 = C(O)N^{13}R^{14}$ oder $C(S)N^{13}N^{14}$ sein sollen, nach Ablauf der Grundreaktion das Reaktionsprodukt mit Kaliumcyanat oder Kaliumisothiocyanat in Wasser bei Temperaturen bis zu 100°C bzw. mit organischen Isocyanaten oder Isothiocyanaten in Alkoholen, vorzugsweise Methanol, Ethanol oder Isopropanol bei Temperaturen bis zur Siedetemperatur, umgesetzt werden.

Weiter kann, wenn das Produkt substituierte 1,2,3,4-Tetrahydrochinolin-2-carbonsäurederivate gemäß Formel I mit $R^4 = C(NR^{13})NR^{14}R^{15}$ sein sollen, nach Ablauf der Grundreaktion das Reaktionsprodukt unter alkalischen Bedingungen mit O-Methylisoharnstoffen oder S-Methylisothio-harnstoffen bei Temperaturen von 20-50°C, vorzugsweise ethanolische oder methanolische NaOH oder KOH umgesetzt werden.

Weiter kann auch, wenn das Produkt substituierte 1,2,3,4-Tetrahydrochinolin-2-carbonsäurederivate gemäß Formel I mit $R^4 = C(O)NR^{13}R^{14}$ sein sollen, nach Ablauf der Grundreaktion das Reaktionsprodukt in Wasser/Eisessig bei 30-60°C mit Propanon-2-semicarbazon umgesetzt werden.

Ebenso kann, wenn das Produkt substituierte 1,2,3,4-Tetrahydrochinolin-2-carbonsäurederivate gemäß Formel I mit $R^4 = C(S)NR^{13}R^{14}$ sein sollen, nach Ablauf der Grundreaktion das Reaktionsprodukt in Wasser/NaOH bei

30-60°C mit $CS_2$ und Hydrazinen umgesetzt werden.

Als letzte hier zu nennende Möglichkeit kann, wenn das Produkt substituierte 1,2,3,4-Tetrahydrochinolin-2-carbonsäurederivate gemäß Formel I mit $R^4$ = Alkyl, Benzyl oder Phenethyl sein sollen, nach Ablauf der Grundreaktion das Reaktionsprodukt mit einem entsprechenden Alkylierungshalogenid, Benzylhalogenid oder Phenethylhalogenid und einer geeigneten Base, vorzugsweise Natriumhydrid oder Kalium-tert-butylat, in einem Lösungsmittel, beispielsweise Ethanol zwischen 0 und 100°C umgesetzt wird (J. Org. Chem. 1947, 12, 760; Zh. Obshch. Khim 1942, 12, 418.

Es kann auch günstig sein, zur Herstellung substituierter 1,2,3,4-Tetrahydrochinolin-2-carbonsäurederivate gemäß Formel I mit $R^3$ = H für das Grundverfahren Ausgangsprodukte gemäß Formel III zu verwenden, in denen $R^3 \neq$ H und $R^3$ vorzugsweise Alkyl, insbesondere $CH_3$ und $C_2H_5$ sind, einzusetzen. Nach dem Grundverfahren und auch den möglicherweise daran anschließenden Folgereaktionen wird das Reaktionsprodukt mit einer entsprechenden Base, vorzugsweise mit 6N NaOH in Ethanol, bei Temperaturen zwischen O-100 °C verseift (Organikum, 1990, S. 418).

Unter vielen der genannten Reaktionsbedingungen können OH- SH und $NH_2$-Gruppen möglicherweise unerwünschte Nebenreaktionen eingehen. Es ist daher bevorzugt, diese mit Schutzgruppen zu versehen oder im Falle von $NH_2$ durch $NO_2$ zu ersetzen und vor der Aufreinigung des Endprodukts die Schutzgruppe abzuspalten, bzw. die $NO_2$-Gruppe zu reduzieren. Ein weiterer Gegenstand der Anmeldung ist daher eine Abwandlung der oben beschriebenen Verfahrens, bei dem in den Ausgangsverbindungen mindestens eine OH-Gruppe durch eine $OSi(Ph)_2$tert-Butyl-Gruppe, mindestens eine SH-Gruppe durch eine S-p-Methoxybenzylgruppe und/oder mindestens eine $NH_2$-Gruppe durch eine $NO_2$-Gruppe ersetzt wurde und vor der Aufreinigung des Endprodukts mindestens eine - vorzugsweise alle - $OSi(Ph)_2$tert-Butyl-Gruppe/n, mit Tetrabutylammoniumfluorid in Tetrahydrofuran und/oder mindestens eine - vorzugsweise alle - p-Methoxybenzylgruppe/n mit einem Metallamin, bevorzugt Natriumamin, abgespalten und/oder mindestens eine - vorzugsweise alle - $NO_2$-Gruppe/n zu $NH_2$ reduziert wird.

Weiter sind Carbonsäure- oder Thiocarbonsäure-Gruppen unter den genannten Reaktionsbedingungen unter Umständen nicht stabil, so daß es bevorzugt ist, deren Methylester in den Reaktionen einzusetzen und das Verfahrensprodukt anschließend mit KOH-Lösung bzw. NaOH-Lösung in Methanol bei 40°C - 60°C zu verseifen. Ein weiterer Gegenstand der Erfindung ist daher eine Abwandlung der oben beschriebenen Verfahren, in dem vor der Aufreinigung des Endprodukts ein Verfahrensprodukt mit mindestens einer $C(O)OCH_3$- $OC(O)OCH_3$- und/oder $C(S)OCH_3$-Gruppe mit KOH-Lösung bzw. NaOH-Lösung in Methanol bei 40°C - 60°C verseift wird.

Die erfindungsgemäßen substituierten 1,2,3,4-Tetrahydrochinolin-2-carbonsäurederivate sind toxikologisch unbedenklich, so daß sie sich als pharmazeutischer Wirkstoff in Arzneimitteln eignen.

Ein weiterer Gegenstand der Erfindung ist daher auch ein Arzneimittel enthaltend als Wirkstoff mindestens ein erfindungsgemäßes substituiertes 1,2,3,4-Tetrahydrochinolin-2-carbonsäurederivat gemäß Formel I auch in Form seiner Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren, insbesondere des Hydrochloridsalzes.

Bevorzugt sind dabei Arzneimittel, die als als Wirkstoff mindestens ein substituiertes 1,2,3,4-Tetrahydrochinotin-2-carbonsäurederivat gemäß Formel I enthalten, worin

$R^5$, $R^6$ und $R^8$ H sowie $R^7$ Cl oder

$R^5$ und $R^7$ H sowie $R^8$ und $R^8$ Cl bedeuten.

Besonders bevorzugt sind Arzneimittel, die mindestens eine der folgenden erfindungsgemäßen substituierten 1,2,3,4-Tetrahydrochinolin-2-carbonsäurederivate enthalten:

7,9-Dichlor-3a,4,5,9b-tetrahydro-3H-cyclopenta[c]chinolin-4-carbonsäureethylester,

7,9-Dichlor-3a,4,5,9b-tetrahydro-3H-cyclopenta[c]chinolin-4-carbonsäure,

8-Chlor-3a,4,5,9b-tetrahydro-3H-cyclopenta[c]chinolin-4-carbonsäureethylester,

8-Chlor-3a,4,5,9b-tetrahydro-3H-cyclo-penta[c]chinolin-4-carbonsäure,

2-Phenoxy-5,6a,11,11a-tetrahydro-6H-inden[1,2-c]chinolin-6-carbonsäure-ethylester,

1,3-Dichlor-5,6,6a,7,8,12b-hexahydrobenzo[k]phenanthridin-6-carbonsäure-ethylester,

7,9-Dichlor-2,3,3a,4,5,9b-hexahydro- 1*H*-cyclopenta[*c*]chinolin-4-carbonsäure-ethylester,

1,3-Dichlor-7,10-methano-5,6,6a,7,8,9,10,10a-octahydrophenanthridin-6-carbon-säureethylester,

5,6a,7,11b-Tetrahydro-6*H*-indeno-[2,1-*c*]chinolin-6-carbonsäureethylester

10,12-Dichlor-6b,7,8,12b-tetrahydro-8-azabenzo[*j*]fluoranthren-7-carbonsäureethyl-ester,

1,3-Dichlor-5,6,6a,11a-tetrahydro-11-oxa-5-aza-benzo[*a*]fluoren-6-carbonsäureethyl-ester,

1,3-Dichlor-5,6,6a,11a-tetrahydro-11-thia-5-aza-benzo[*a*]fluoren-6-carbonsäureethyl-ester,

6,8,9-Trichlor-2,3,3a,4,5,9b-hexahydro-furo[3,2-c]chinolin-4-carbonsäure

2-Trifluormethoxy-5,6,6a,7,8,9,10,11,12,12a-decahydro-5-aza-cycloocta[a]naphthalen-6-carbonsäure ethyl ester

2-Cyano-5,6a,7,11b-tetrahydro-6H-indeno[2,1-c]chinolin-6-carbonsäureethy ester

1,3-Dichlor-5,6,6a,7,8,12b-hexahydro-benzo[k]phenanthridine-6-carbonsäure

1,3-Dichlor-5,6a,7,11b-tetrahydro-6H-indeno[2,1-c]chinolin-6-carbonsäure

in Form ihrer Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren, insbesondere des Hydrochloridsalzes.

Die erfindungsgemäßen Arzneimittel können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden und enthalten neben mindestens einem erfindungsgemäßen substituierten Tetrahydrochinolinderivat je nach galenischer Form gegebenenfalls Trägermaterialien Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Tetrahydrochinolinderivate in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Tetrahydrochinolinderivate verzögert freisetzen. Die an den Patienten zu verabreichende Wirkstoffmenge variiert in Abhängigkeit vom Gewicht des Patienten, von der Applikationsart, der Indikation und dem Schweregrad der Erkrankung. Üblichweise werden 2 bis 500 mg/kg wenigstens eines erfindungsgemäßen substituierten Tetrahydrochinolinderivats der Formel I appliziert.

Vorzugsweise werden die erfindungsgemäßen substituierten 1,2,3,4-Tetrahydrochinolin-2-carbonsäurederivate zur Schmerzbehandlung, insbesondere chronischer und neuropathischer Schmerzen, aber auch bei Migräne eingesetzt, so daß ein weiterer Erfindungsgegenstand die Verwendung mindestens eines erfindungsgemäßen substituierten 1,2,3,4-Tetrahydrochinolin-2-carbonsäurederivats gemäß Formel I auch in Form seiner Razemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochloridsalzes, zur Herstellung eines Arzneimittels zur Behandlung von Schmerz, insbesondere des neuropathischen und/oder chronischen Schmerzes, und/oder zur Behandlung von Migräne ist.

Aus der Affinität an den NMDA-Rezeptor ergeben sich weitere Anwendungsgebiete, da NMDA-Antagonisten bekanntermaßen u.a. eine neuroprotektive Wirkung haben und daher auch gut bei mit Neurodegeneration und -schädigung einhergehenden Krankheitsbildern, wie Morbus Parkinson und Morbus Huntington etc. eingesetzt werden können. Weitere Indikationen der erfindungsgemäßen NMDA-Antagonisten sind Epilepsie, Glaukom, Osteoporose, Ototoxizität, die mit Alkohol- und/oder Drogenmißbrauch einhergehenden Entzugserscheinungen, der Schlaganfall, sowie damit zusammenhängend cerebrale Ischämien, cerebrale Infarkten, Hirnödem, Hypoxie, Anoxie, sowie auch der Einsatz zur Anxiolyse und in der Anästhesie. Ein weiterer Gegenstand der Erfindung ist daher die Verwendung mindestens eines erfindungsgemäßen substituierten 1,2,3,4-Tetrahydrochinolin-2-carbonsäurederivats gemäß Formel I, auch in Form seiner Razemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochloridsalzes, zur Herstellung eines Arzneimittels zur Behandlung/Prophylaxe von/ bei Epilepsie, Morbus Parkinson, Morbus Huntington, Glaukom, Ototoxizität, Entzugserscheinungen bei Alkohol- und/ oder Drogenmißbrauch, Schlaganfall, cerebralen Ischämien, cerebralen Infarkten, Hirnödem, Hypoxie, Anoxie und/oder zur Anxiolyse und/oder Anästhesie.

Überraschenderweise hat es sich herausgestellt, daß die erfindungsgemäßen substituierten 1,2,3,4-Tetrahydrochinolin-2-carbonsäurederivats auch für weitere Indikationen, insbesondere zur Behandlung von Harninkontinenz, Juckreiz, Tinnitus aurium und/oder Diarrhoe sehr geeignet sind. Ein weiterer Gegenstand der Anmeldung ist daher die Verwendung mindestens eines erfindungsgemäßen substituierten 1,2,3,4-Tetrahydrochinolin-2-carbonsäurederivats gemäß Formel I, auch in Form seiner Razemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochloridsalzes, zur Herstellung eines Arzneimittels zur Behandlung von Harninkontinenz, Juckreiz, Tinnitus aurium und/oder Diarrhoe

Aber auch in andereren Indikationen sind die erfindungsgemäßen Verbindungen wirksam. Ein weiterer Gegenstand der Anmeldung ist daher die Verwendung mindestens eines erfindungsgemäßen substituierten 1,2,3,4-Tetrahydrochinolin-2-carbonsäurederivats gemäß Formel I, auch in Form seiner Razemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochloridsalzes, zur Herstellung eines Arzneimittels zur Behandlung/Prophylaxe von/bei Schizophrenie, Morbus Alzheimer, Psychosen bedingt durch erhöhten Aminosäurespiegel, AIDS-Demens, Encephalomyelitis, Tourette-Syndrom, perinataler Asphyxie inflammatorischen und allergischen Reaktionen, Depressionen, Drogen- und/oder Alkoholmißbrauch, Gastritis, Diabetes, cardiovaskulären Erkrankungen, Atemwegserkrankungen, Husten und/oder seelischen Erkrankungen.

Ein weiterer Gegenstand der Erfindung ist ein Verfahren zur Behandlung eines nichthumanen Säugetieres oder Menschen, das oder der eine Behandlung medizinisch relevanter Symptome benötigt, durch Verabreichung einer therapeutisch wiksamen Dosis eines erfindungsgemäßen substituierten 1,2,3,4-Tetrahydrochinolin-2-carbonsäurederivats gemäß Formel I, auch in Form seiner Razemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochloridsalzes, oder eines erfindungsgemäßen Arzneimittels. Die Erfindung betrifft insbesondere entsprechende Verfahren zur Behandlung von Schmerz, insbesondere von neuropathischem und/oder chronischem Schmerz und/oder zur Behandlung von Migräne zur Behandlung von Harninkontinenz, Juckreiz, Tinnitus aurium und/oder Diarrhoe, zur Behandlung/Prophylaxe von/bei Epilepsie, Morbus Parkinson, Morbus Huntington, Glaukom, Osteoporose, Ototoxizität, Entzugserscheinungen bei Alkohol- und/oder Drogenmißbrauch, Schlaganfall, cerebralen Ischämien, cerebralen Infarkten, Hirnödem, Hypoxie, Anoxie und/oder zur Anxiolyse und/oder Anästhesie oder zur Behandlung/Prophylaxe von/bei Schizophrenie, Morbus Alzheimer, Psychosen bedingt durch erhöhten Aminosäurespiegel, AIDS-Demens, Encephalomyelitis, Tourette-Syndrom, perinataler Asphyxie inflammatorischen und allergischen Reaktionen, Depressionen, Drogen- und/oder Alkoholmißbrauch, Gastritis, Diabetes, cardiovaskulären Erkrankungen, Atemwegserkrankungen, Husten und/oder seelischen Erkrankungen.

Im folgenden wird die Erfindung weiter durch Beispiele und Abbildungen erläutert, ohne sie darauf zu beschränken.

**Abbildungen**

Abbildung 1: Wirkung der erfindungsgemäßen Verbindung 2 auf die Dosiswirkungskurve von Glycin an RNA-injizierten Oozyten. Relative Amplitude: Stromamplitude, normiert auf die Antwort nach Gabe von NMDA/Glycin (100/10 µmol/l).

**Beispiele**

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die eingesetzten Chemikalien und Lösungsmittel wurden kommerziell bei den herkömmlichen Anbietem erworben (Acros, Avocado, Aldrich, Fluka, Lancaster, Maybridge, Merck, Sigma, TCI etc. oder synthetisiert).

Die dünschichtchromatographischen Untersuchungen wurden mit HPTLC-Fertigplatten, Kieselgel 60 F 254, der Firma E. Merck, Darmstadt, durchgeführt.

Die Ausbeuten der hergestellten Verbindungen sind nicht optimiert.

Die Analytik erfolgte über ESI-Massenspektroskopie.

Die Verbindungen sind numeriert, wobei die Angabe in Klammern grundsätzlich der Nummer der zugeordneten Verbindung entspricht.

**Beispiel 0**

**Grundverfahren**

a) Je ein Äquivalent Anilinderivat und Trifluoressigsäure werden unter Rühren bei Raumtemperatur in 6 ml/mmol Acetonitril gelöst und anschließend 1,1 Äquivalente Ethylglyoxalat (50 % in Toluol) bzw. 1,1 Äquivalente Glyoxalsäuremonohydrat zugegeben. Nach zehn Minuten werden hierzu 3 Äquivalente der Olefin-Komponente zugesetzt und der Verlauf der Reaktion durch Dünnschichtchromatographie verfolgt (Laufmittelsystem Diethylether / Hexan, 1:1). Die Reaktion ist nach 2 Stunden beendet (DC-Kontrolle). Der Reaktionsansatz wird mit einem Überschuß an gesättigter, wäßriger Natriumhydrogencarbonat-Lösung versetzt und die organische Phase drei Mal mit Diethylether extrahiert. Die organische Phase wird mit Wasser neutral gewaschen, über Magnesiumsulfat getrocknet, abfiltriert, mit Diethylether gewaschen und nach Einengen durch Umkristallisation bzw. Kieselgel-Chromatographie isoliert. Die Charakterisierung der 1,2,3,4-Tetrahydro-chinolin-2-carbonsäureester erfolgt durch ESI-Massenspektrometrie.

b) Optionale anschließende Darstellung der freien 1,2,3,4-Tetrahydrochinolin-2-carbonsäuren.

Der zuvor beschriebene 1,2,3,4-Tetrahydrochinolin-2-carbonsäureester (1 Äquivalent) wird in 4 ml/mmol Ethanol gelöst und unter Rühren bei Raumtemperatur mit 1,2 Äquivalenten wässriger 6N Natronlauge versetzt. Der Verlauf der Ester-Verseifung wird durch Dünnschichtchromatographie verfolgt (Laufmittelsystem Diethylether / Hexan, 1:1) und ist nach 30 Minuten beendet (DC-Kontrolle). Das Reaktionsgemisch wird am Rotationsverdampfer eingeengt, in ca. 10 ml Wasser aufgenommen und mit 32 %-iger HCl auf pH 1 eingestellt. Die wäßrige Lösung wird fünf Mal mit Diethylether extrahiert und nach Trocknen über Magnesiumsulfat eingeengt.

**Automatisiertes Verfahren**

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde in den auf 20 °C temperierten Rührblock gestellt. Anschließend wurden nacheinander die folgenden Reagenzien hinzupipettiert:

1 ml einer Lösung aus Trifluoressigsäure, 0,1 M, und Anilinkomponente,
0,1 M, in Acetonitril;
1 ml einer 0,11 M Lösung des Aldehyds in Acetonitril;
1 ml einer 0,3 M Lösung des Olefins in Acetonitril.

Das Reaktionsgemisch wurde bei 20 °C in einem der Rührblöcke 10 h lang gerührt. Danach wurde die Reaktionslösung abfiltriert. Das Röhrchen wurde dabei zweimal mit je 1,5 ml einer 7,5% $NaHCO_3$-Lösung gespült.

Das Reaktionsgemisch wurde auf einem Vortexer mit 2 ml Ethylacetat versetzt und geschüttelt. Zur Ausbildung der Phasengrenze wurde in der Zentrifuge kurz zentrifugiert. Die Phasengrenze wurde optisch detektiert und die organische Phase abpipettiert. Im nächsten Schritt wurde die wäßrige Phase erneut mit 2 ml Ethylacetat versetzt, geschüttelt, zentrifugiert und die organische Phase abpipettiert. Die vereinigten organischen Phasen wurden über 2,4 g $MgSO_4$ (granuliert) getrocknet. Das Lösungsmittel wurde in einer Vakuumzentrifuge entfernt.

Die Charakterisierung der freien 1,2,3,4-Tetrahydro-chinolin-2-carbonsäure erfolgt durch ESI-Massenspektrometrie.

**Beispiel 1**

**7,9-Dichlor-3a,4,5,9b-tetrahydro-3H-cyclopenta[c]chinolin-4-carbonsäureethylester (1)**

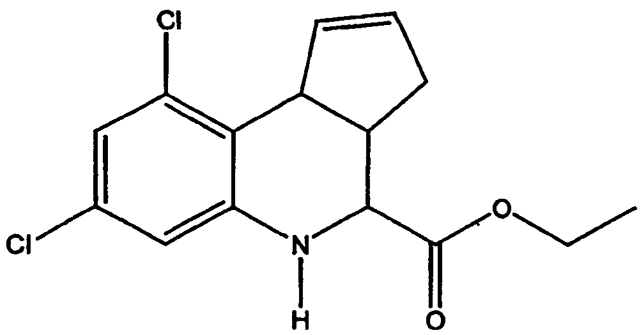

Verbindung 1 wurde gemäß Grundverfahren aus 5.0 mmol 3,5-Dichloranilin, 5.5 mmol Ethylglyoxalat-Lösung (50%, Toluol), 15.0 mmol Cyclopentadien und 5.0 mmol Trifluoressigsäure in 30.0 ml Acetonitril in 89% Ausbeute dargestellt.
Zur Charakterisierung wurde ein ESI-MS aufgenommen:

MS (EI) m/z: 311 (M*).

**Beispiel 2**

**7,9-Dichlor-3a,4,5,9b-tetrahydro-3H-cyclopenta[c]chinolin-4-carbonsäure (2)**

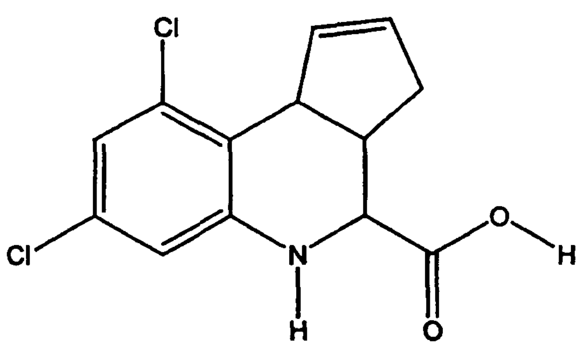

Verbindung 1 wurde mit 1.0 ml Natronlauge (6 N, Wasser) in 20.0 ml Ethanol verseift. Die ethanolische Lösung wurde am Rotationverdampfer eingeengt, der Rückstand in Wasser aufgenommen, mit 6N HCl vesetzt und die wäßrige Lösung dreimal mit Ether extrahiert. Die organische Phase wurde mit Wasser neutral gewaschen, über Magnesiumsulfat getrocknet und am Rotationsverdampfer eingeengt.
Zur Charakterisierung wurde ein ESI-MS aufgenommen:

MS (EI) m/z: 284 (M*).

**Beispiel 3**

8-Chlor-3a,4,5,9b-tetrahydro-3H-cyclopenta[c]chinolin-4-carbonsäureethylester (3)

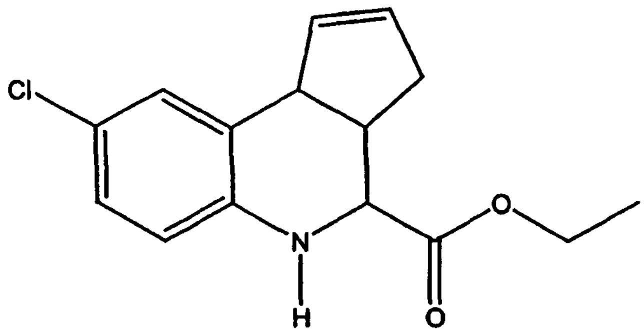

Verbindung 3 wurde gemäß dem Grundverfahren aus 5.0 mmol 4-Chloranilin, 5.5 mmol Ethylglyoxalat-Lösung (50%, Toluol), 15.0 mmol Cyclopentadien und 5.0 mmol Trifluoressigsäure in 30.0 ml Acetonitril in 89% Ausbeute dargestellt.
Zur Charakterisierung wurde ein ESI-MS aufgenommen:

MS (EI) m/z: 278(M*).

**Beispiel 4**

8-Chlor-3a,4,5,9b-tetrahydro-3H-cyclo-penta[c]chinolin-4-carbonsäure

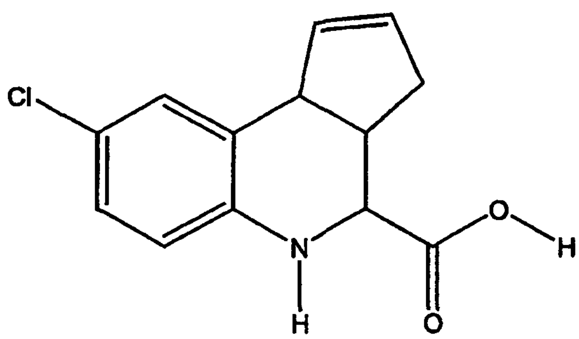

(4)

Verbindung 3 wurde mit 1.0 ml Natronlauge (6 N, Wasser) in 20.0 ml Ethanol verseift. Die ethanolische Lösung wurde am Rotationverdampfer eingeengt, der Rückstand in Wasser aufgenommen, mit 6N HCl vesetzt und die wäßrige Lösung dreimal mit Ether extrahiert. Die organische Phase wurde mit Wasser neutral gewaschen, über Magnesiumsulfat getrocknet und am Rotationsverdampfer eingeengt.
Zur Charakterisierung wurde ein ESI-MS aufgenommen:

MS (EI) m/z: 250 (M*).

**REFERENZ-Beispiel 5**

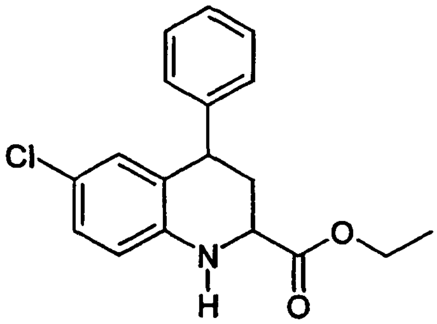

**6-Chlor-4-phenyl-1,2,3,4-tetrahydro-chinolin-2-carbonsäure- ethylester (5)**

Verbindung 5 wurde gemäß dem Grundverfahren aus 5.0 mmol 4-Chloranilin, 5.5 mmol Ethylglyoxalat-Lösung (50%, Toluol), 15.0 mmol Styrrol und 5.0 mmol Trifluoressigsäure in 30.0 ml Acetonitril in 94% Ausbeute dargestellt. Zur Charakterisierung wurde ein ESI-MS aufgenommen:

MS (EI) m/z: 315.5 (M*)

**Beispiel 6**

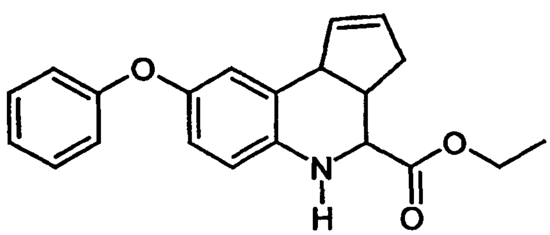

**2-Phenoxy-5,6a,11,11a-tetrahydro-6H-inden[1,2-c]chinolin-6-carbonsäureethylester (6)**

Verbindung **6** wurde gemäß dem Grundverfahren aus 5.0 mmol 4-Phenoxyanilin, 5.5 mmol Ethylglyoxalat-Lösung (50%, Toluol), 15.0 mmol Styrrol und 5.0 mmol Trifluoressigsäure in 30.0 ml Acetonitril in 72% Ausbeute dargestellt.
Zur Charakterisierung wurde ein ESI-MS aufgenommen:

MS (EI) m/z: 385.0 (M*)

**REFERENZ-Bielspiel 7**

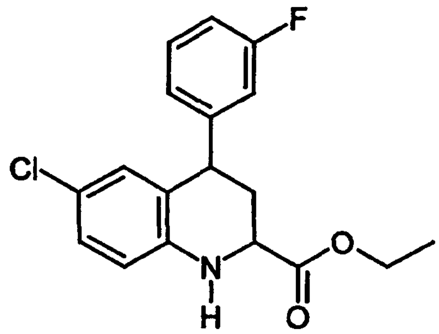

**6-Chlor-4-(3-fluor-phenyl)-1,2,3,4-tetrahydrochinolin-2-carbonsäureethylester(7)**

Verbindung **7** wurde gemäß dem Grundverfahren aus 5.0 mmol 4-Chloranilin, 5.5 mmol Ethylglyoxalat-Lösung (50%, Toluol), 15.0 mmol 3-Fluorstyrrol und 5.0 mmol Trifluoressigsäure in 30.0 ml Acetonitril in 66% Ausbeute dargestellt.
Zur Charakterisierung wurde ein ESI-MS aufgenommen:

MS (EI) m/z: 333.0 (M*)

**REFERENZ-Beispiel 8**

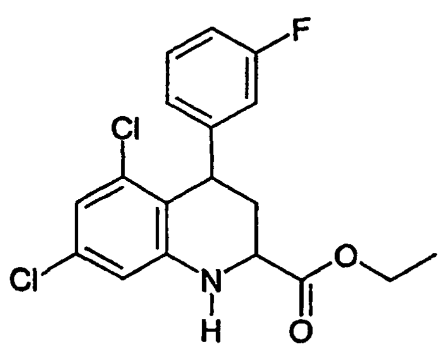

**5,7-Dichlor-4-(3-fluor-phenyl)-1,2,3,4-tetrahydro-chinolin-2-carbonsäureethylester(8)**

Verbindung **8** wurde gemäß dem Grundverfahren aus 5.0 mmol 3,5-Dichloranilin, 5.5 mmol Ethylglyoxalat-Lösung (50%, Toluol), 15.0 mmol 3-Fluorstyrrol und 5.0 mmol Trifluoressigsäure in 30.0 ml Acetonitril in 44% Ausbeute dargestellt.
Zur Charakterisierung wurde ein ESI-MS aufgenommen:

MS (EI) m/z: 367.0 (M*)

**REFERENZ-Beispiel 9**

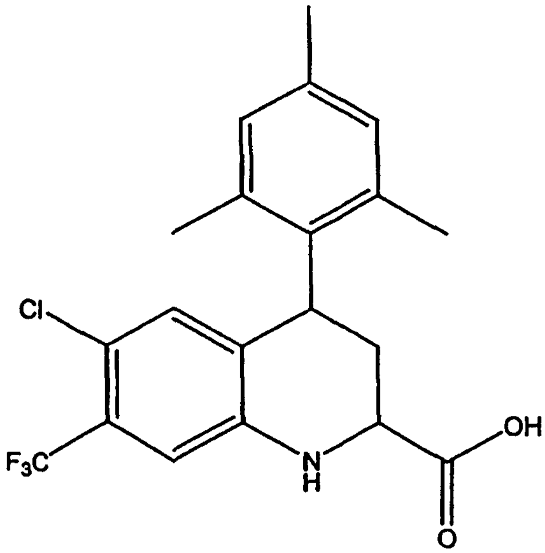

**6-Chlor-7-trifluormethyl-4-(2,4,6-trimethyl-phenyl)-1,2,3,4-tetrahydrochinolin-2-carbonsäure (9)**

Verbindung **9** wurde gemäß dem Grundverfahren aus 5.0 mmol 4-Chlor-4-trifluormethylanilin, 5.5 mmol Glyoxalsäure-Monohydrat und 15.0 mmol 2,4,6-Trimethylstyrrol in 30 ml Acetonitril dargestellt.
Zur Charakterisierung wurde ein ESI-MS aufgenommen:

MS (EI) m/z 398.1 (M*)

**REFERENZ-Beispiel 10**

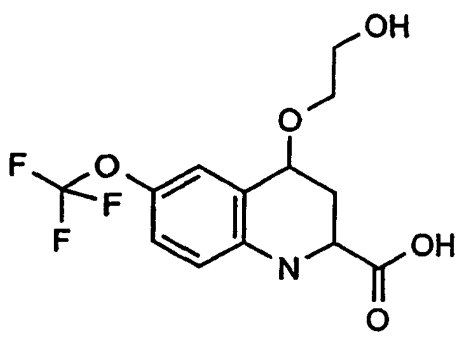

**4-(2-Hydroxy-ethoxy)-6-trifluormethoxy-1,2,3,4-tetrahydrochinolin-2-carbonsäure (10)**

Verbindung **10** wurde gemäß des automatisierten Verfahrens aus 4-(Trifluormethoxy)-anilin, Glyoxylsäure

und Ethylenglykolmonovinylether dargestellt.

**REFERENZ-Beispiel 11**

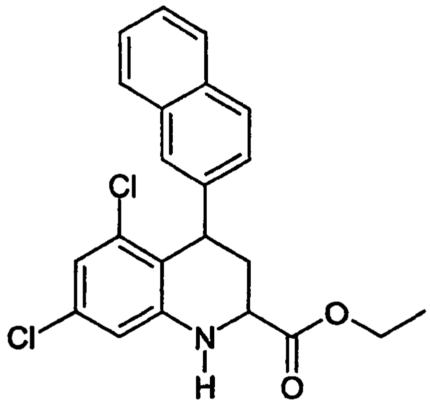

5,7-Dichlor-4-naphthalen-2-yl-1,2,3,4-tetrahydro-chinolin-2-carbonsäureethylester (11)

Verbindung **11** wurde gemäß dem Grundverfahren aus 5.0 mmol 3,5-Dichloranilin, 5.5 mmol Ethylglyoxalat-Lösung (50%, Toluol), 15.0 mmol 2-Vinylnapthalin und 5.0 mmol Trifluoressigsäure in 30.0 ml Acetonitril in 84% Ausbeute dargestellt.
Zur Charakterisierung wurde ein ESI-MS aufgenommen:

MS (EI) m/z: 399.0 (M*)

**Beispiel 12**

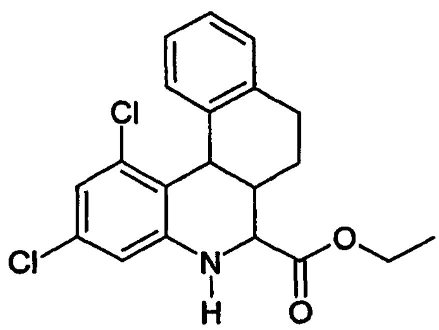

1,3-Dichlor-5,6,6a,7,8,12b-hexahydrobenzo[k]phenanthridin-6-carbonsäureethylester (12)

Verbindung **12** wurde gemäß dem Grundverfahren aus 5.0 mmol 3,5-Dichloranilin, 5.5 mmol Ethylglyoxalat-Lösung (50%, Toluol), 15.0 mmol 1,2-Dihydronaphthalin und 5.0 mmol Trifluoressigsäure in 30.0 ml Acetonitril in 85% Ausbeute dargestellt.
Zur Charakterisierung wurde ein ESI-MS aufgenommen:

MS (EI) m/z: 375.0 (M*)

**REFERENZ-Beispiel 13**

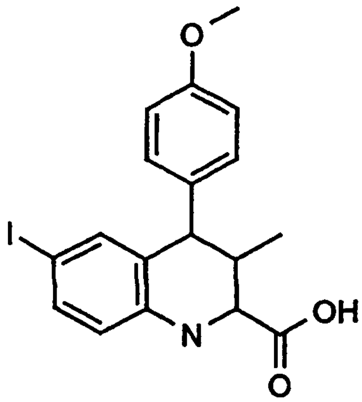

**6-Iod-4-(4-methoxy-phenyl)-3-methyl-1,2,3,4-tetrahydrochinolin-2-carbonsäure (13)**

Verbindung **13** wurde gemäß des automatisierten Verfahrens aus 4-Iodanilin, Glyoxylsäure und trans-Anethol dargestellt.

**REFERENZ Beispiel 14**

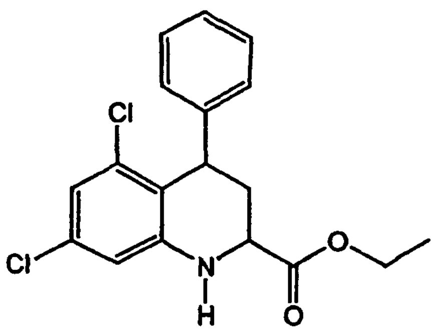

**5,7-Dichlor-4-phenyl-1,2,3,4-tetrahydrochinolin-2-carbonsäureethylester (14)**

Verbindung **14** wurde gemäß dem Grundverfahren aus 5.0 mmol 3,5-Dichloranilin, 5.5 mmol Ethylglyoxalat-Lösung (50%, Toluol), 15.0 mmol Styrol und 5.0 mmol Trifluoressigsäure in 30.0 ml Acetonitril dargestellt.
Zur Charakterisierung wurde ein ESI-MS aufgenommen:

MS (EI) m/z: (M*) 349

**REFERENZ-Beispiel 15**

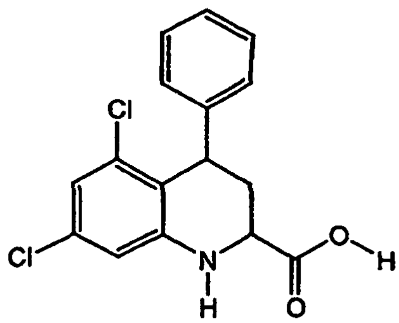

5,7-Dichlor-4-phenyl-1,2,3,4-tetrahydrochinolin-2-carbonsäure (15)

Verbindung **15** wurde gemäß dem Grundverfahren aus 5.0 mmol 3,5-Dichloranilin, 5.5 mmol Ethylglyoxalat-Lösung (50%, Toluol), 15.0 mmol Styrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*) 315

**REFERENZ-Beispiel 16**

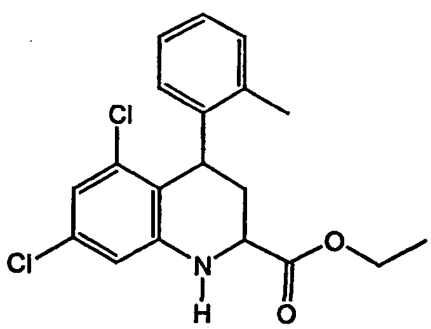

5,7-Dichlor-4-o-tolyl-1,2,3,4-tetrahydrochinolin-2 -carbonsäureethylester (16)

Verbindung **16** wurde gemäß dem Grundverfahren aus 5.0 mmol 3,5-Dichloranilin, 5.5 mmol Ethylglyoxalat-Lösung (50%, Toluol), 15.0 mmol 2-Methylstyrol und 5.0 mmol Trifluoressigsäure in 30.0 ml Acetonitril dargestellt.
Zur Charakterisierung wurde ein ESI-MS aufgenommen:

MS (EI) m/z: (M*) 363

**REFERENZ-Beispiel 17**

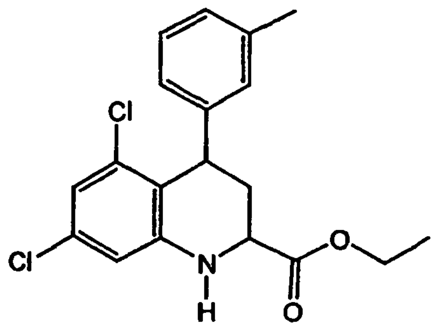

5,7-Dichlor-4-m-tolyl-1,2,3,4-tetrahydrochinolin-2-carbonsäureethyl ester (17)

Verbindung **17** wurde gemäß dem Grundverfahren aus 5.0 mmol 3,5-Dichloranilin, 5.5 mmol Ethylglyoxalat-Lösung (50%, Toluol), 15.0 mmol 3-Methylstyrol und 5.0 mmol Trifluoressigsäure in 30.0 ml Acetonitril dargestellt. Zur Charakterisierung wurde ein ESI-MS aufgenommen:

MS (EI) m/z: (M*) 363

**REFERENZ-Beispiel 18**

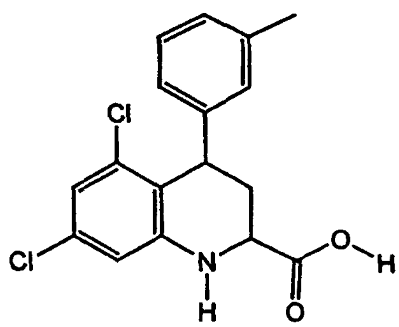

5,7-Dichlor-4-*m*-tolyl-1,2,3,4-tetrahydrochinolin-2-carbonsäure (18)

Verbindung **18** wurde gemäß dem Grundverfahren aus 5.0 mmol 3,5-Dichloranilin, 5.5 mmol Ethylglyoxalat-Lösung (50%, Toluol), 15.0 mmol 3-Methylstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*) 335

**REFERENZ-Beispiel 19**

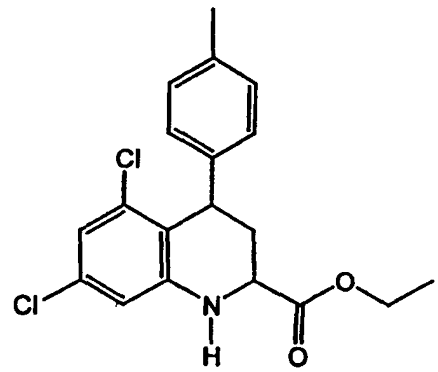

5,7-Dichlor-4-*p*-tolyl-1,2,3,4-tetrahydrochinolin-2-carbonsäureethylester (19)

Verbindung 19 wurde gemäß dem Grundverfahren aus 5.0 mmol 3,5-Dichloranilin, 5.5 mmol Ethylglyoxalat-Lösung (50%, Toluol), 15.0 mmol 4-Methylstyrol und 5.0 mmol Trifluoressigsäure in 30.0 ml Acetonitril dargestellt. Zur Charakterisierung wurde ein ESI-MS aufgenommen:

MS (EI) m/z: (M*) 363

**REFERENZ-Beispiel 20**

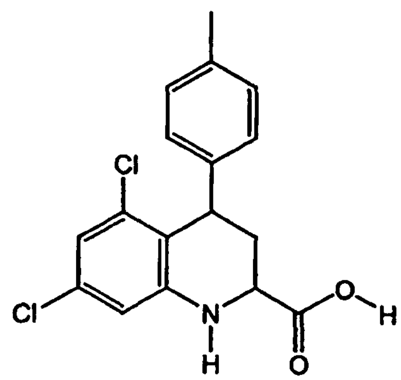

5,7-Dichlor-4-*p*-tolyl-1,2,3,4-tetrahydrochinolin-2-carbonsäure (20)

Verbindung **20** wurde gemäß dem Grundverfahren aus 5.0 mmol 3,5-Dichloranilin, 5.5 mmol Ethylglyoxalat-Lösung (50%, Toluol), 15.0 mmol 4-Methylstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*)335

**REFERENZ-Beispiel 21**

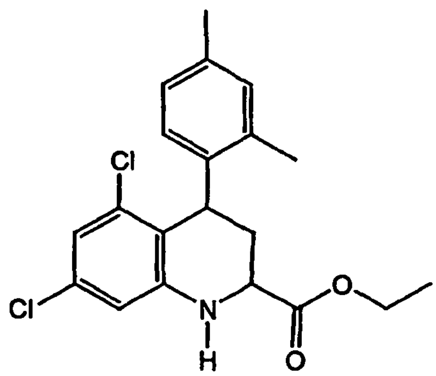

5,7-Dichlor-4-(2,4-dimethyl-phenyl)-1,2,3,4-tetrahydrochinolin-2-carbonsäureethylester (2:

Verbindung **21** wurde gemäß dem Grundverfahren aus 5.0 mmol 3,5-Dichloranilin, 5.5 mmol Ethylglyoxalat-Lösung (50%, Toluol), 15.0 mmol 2,4-Dimethylstyrol und 5.0 mmol Trifluoressigsäure in 30.0 ml Acetonitril dargestellt. Zur Charakterisierung wurde ein ESI-MS aufgenommen:

MS (EI) m/z: (M*) 377

**REFERENZ-Beispiel 22**

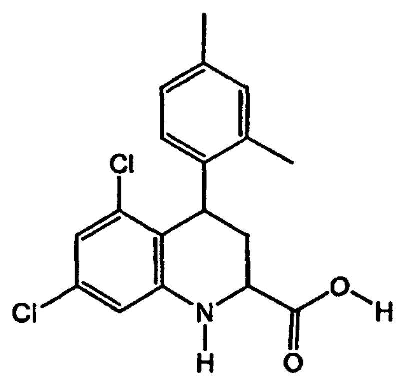

5,7-Dichlor-4-(2,4-dimethyl-phenyl)-1,2,3,4-tetrahydrochinolin-2-carbonsäure (22)

Verbindung 22 wurde gemäß dem Grundverfahren aus 5.0 mmol 3,5-Dichloranilin, 5.5 mmol Ethylglyoxalat-Lösung (50%, Toluol), 15.0 mmol 2,4-Dimethylstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*) 349

**REFERENZ-Beispiel 23**

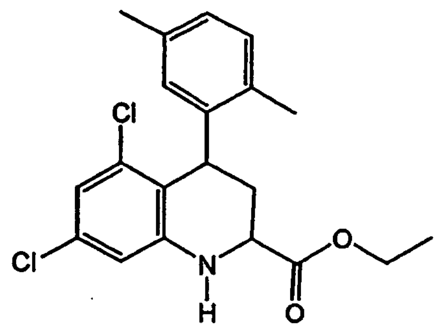

**5,7-Dichlor-4-(2,5-dimethyl-phenyl)-1,2,3,4-tetrahydrochinolin-2-carbonsäureethylester (2**

Verbindung **23** wurde gemäß dem Grundverfahren aus 5.0 mmol 3,5-Dichloranilin, 5.5 mmol Ethylglyoxalat-Lösung (50%, Toluol), 15.0 mmol 2,5-Dimethylstyrol und 5.0 mmol Trifluoressigsäure in 30.0 ml Acetonitril in 89% Ausbeute dargestellt.
Zur Charakterisierung wurde ein ESI-MS aufgenommen:

MS (EI) m/z: (M*) 377

**REFERENZ-Beispiel 24**

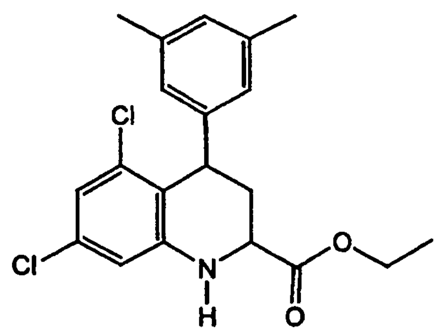

**5,7-Dichlor-4-(3,5-dimethyl-phenyl)-1,2,3,4-tetrahydrochinolin-2-carbonsäureethylester (2**

Verbindung **24** wurde gemäß dem Grundverfahren aus 5.0 mmol 3,5-Dichloranilin, 5.5 mmol Ethylglyoxalat-Lösung (50%, Toluol), 15.0 mmol 3,5-Dimethylstyrol und 5.0 mmol Trifluoressigsäure in 30.0 ml Acetonitril dargestellt.
Zur Charakterisierung wurde ein ESI-MS aufgenommen:

MS (EI) m/z: (M*) 377

**REFERENZ-Beispiel 25**

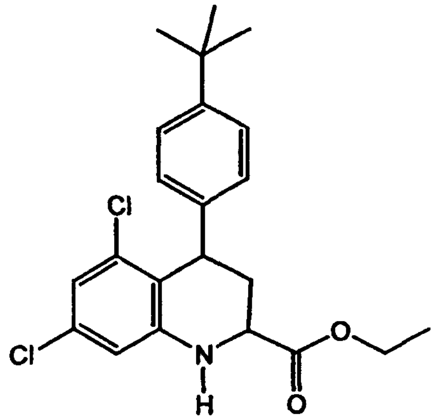

4-(4-*tert*-Butyl-phenyl)-5,7-dichloro-1,2,3,4-tetrahydrochinolin-2-carbonsäureethylester (25

Verbindung **25** wurde gemäß dem Grundverfahren aus 5.0 mmol 3,5-Dichloranilin, 5.5 mmol Ethylglyoxalat-Lösung (50%, Toluol), 15.0 mmol 4-tert-Butylstyrol und 5.0 mmol Trifluoressigsäure in 30.0 ml Acetonitril in 89% Ausbeute dargestellt.
Zur Charakterisierung wurde ein ESI-MS aufgenommen:

MS (EI) m/z: (M*) 405

**REFERENZ-Beispiel 26**

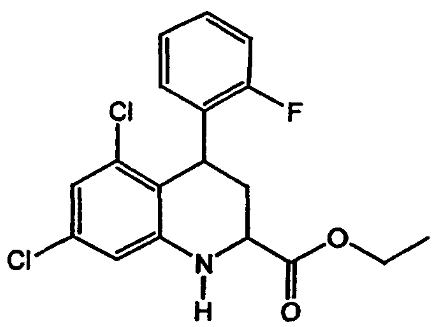

5,7-Dichlor-4-(2-fluoro-phenyl)-1,2,3,4-tetrahydrochinolin-2-carbonsäureethylester (26)

Verbindung **26** wurde gemäß dem Grundverfahren aus 5.0 mmol 3,5-Dichloranilin, 5.5 mmol Ethylglyoxalat-Lösung (50%, Toluol), 15.0 mmol 2-Fluorstyrol und 5.0 mmol Trifluoressigsäure in 30.0 ml Acetonitril dargestellt.
Zur Charakterisierung wurde ein ESI-MS aufgenommen:

MS (EI) m/z: (M*)367

**REFERENZ-Beispiel 27**

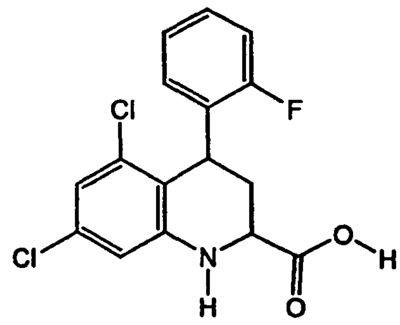

**5,7-Dichlor-4-(2-fluoro-phenyl)-1,2,3,4-tetrahydrochinolin-2-carbonsäure (27)**

Verbindung **27** wurde gemäß dem Grundverfahren aus 5.0 mmol 3,5-Dichloranilin, 5.5 mmol Ethylglyoxalat-Lösung (50%, Toluol), 15.0 mmol 2-Fluorstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*)339

**REFERENZ-Beispiel 28**

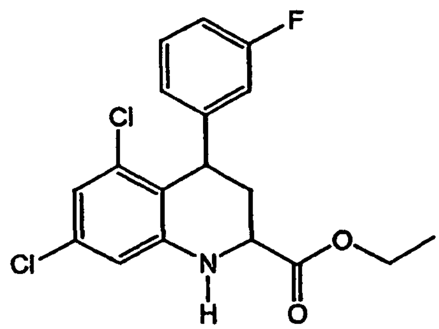

**5,7-Dichlor-4-(3-fluoro-phenyl)-1,2,3,4-tetrahydrochinolin-2-carbonsäureethylester (28)**

Verbindung **28** wurde gemäß dem Grundverfahren aus 5.0 mmol 3,5-Dichloranilin, 5.5 mmol Ethylglyoxalat-Lösung (50%, Toluol), 15.0 mmol 3-Fluorstyrol und 5.0 mmol Trifluoressigsäure in 30.0 ml Acetonitril dargestellt.
Zur Charakterisierung wurde ein ESI-MS aufgenommen:

MS (EI) m/z: (M*) 367

**REFERENZ-Beispiel 29**

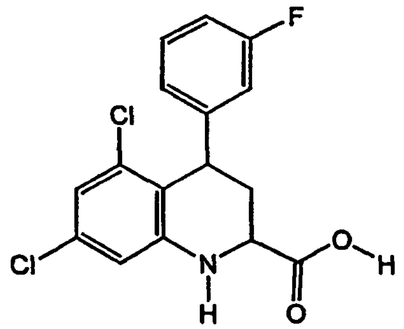

**5,7-Dichlor-4-(3-fluor-phenyl)-1,2,3,4-tetrahydrochinolin-2-carbonsäure (29)**

Verbindung **29** wurde gemäß dem Grundverfahren aus 5.0 mmol 3,5-Dichloranilin, 5.5 mmol Ethylglyoxalat-Lösung (50%, Toluol), 15.0 mmol 3-Fluorstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*) 340

**REFERENZ-Beispiel 30**

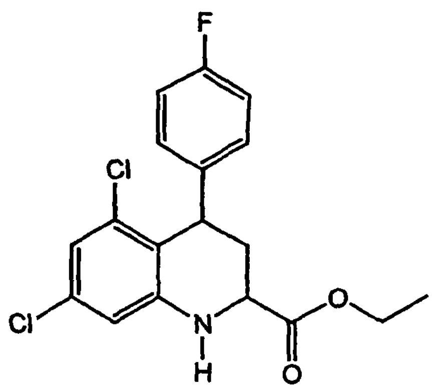

**5,7-Dichlor-4-(4-fluor-phenyl)-1,2,3,4-tetrahydrochinolin-2-carbonsäureethylester (30)**

Verbindung **30** wurde gemäß dem Grundverfahren aus 5.0 mmol 3,5-Dichloranilin, 5.5 mmol Ethylglyoxalat-Lösung (50%, Toluol), 15.0 mmol 4-Fluorstyrol und 5.0 mmol Trifluoressigsäure in 30.0 ml Acetonitril dargestellt.
Zur Charakterisierung wurde ein ESI-MS aufgenommen:

MS (EI) m/z: (M*) 367

**REFERENZ-Beispiel 31**

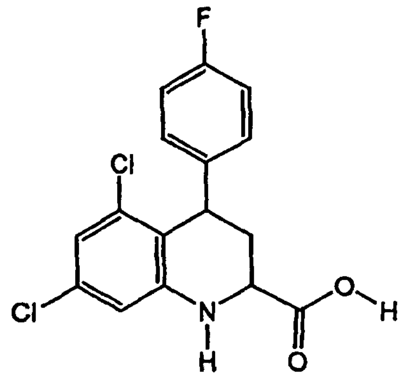

5,7-Dichlor-4-(4-fluor-phenyl)-1,2,3,4-tetrahydrochinolin-2-carbonsäure (31)

Verbindung **31** wurde gemäß dem Grundverfahren aus 5.0 mmol 3,5-Dichloranilin, 5.5 mmol Ethylglyoxalat-Lösung (50%, Toluol), 15.0 mmol 4-Fluorstyrol und 5.0 mmol Trifluoressigsäure in 30.0 ml Acetonitril dargestellt.

Die anschließende Verseifung erfolgte mit 1.0 ml Natronlauge (6 N, Wasser) in 20.0 ml Ethanol. Zur Charakterisierung wurde ein ESI-MS aufgenommen:

MS (EI) m/z: (M*)340

**REFERENZ-Beispiel 32**

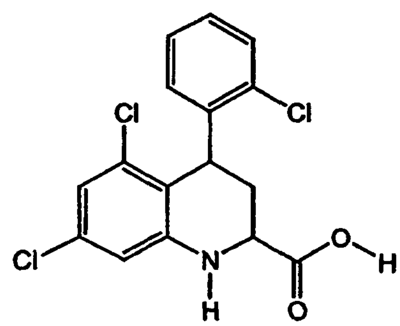

5,7-Dichlor-4-(2-chlor-phenyl)-1,2,3,4-tetrahydrochinolin-2-carbonsäure (32)

Verbindung 32 wurde gemäß dem Grundverfahren aus 5.0 mmol 3,5-Dichloranilin, 5.5 mmol Ethylglyoxalat-Lösung (50%, Toluol), 15.0 mmol 2-Chlorstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*)356

**REFERENZ-Beispiel 33**

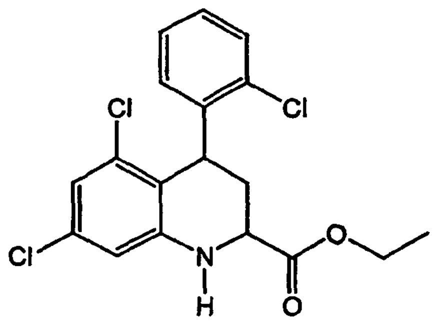

5,7-Dichlor-4-(2-chlor-phenyl)-1,2,3,4-tetrahydrochinolin-2-carbonsäureethylester (33)

Verbindung 33 wurde gemäß dem Grundverfahren aus 5.0 mmol 3,5-Dichloranilin, 5.5 mmol Ethylglyoxalat-Lösung (50%, Toluol), 15.0 mmol 2-Chlorstyrol und 5.0 mmol Trifluoressigsäure in 30.0 ml Acetonitril dargestellt. Zur Charakterisierung wurde ein ESI-MS aufgenommen:

MS (EI) m/z: (M*) 384

**REFERENZ Beispiel 34**

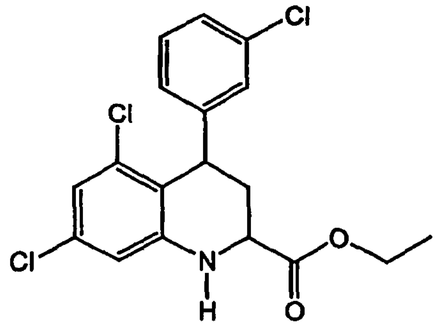

5,7-Dichlor-4-(3-chlor-phenyl)-1,2,3,4-tetrahydrochinolin-2-carbonsäureethylester (34)

Verbindung **34** wurde gemäß dem Grundverfahren aus 5.0 mmol 3,5-Dichloranilin, 5.5 mmol Ethylglyoxalat-Lösung (50%, Toluol), 15.0 mmol 3-Chlorstyrol und 5.0 mmol Trifluoressigsäure in 30.0 ml Acetonitril dargestellt. Zur Charakterisierung wurde ein ESI-MS aufgenommen:

MS (EI) m/z: (M*) 384

**REFERENZ-Beispiel 35**

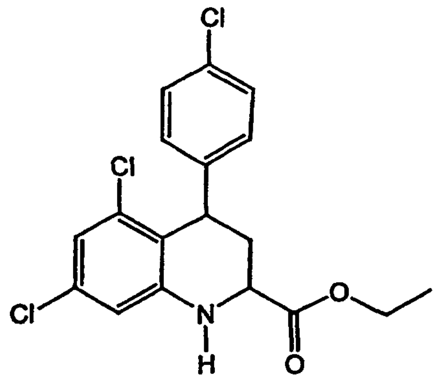

**5,7-Dichlor-4-(4-chlor-phenyl)-1,2,3,4-tetrahydrochinolin-2-carbonsäureethylester (35)**

Verbindung **35** wurde gemäß dem Grundverfahren aus 5.0 mmol 3,5-Dichloranilin, 5.5 mmol Ethylglyoxalat-Lösung (50%, Toluol), 15.0 mmol 4-Chlorstyrol und 5.0 mmol Trifluoressigsäure in 30.0 ml Acetonitril dargestellt. Zur Charakterisierung wurde ein ESI-MS aufgenommen:

MS (EI) m/z: (M*) 384

**REFERENZ-Beispiel 36**

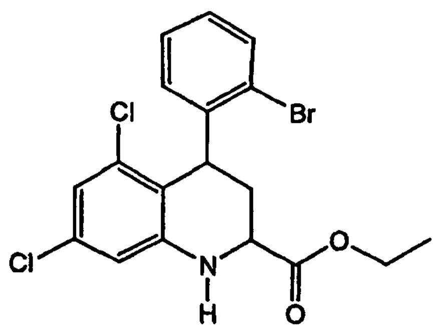

**4-(2-Brom-phenyl)-5,7-dichlor-1,2,3,4-tetrahydrochinolin-2-carbonsäureethylester (36)**

Verbindung **36** wurde gemäß dem Grundverfahren aus 5.0 mmol 3,5-Dichloranilin, 5.5 mmol Ethylglyoxalat-Lösung (50%, Toluol), 15.0 mmol 2-Bromstyrol und 5.0 mmol Trifluoressigsäure in 30.0 ml Acetonitril dargestellt. Zur Charakterisierung wurde ein ESI-MS aufgenommen:

MS (EI) m/z: (M*) 429

**REFERENZ-Beispiel 37**

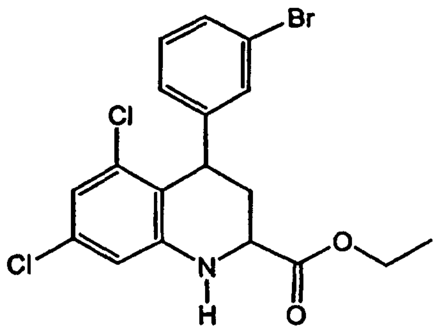

4-(3-Brom-phenyl)-5,7-dichlor-1,2,3,4-tetrahydrochinolin-2-carbonsäureethylester (37)

Verbindung **37** wurde gemäß dem Grundverfahren aus 5.0 mmol 3,5-Dichloranilin, 5.5 mmol Ethylglyoxalat-Lösung (50%, Toluol), 15.0 mmol 3-Bromstyrol und 5.0 mmol Trifluoressigsäure in 30.0 ml Acetonitril dargestellt.
Zur Charakterisierung wurde ein ESI-MS aufgenommen:

MS (EI) m/z: (M*)429

**REFERENZ-Beispiel 38**

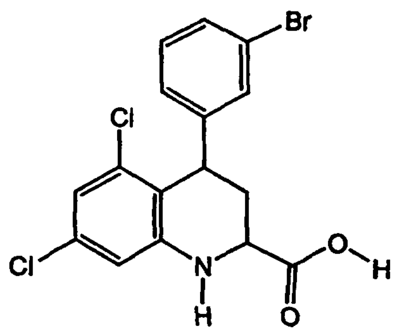

4-(3-Brom-phenyl)-5,7-dichlor-1,2,3,4-tetrahydrochinolin-2-carbonsäure (38)

Verbindung **38** wurde gemäß dem Grundverfahren aus 5.0 mmol 3,5-Dichloranilin, 5.5 mmol Ethylglyoxalat-Lösung (50%, Toluol), 15.0 mmol 3-Bromstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*)401

**REFERENZ-Beispiel 39**

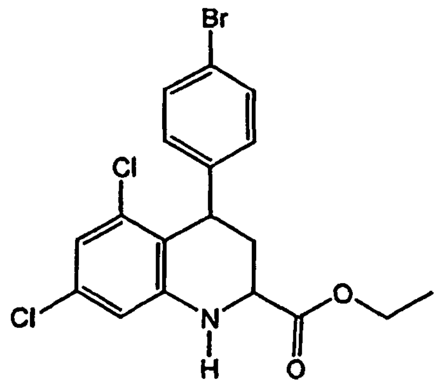

4-(4-Brom-phenyl)-5,7-dichlor-1,2,3,4-tetrahydrochinolin-2-carbonsäureethylester (39)

Verbindung **39** wurde gemäß dem Grundverfahren aus 5.0 mmol 3,5-Dichloranilin, 5.5 mmol Ethylglyoxalat-Lösung (50%, Toluol), 15.0 mmol 4-Bromstyrol und 5.0 mmol Trifluoressigsäure in 30.0 ml Acetonitril dargestellt. Zur Charakterisierung wurde ein ESI-MS aufgenommen:

MS (EI) m/z: (M*)429

**REFERENZ-Beispiel 40**

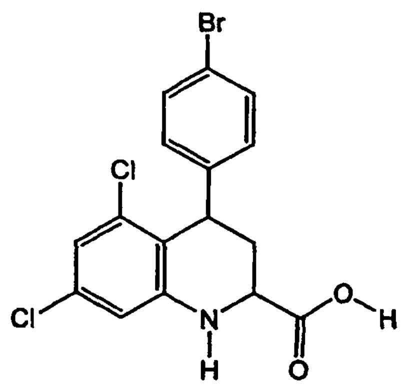

4-(4-Brom-phenyl)-5,7-dichlor-1,2,3,4-tetrahydrochinolin-2-carbonsäure (40)

Verbindung **40** wurde gemäß dem Grundverfahren aus 5.0 mmol 3,5-Dichloranilin, 5.5 mmol Ethylglyoxalat-Lösung (50%, Toluol), 15.0 mmol 4-Bromstyrol und 5.0 mmol Trifluoressigsäure in 30.0 ml Acetonitril dargestellt. Die anschließende Verseifung erfolgte mit 1.0 ml Natronlauge (6 N, Wasser) in 20.0 ml Ethanol.
Zur Charakterisierung wurde ein ESI-MS aufgenommen:

MS (EI) m/z: (M*)401

**REFERENZ-Beispiel 41**

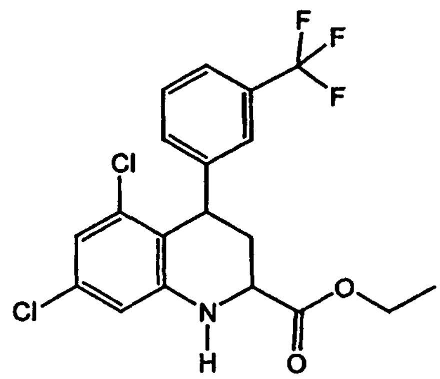

**5,7-Dichlor-4-(3-trifluoromethyl-phenyl)-1,2,3,4-tetrahydrochinolin-2-carbonsäureethylester (41**

Verbindung **41** wurde gemäß dem Grundverfahren aus 5.0 mmol 3,5-Dichloranilin, 5.5 mmol Ethylglyoxalat-Lösung (50%, Toluol), 15.0 mmol 3-Trifluormethylstyrol und 5.0 mmol Trifluoressigsäure in 30.0 ml Acetonitril dargestellt.
Zur Charakterisierung wurde ein ESI-MS aufgenommen:

MS (EI) m/z: (M*)417

**REFERENZ-Beispiel 42**

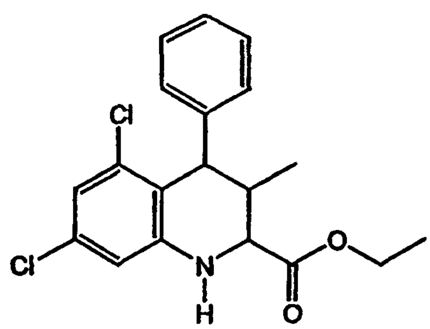

**5,7-Dichlor-3-methyl-4-phenyl-1,2,3,4-tetrahydrochinolin-2-carbonsäureethylester (42)**

Verbindung 42 wurde gemäß dem Grundverfahren aus 5.0 mmol 3,5-Dichloranilin, 5.5 mmol Ethylglyoxalat-Lösung (50%, Toluol), 15.0 mmol β-Methylstyrol und 5.0 mmol Trifluoressigsäure in 30.0 ml Acetonitril dargestellt.
Zur Charakterisierung wurde ein ESI-MS aufgenommen:

MS (EI) m/z: (M*)363

**REFERENZ-Beispiel 43**

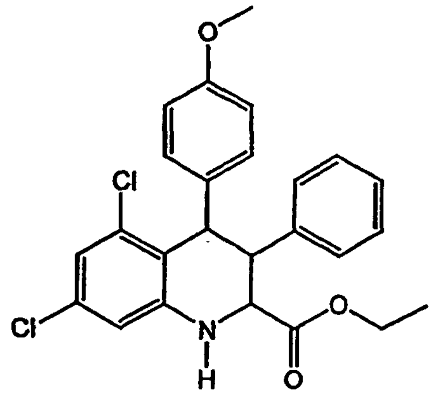

**5,7-Dichlor-4-(4-methoxy-phenyl)-3-phenyl-1,2,3,4-tetrahydrochinolin-2-carbonsäureethylester (**

Verbindung **43** wurde gemäß dem Grundverfahren aus 5.0 mmol 3,5-Dichloranilin, 5.5 mmol Ethylglyoxalat-Lösung (50%, Toluol), 15.0 mmol trans-4-Methoxystilben und 5.0 mmol Trifluoressigsäure In 30.0 ml Acetonitril dargestellt.
Zur Charakterisierung wurde ein ESI-MS aufgenommen:

MS (EI) m/z: (M*) 455

**REFERENZ-Beispiel 44**

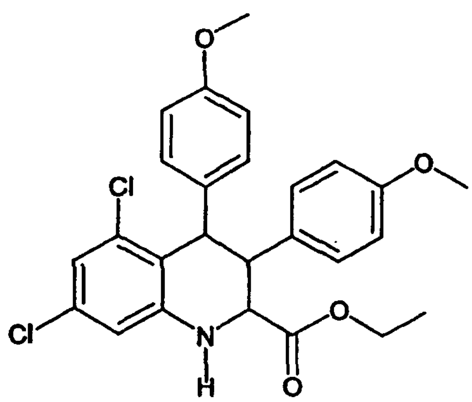

**5,7-Dichlor-3,4-bis-(4-methoxy-phenyl)-1,2,3,4-tetrahydrochinolin-2-carbonsäureethylester (44)**

Verbindung **44** wurde gemäß dem Grundverfahren aus 5.0 mmol 3,5-Dichloranilin, 5.5 mmol Ethylglyoxalat-Lösung (50%, Toluol), 15.0 mmol trans-4,4'-Dimethoxystilben und 5.0 mmol Trifluoressigsäure in 30.0 ml Acetonitril dargestellt.
Zur Charakterisierung wurde ein ESI-MS aufgenommen:

MS (EI) m/z: (M*)485

**REFERENZ-Beispiel 45**

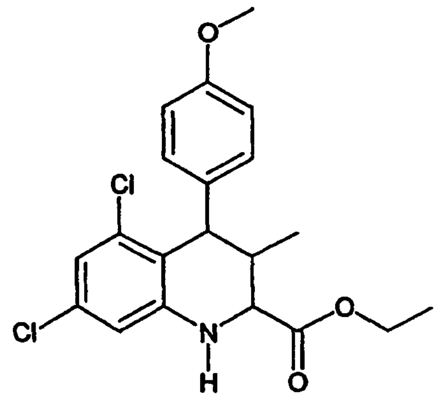

**5,7-Dichlor-4-(4-methoxy-phenyl)-3-methyl-1,2,3,4-tetrahydrochinolin-2-carbonsäureethylester (45)**

Verbindung **45** wurde gemäß dem Grundverfahren aus 5.0 mmol 3,5-Dichloranilin, 5.5 mmol Ethylglyoxalat-Lösung (50%, Toluol), 15.0 mmol Anethol und 5.0 mmol Trifluoressigsäure in 30.0 ml Acetonitril dargestellt.
Zur Charakterisierung wurde ein ESI-MS aufgenommen:

MS (EI) m/z: (M*)393

**REFERENZ-Beispiel 46**

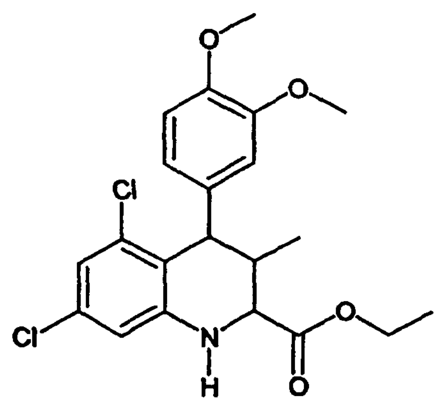

**5,7-Dichlor-4-(3,4-dimethoxy-phenyl)-3-methyl-1,2,3,4-tetrahydrochinolin-2-carbonsäureethylester (46)**

Verbindung **46** wurde gemäß dem Grundverfahren aus 5.0 mmol 3,5-Dichloranilin, 5.5 mmol Ethylglyoxalat-Lösung (50%, Toluol), 15.0 mmol 1,2-Dimethoxy-4-prop-1-enylbenzol und 5.0 mmol Trifluoressigsäure in 30.0 ml Acetonitril dargestellt.
Zur Charakterisierung wurde ein ESI-MS aufgenommen:

MS (EI) m/z: (M*)423

**REFERENZ-Beispiel 47**

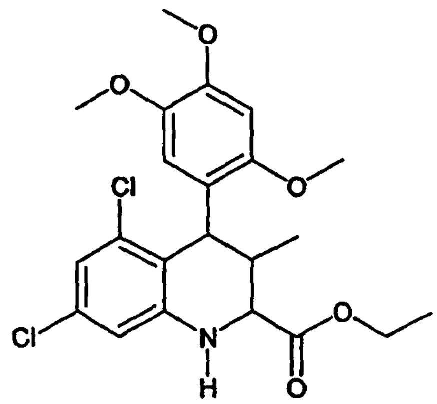

**5,7-Dichlor-3-methyl-4-(2,4,5-trimethoxy-phenyl)-1,2,3,4-tetrahydrochinolin-2-carbonsäureethylest**

Verbindung **47** wurde gemäß dem Grundverfahren aus 5.0 mmol 3,5-Dichloranilin, 5.5 mmol Ethylglyoxalat-Lösung (50%, Toluol), 15.0 mmol β-Asaron und 5.0 mmol Trifluoressigsäure in 30.0 ml Acetonitril dargestellt. Zur Charakterisierung wurde ein ESI-MS aufgenommen:

MS (EI) m/z: (M*)453

**REFERENZ-Beispiel 48**

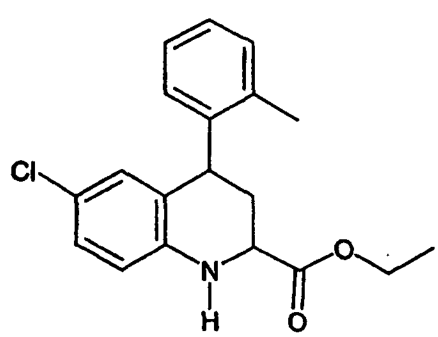

**6-Chlor-4-*o*-tolyl-1,2,3,4-tetrahydrochinolin-2-carbonsäurethylester (48)**

Verbindung **48** wurde gemäß dem Grundverfahren aus 5.0 mmol 3,5-Dichloranilin, 5.5 mmol Ethylglyoxalat-Lösung (50%, Toluol), 15.0 mmol 2-Methylstyrol und 5.0 mmol Trifluoressigsäure in 30.0 ml Acetonitril dargestellt. Zur Charakterisierung wurde ein ESI-MS aufgenommen:

MS (EI) m/z: (M*)329

**REFERENZ-Beispiel 49**

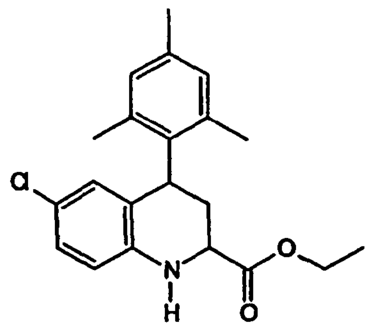

**6-Chlor-4-(2,4,6-trimethylphenyl)-1,2,3,4-tetrahydrochinolin-2-carbonsäureethylester (49)**

Verbindung **49** wurde gemäß dem Grundverfahren aus 5.0 mmol 4-Chloranilin, 5.5 mmol Ethylglyoxalat-Lösung (50%, Toluol), 15.0 mmol 2,4,6-Trimethylstyrol und 5.0 mmol Trifluoressigsäure in 30.0 ml Acetonitril dargestellt. Zur Charakterisierung wurde ein ESI-MS aufgenommen:

MS (EI) m/z: (M*) 357

**REFERENZ-Beispiel 50**

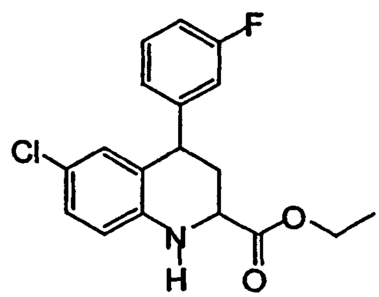

**6-Chlor-4-(3-fluorphenyl)-1,2,3,4-tetrahydrochinolin-2-carbonsäureethylester (50)**

Verbindung **50** wurde gemäß dem Grundverfahren aus 5.0 mmol 4-Chloranilin, 5.5 mmol Ethylglyoxalat-Lösung (50%, Toluol), 15.0 mmol 3-Fluorstyrol und 5.0 mmol Trifluoressigsäure in 30.0 ml Acetonitril dargestellt. Zur Charakterisierung wurde ein ESI-MS aufgenommen:

MS (EI) m/z: (M*)333

**REFERENZ-Beispiel 51**

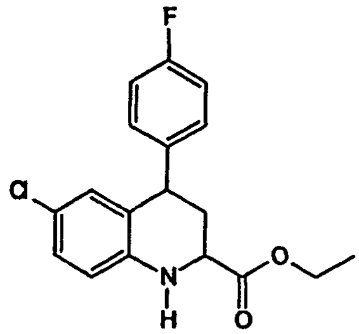

**6-Chlor-4-(4-fluorphenyl)-1,2,3,4-tetrahydrochinolin-2-carbonsäureethylester (51)**

Verbindung **51** wurde gemäß dem Grundverfahren aus 5.0 mmol 4-Chloranilin, 5.5 mmol Ethylglyoxalat-Lösung (50%, Toluol), 15.0 mmol 4-Fluorstyrol und 5.0 mmol Trifluoressigsäure in 30.0 ml Acetonitril dargestellt. Zur Charakterisierung wurde ein ESI-MS aufgenommen:

MS (EI) m/z: (M*)333

**REFERENZ-Beispiel 52**

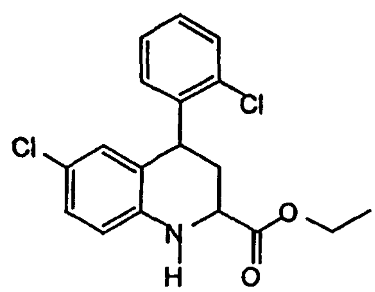

**6-Chlor-4-(2-chlorphenyl)-1,2,3,4-tetrahydrochinolin-2-carbonsäureethylester (52)**

Verbindung **52** wurde gemäß dem Grundverfahren aus 5.0 mmol 4-Chloranilin, 5.5 mmol Ethylglyoxalat-Lösung (50%, Toluol), 15.0 mmol 2-Chlorstyrol und 5.0 mmol Trifluoressigsäure in 30.0 ml Acetonitril dargestellt. Zur Charakterisierung wurde ein ESI-MS aufgenommen:

MS (EI) m/z: (M*)383

**REFERENZ-Beispiel 53**

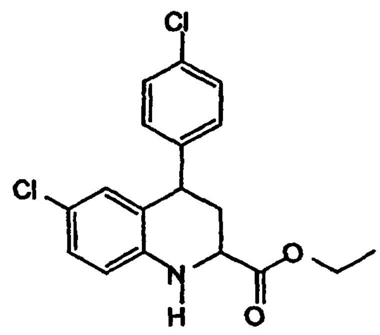

**6-Chlor-4-(4-chlorphenyl)-1,2,3,4-tetrahydrochinolin-2-carbonsäureethylester (53)**

Verbindung **53** wurde gemäß dem Grundverfahren aus 5.0 mmol 4-Chloranilin, 5.5 mmol Ethylglyoxalat-Lösung (50%, Toluol), 15.0 mmol 4-Chlorstyrol und 5.0 mmol Trifluoressigsäure in 30.0 ml Acetonitril dargestellt. Zur Charakterisierung wurde ein ESI-MS aufgenommen:

MS (EI) m/z: (M*)383

**REFERENZ-Beispiel 54**

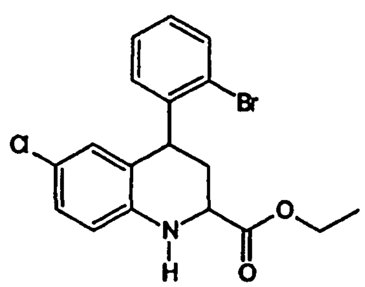

**6-Chlor-4-(2-bromphenyl)-1,2,3,4-tetrahydrochinolin-2-carbonsäureethylester (54)**

Verbindung **54** wurde gemäß dem Grundverfahren aus 5.0 mmol 4-Chloranilin, 5.5 mmol Ethylglyoxalat-Lösung (50%, Toluol), 15.0 mmol 2-Bromstyrol und 5.0 mmol Trifluoressigsäure in 30.0 ml Acetonitril dargestellt. Zur Charakterisierung wurde ein ESI-MS aufgenommen:

MS (EI) m/z: (M*)395

**REFERENZ-Beispiel 55**

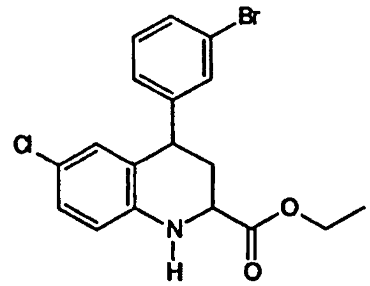

**6-Chlor-4-(3-bromphenyl)-1,2,3,4-tetrahydrochinolin-2-carbonsäureethylester (55)**

Verbindung **55** wurde gemäß dem Grundverfahren aus 5.0 mmol 4-Chloranilin, 5.5 mmol Ethylglyoxalat-Lösung (50%, Toluol), 15.0 mmol 3-Bromstyrol und 5.0 mmol Trifluoressigsäure in 30.0 ml Acetonitril dargestellt.
Zur Charakterisierung wurde ein ESI-MS aufgenommen:

MS (EI) m/z: (M*)395

**REFERENZ-Beispiel 56**

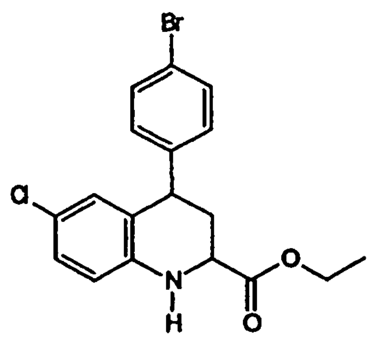

**6-Chlor-4-(4-bromphenyl)-1,2,3,4-tetrahydrochinolin-2-carbonsäureethylester (56)**

Verbindung **56** wurde gemäß dem Grundverfahren aus 5.0 mmol 4-Chloranilin, 5.5 mmol Ethylglyoxalat-Lösung (50%, Toluol), 15.0 mmol 4-Bromstyrol und 5.0 mmol Trifluoressigsäure in 30.0 ml Acetonitril dargestellt. Zur Charakterisierung wurde ein ESI-MS aufgenommen:

MS (EI) m/z: (M*)395

**REFERENZ-Beispiel 57**

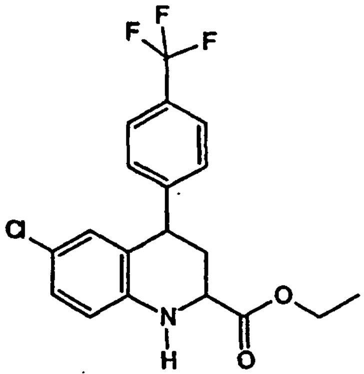

6-Chlor-4-(4-trifluormethylphenyl)-1,2,3,4-tetrahydrochinolin-2-carbonsäureethylester (57)

Verbindung **57** wurde gemäß dem Grundverfahren aus 5.0 mmol 4-Chloranilin, 5.5 mmol Ethylglyoxalat-Lösung (50%, Toluol), 15.0 mmol 4-Trifluormethylstyrol und 5.0 mmol Trifluoressigsäure in 30.0 ml Acetonitril dargestellt. Zur Charakterisierung wurde ein ESI-MS aufgenommen:

MS (EI) m/z: (M*)383

**REFERENZ-Beispiel 58**

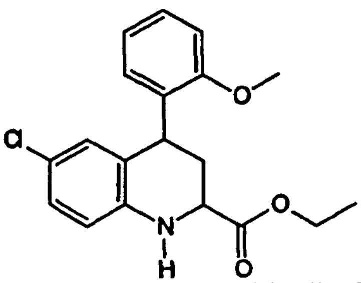

6-Chlor-4-(2-methoxyphenyl)-1,2,3,4-tetrahydrochinolin-2-carbonsäureethylester (58)

Verbindung **58** wurde gemäß dem Grundverfahren aus 5.0 mmol 4-Chloranilin, 5.5 mmol Ethylglyoxalat-Lösung (50%, Toluol), 15.0 mmol 2-Methoxystyrol und 5.0 mmol Trifluoressigsäure in 30.0 ml Acetonitril dargestellt. Zur Charakterisierung wurde ein ESI-MS aufgenommen:

MS (EI) m/z: (M*)345

**Beispiel 59**

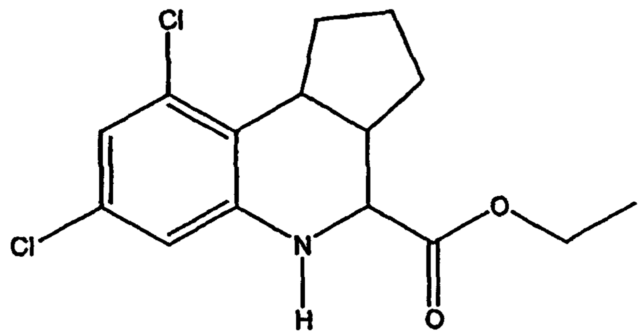

**7,9-Dichlor-2,3,3a,4,5,9b-hexahydro-1*H*-cyclopenta[c]chinolin-4-carbonsäure-ethylester (59)**

Verbindung **59** wurde gemäß dem Grundverfahren aus 5.0 mmol 3,5-Dichloranilin, 5.5 mmol Ethylglyoxalat-Lösung (50%, Toluol), 15.0 mmol Cyclopenten und 5.0 mmol Trifluoressigsäure in 30.0 ml Acetonitril in 89% Ausbeute dargestellt.
Zur Charakterisierung wurde ein ESI-MS aufgenommen:

MS (EI) m/z: 313 (M*).

**Beispiel 60**

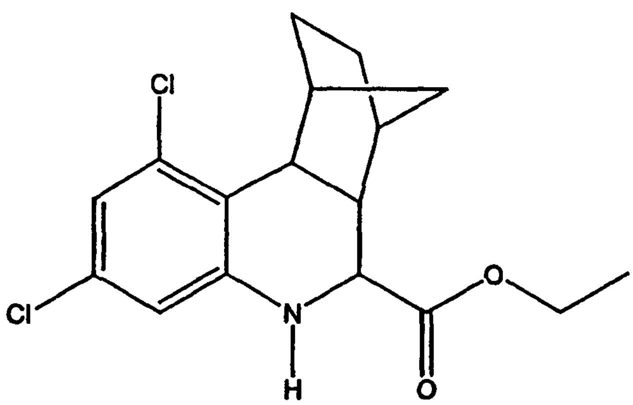

**1,3-Dichlor-7,10-methano-5,6,6a,7,8,9,10,10a-octahydrophenanthridin-6-carbon-säureethylester (60)**

Verbindung **60** wurde gemäß dem Grundverfahren aus 5.0 mmol 3,5-Dichloranilin, 5.5 mmol Ethylglyoxalat-Lösung (50%, Toluol), 15.0 mmol Bicyclo[2.2.1]hept-2-en und 5.0 mmol Trifluoressigsäure in 30.0 ml Acetonitril in 89% Ausbeute dargestellt.
Zur Charakterisierung wurde ein ESI-MS aufgenommen:

MS (EI) m/z: 339 (M*).

**Beispiel 61**

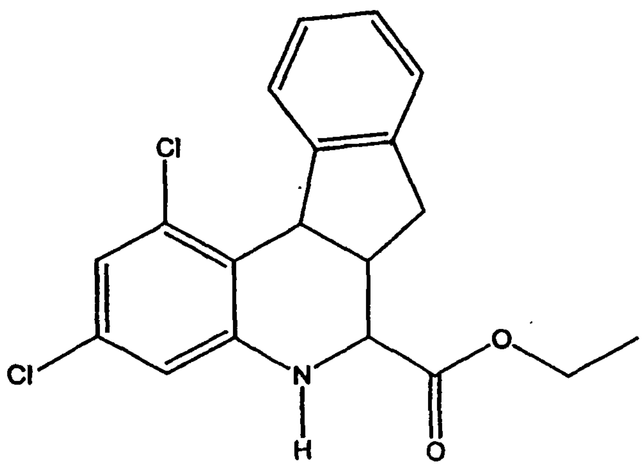

**5,6a,7,11b-tetrahydro-6*H*-indeno-[2,1-*c*]chinolin-6-carbonsäureethylester (61)**

Verbindung **61** wurde gemäß dem Grundverfahren aus 5.0 mmol 3,5-Dichloranilin, 5.5 mmol Ethylglyoxalat-Lösung (50%, Toluol), 15.0 mmol Inden und 5.0 mmol Trifluoressigsäure in 30.0 ml Acetonitril in 89% Ausbeute dargestellt.
Zur Charakterisierung wurde ein ESI-MS aufgenommen:

MS (EI) m/z: 361 (M*).

**Beispiel 62**

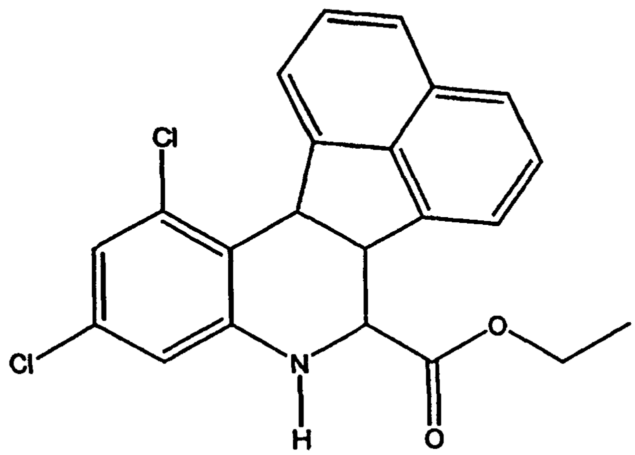

**10,12-Dichlor-6b,7,8,12b-tetrahydro-8-azabenzo[*j*]fluoranthren-7-carbonsäureethyl-ester (62)**

Verbindung **62** wurde gemäß dem Grundverfahren aus 5.0 mmol 3,5-Dichloranilin, 5.5 mmol Ethylglyoxalat-Lösung (50%, Toluol), 15.0 mmol Acenaphthen und 5.0 mmol Trifluoressigsäure in 30.0 ml Acetonitril in 89% Ausbeute dargestellt.

Zur Charakterisierung wurde ein ESI-MS aufgenommen:

MS (EI) m/z: 397 (M*).

**Beispiel 63**

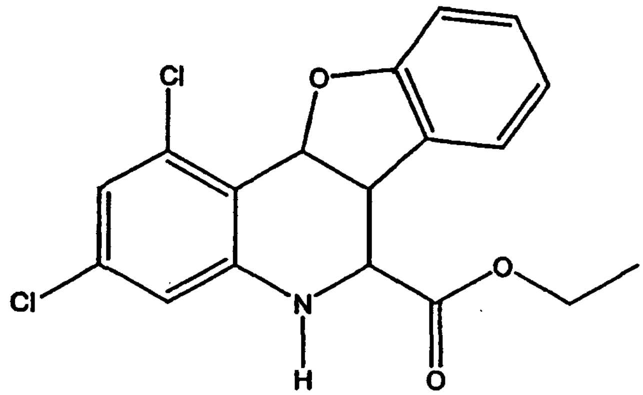

**1,3-Dichlor-5,6,6a,11a-tetrahydro-11-oxa-5-aza-benzo[*a*]fluoren-6-carbonsäureethyl-ester (63)**

Verbindung **63** wurde gemäß dem Grundverfahren aus 5.0 mmol 3,5-Dichloranilin, 5.5 mmol Ethylglyoxalat-Lösung (50%, Toluol), 15.0 mmol Benzofuran und 5.0 mmol Trifluoressigsäure in 30.0 ml Acetonitril in 89% Ausbeute dargestellt.
Zur Charakterisierung wurde ein ESI-MS aufgenommen:

MS (EI) m/z: 363 (M*).

**Beispiel 64**

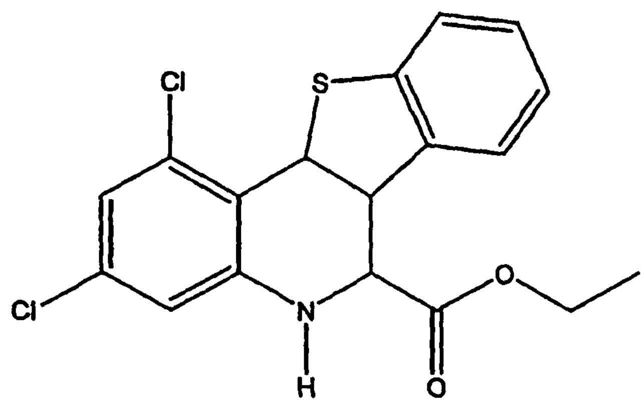

**1,3-Dichlor-5,6,6a,11a-tetrahydro-11-thia-5-aza-benzo[*a*]fluoren-6-carbonsäureethyl-ester (64)**

Verbindung **64** wurde gemäß dem Grundverfahren aus 5.0 mmol 3,5-Dichloranilin, 5.5 mmol Ethylglyoxalat-Lösung (50%, Toluol), 15.0 mmol Thianaphathen und 5.0 mmol Trifluoressigsäure in 30.0 ml Acetonitril in 89% Ausbeute dargestellt.
Zur Charakterisierung wurde ein ESI-MS aufgenommen:

MS (EI) m/z: 379 (M*).

**REFERENZ Beispiel 65**

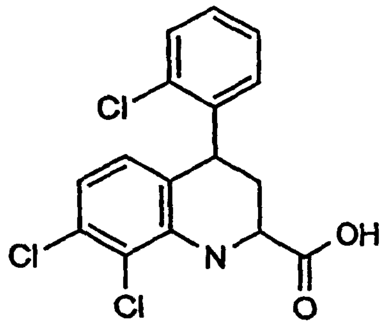

7,8-Dichlor-4-(2-chlor-phenyl)-1,2,3,4-tetrahydrochinolin-2-carbonsäure (65)

Verbindung **65** wurde gemäß des automatisierten Verfahrens aus 2,3-Dichloranilin, Glyoxylsäure und 2-Chlorstyrol dargestellt.

**REFERENZ-Beispiel 66**

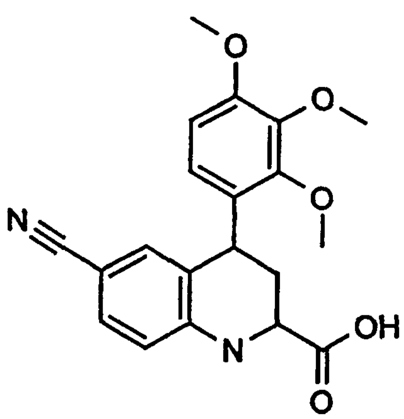

6-Cyano-4-(2,3,4-trimethoxy-phenyl)-1,2,3,4-tetrahydrochinolin-2-carbonsäure (66)

Verbindung **66** wurde gemäß des automatisierten Verfahrens aus 4-Aminobenzonitril, Glyoxylsäure und 2,3,4-Tetramethoxystyrol dargestellt.

**Beispiel 67**

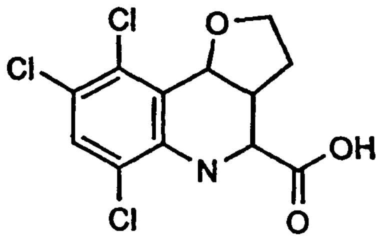

**6,8,9-Trichlor-2,3,3a,4,5,9b-hexahydro-furo[3,2-c]chinolin-4-carbonsäure (67)**

Verbindung **67** wurde gemäß des automatisierten Verfahrens aus 2,4,5-Trichloranilin, Glyoxylsäure und 2,3-Dihydrofuran dargestellt.

**REFERENZ-Beispiel 68**

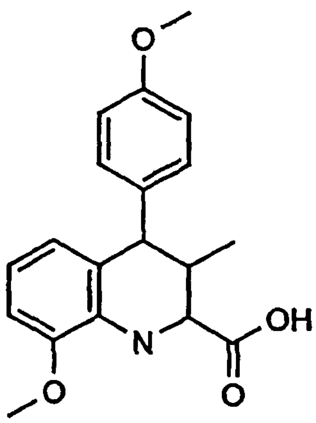

**8-Methoxy-4-(4-methoxy-phenyl)-3-methyl-1,2,3,4-tetrahydrochinolin-2-carbonsäure (68)**

Verbindung **68** wurde gemäß des automatisierten Verfahrens aus 2-Methoxyanilin, Glyoxylsäure und *trans*-Anethol dargestellt.

**REFERENZ-Beispiel 69**

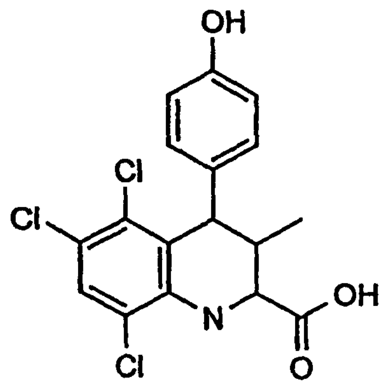

**5,6,8-Trichlor-4-(4-hydroxy-phenyl)-3-methyl-1,2,3,4-tetrahydrochinolin-2-carbonsäure (69)**

Verbindung **69** wurde gemäß des automatisierten Verfahrens aus 2,3,5-Tnchloranilin, Glyoxylsäure und 2-Propenylphenol dargestellt.

**REFERENZ-Beispiel 70**

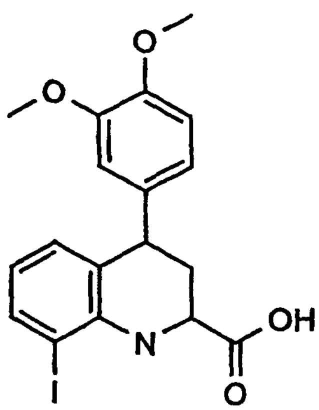

**4-(3,4-Dimethoxy-phenyl)-8-Iod-1,2,3,4-tetrahydrochinolin-2-carbonsäure (70)**

Verbindung **70** wurde gemäß des automatisierten Verfahrens aus 2-Iodanllin, Glyoxylsäure und 3,4-Dimethoxystyrol dargestellt.

**REFERENZ-Beispiel 71**

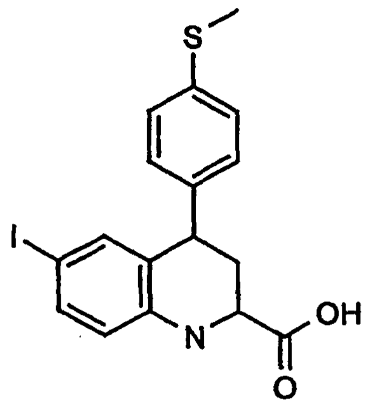

**6-Iod-4-(4-methylsulfanyl-phenyl)-1,2,3,4-tetrahydrochinolin-2-carbonsäure (71)**

Verbindung **71** wurde gemäß des automatisierten Verfahrens aus 4-Iodanilin, Glyoxylsäure und 1-Methylsulfanyl-4-vinylbenzol dargestellt.

**REFERENZ-Beispiel 72**

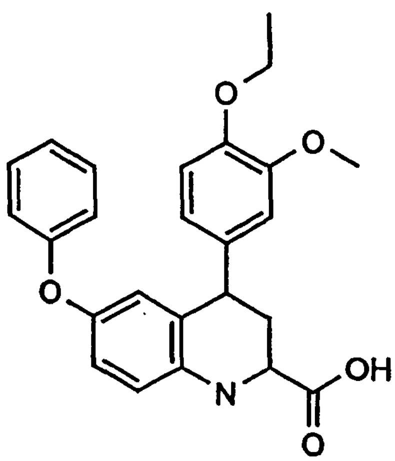

**4-(4-Ethoxy-3-methoxy-phenyl)-6-phenoxy-1,2,3,4-tetrahydrochinolin-2-carbonsäure (72)**

Verbindung **72** wurde gemäß des automatisierten Verfahrens aus 4-Phenoxyanllin, Glyoxylsäure und 1-Ethoxy-2-methoxy-4-vinyl-benzol dargestellt.

**REFERENZ-Beispiel 73**

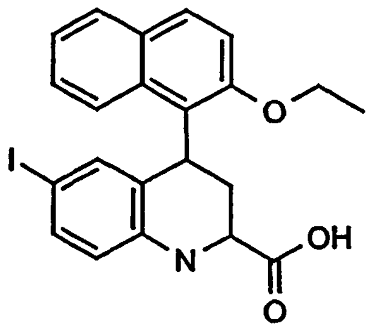

**4-(2-Ethoxy-naphthalen-1-yl)-6-Iod-1,2,3,4-tetrahydrochinolin-2-carbonsäure (73)**

Verbindung **73** wurde gemäß des automatisierten Verfahrens aus 4-Iodanilin, Glyoxylsäure und 2-Ethoxy-1-vinylnaphthalin dargestellt.

**REFERENZ-Beispiel 74**

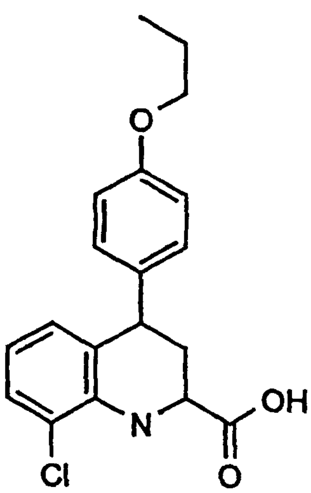

**8-Chlor-4-(4-propoxyphenyl)-1,2,3,4-tetrahydrochinolin-2-carbonsäure (74)**

Verbindung **74** wurde gemäß des automatisierten Verfahrens aus 2-Chloranilin, Glyoxylsäure und 4-Propoxystyrol dargestellt

**REFERENZ-Beispiel 75**

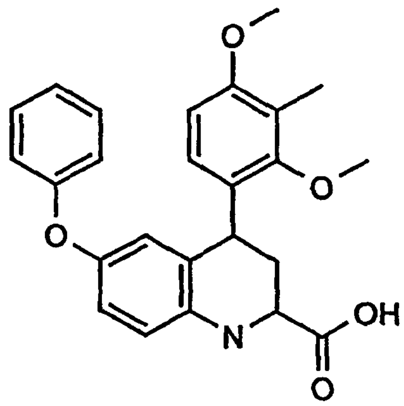

**4-(2,4-Dimethoxy-3-methylphenyl)-6-phenoxy-1,2,3,4-tetrahydrochinolin-2-carbonsäure (75)**

Verbindung **75** wurde gemäß des automatisierten Verfahrens aus 4-Phenoxyanilin, Glyoxylsäure und 2,4-Dimethoxy-3-methylstyrol dargestellt

**Beispiel 76**

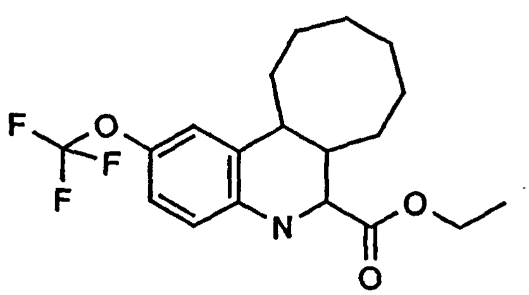

**2-Trifluormethoxy-5,6,6a,7,8,9,10,11,12,12a-decahydro-5-aza-cycloocta[a]naphthalen-6-carbonsäure ethyl ester (76)**

Verbindung **76** wurde gemäß des automatisierten Verfahrens aus 4-(Trifluormethoxy)-anilin, Glyoxylsäureethylester und Cyclooctcen dargestellt

**REFERENZ-Beispiel 77**

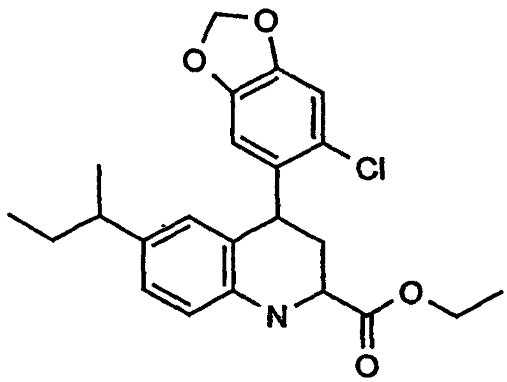

**6-sec-Butyl-4-(6-chlor-benzo[1,3]dioxol-5-yl)-1,2,3,4-tetrahydrochinolin-2-carbonsäure ethyl ester (77)**

Verbindung **77** wurde gemäß des automatisierten Verfahrens aus 4-*sec*-Butylanilin, Glyoxylsäureethylester und 5-Chlor-6-vinyl-benzo[1,3]dioxol dargestellt

Die Beispiele 78 bis 102 wurden analog dargestellt.

| Beispiel | Name |
|---|---|
| R **78** | 4-Anthracen-9-yl-6-chlor-8-methyl-1,2,3,4-tetrahydrochinolin-2-carbonsäure |
| R **79** | 6-sec-Butyl-4-naphthalen-1-yl-1234-tetrahydrochinolin-2-carbonsäure |
| R **80** | 4-(4-Hydroxy-phenyl)-3-methyl-8-phenoxy-1,2,3,4-tetrahydrochinolin-2-carbonsäure |
| R **81** | 8-Chlor-6-fluor-4-naphthalen-2-yl-1,2,3,4-tetrahydrochinolin-2-carbonsäure |
| R **82** | 4-(4-Methoxy-phenyl)-3-methyl-6-phenoxy-1,2,3,4-tetrahydrochinolin-2-carbonsäure |
| R **83** | 6-Chlor-8-fluor-4-m-tolyl-1,2,3,4-tetrahydrochinolin-2-carbonsäure |
| R **84** | 8-Chlor-6-fluor-4-m-tolyl-1,2,3,4-tetrahydrochinolin-2-carbonsäure |
| R **85** | 4-(4-Brom-phenyl)-6-chlor-8-fluor-1,2,3,4-tetrahydrochinolin-2-carbonsäure |
| R **86** | 7,8-Dichlor-4-(2,4-dimethyl-phenyl)-1,2,3,4-tetrahydrochinolin-2-carbonsäure |
| R **87** | 6-Chlor-4-(4-chlor-phenyl)-7-trifluormethyl-1,2,3,4-tetrahydrochinolin-2-carbonsäure |
| **88** | 2-Cyano-5,6a,7,11b-tetrahydro-6H-indeno[2,1-c]chinolin-6-carbonsäureethy ester |
| R **89** | 4-(2-Chlor-phenyl)-6-cyano-1,2,3,4-tetrahydrochinolin-2-carbonsäure |
| R **90** | 6-brom-8-chlor-3-methyl-4-phenyl-1234-tetrahydrochinolin-2-carbonsäure ethyl ester |
| R **91** | 6-Brom-8-chlor-4-(2,4-dimethyl-phenyl)-1,2,3,4-tetrahydrochinolin-2-carbonsäure |
| R **92** | 6-Brom-4-(2-bromphenyl)-8-chlor-1,2,3,4-tetrahydrochinolin-2-carbonsäure |
| R **93** | 4-(4-Hydroxy-3-methoxy-phenyl)-3-methyl-6-methylsulfanyl-1,2,3,4-tetrahydrochinolin-2-carbonsäure |
| R **94** | 6-Cyano-3,4-bis-(4-methoxy-phenyl)-1,2,3,4-tetrahydrochinolin-2-carbonsäure |
| R **95** | 8-Chlor-6-fluor-34-bis-(4-methoxy-phenyl)-1234-tetrahydrochinolin-2-carbonsäure ethyl ester |
| R **96** | 4-(4-Benzyloxy-3-methoxy-phenyl)-6-tert-butyl-1234-tetrahydrochinolin-2-carbonsäure ethyl ester |

(fortgesetzt)

| Beispiel | Name |
|---|---|
| R **97** | 5,7-Dichlor-4-(4-chlor-phenyl)-1,2,3,4-tetrahydrochinolin-2-carbonsäure |
| R **98** | 5,7-Dichlor-4-(3-chlor-phenyl)-1,2,3,4-tetrahydrochinolin-2-carbonsäure |
| R **99** | 5,7-Dichlor-4-(4-chlor-phenyl)-1,2,3,4-tetrahydrochinolin-2-carbonsäure |
| **100** | 1,3-Dichlor-5,6,6a,7,8,12b-hexahydro-benzo[k]phenanthridine-6-carbonsäure |
| **101** | 1,3-Dichlor-5,6a,7,11b-tetrahydro-6H-indeno[2,1-c]chinolin-6-carbonsäure |
| R **102** | 5,7-Dichlor-4-(3,5-dimethyl-phenyl)-1,2,3,4-tetrahydrochinolin-2-carbonsäure |
| R: REFERENZ-BEISPIELE | |

**Beispiel 103**

Rezeptorbindung (Glycin-Bindungsstelle des NMDA-Rezeptorkanals)

Die Untersuchungen zur Bestimmung der Affinität der erfindungsgemäßen Verbindungen der Formel I zur Glycin-Bindungsstelle des NMDA-Rezeptorkanals wurde an Himmembran-homogenaten (Homogenat von Cortex- und Hippocampus-Areal aus dem Hirn von männlichen Ratten, Stamm Wistar) durchgeführt [B.M. Baron, B.W. Siegel, B. L. Harrison, R.S. Gross, C. Hawes and P.Towers, Journal of Pharmacology and Experimental Therapeutics, Vol. 279, S. 62, 1996].

Hierzu wurde Cortex und Hippocampus aus frisch entnommenen Rattengehimen freipräpariert und in 5 mmol/l TRIS-Acetatpuffer, 0,32 mol/l Sacharose pH 7,4 (10 ml/g Frischgewicht) mit einem Potter-Homogenisator (Fa. Braun/Melsungen 10 Kolbenhübe bei 500 Upm) unter Eiskühlung homogenisiert und anschließend für 10 Minuten bei 1.000 g und 4°C zentrifugiert. Der erste Überstand wurde gesammelt und das Sediment eeneut mit 5 mmol/l TRIS-Acetatpuffer, 0,32 mol/l Sacharose pH 7,4 (5 ml/g ursprüngliches Frischgewicht) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wurde mit dem Überstand aus der ersten Zentrifugation vereinigt und bei 17.000 g für 20 Minuten bei 4°C zentrifugiert. Der Überstand nach dieser Zentrifugation wurde verworfen und das Membran-sediment mit 5 mmol/l TRIS-Acotatpuffer pH 8,0 (20 ml/g ursprüngliches Frischgewicht) aufgenommen und mit 10 Kolbenhüben bei 500 Upm homogenisiert.

Anschließend wurde das Membranhomogenat für 1 Stunde bei 4°C inkubiert für 30 Minuten bei 50.000 g und 4°C zentrifugiert. Der Überstand wurde verworfen und das Zentrifugen-röhrchen mit dem Membransediment mit Parafilm verschlossen und für 24 Stunden bei -20°C eingefroren. Am folgenden Tag wurde das Membransediment aufgetaut und mit eiskaltem 5 mmol/l TRIS-Acetatpuffer, 0,1 % Saponin (w/v) pH 7,0 (10 ml/g ursprüngliches Frischgewicht) aufgenommen und mit 10 Kolbenhüben bei 500 Upm homogenisiert und anschließend für 20 Minuten bei 50.000 g und 4°C zentrifugiert. Der resultierende Überstand wurde verworfen und das Sediment in einem kleinen Volumen mit 5 mmol/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wurde das Membranhomogenat mit 5 mmol/l TRIS-Acetatpuffer pH 7,0 auf eine Proteinkonzentration von 10 mg Protein/ml eingestellt und in Aliquoten bis zur Testung eingefroren.

Für den Rezeptorbindungstest wurden Aliquote aufgetaut 1:10 mit 5 mmol/l TRIS-Acetatpuffer pH 7,0 verdünnt, mit 10 Kolbenhüben bei 500 Upm mit dem Potter-Homogenisator (10 Kolbenhübe bei 500 Upm) unter Eiskühlung homogenisiert und für 60 Minuten bei 55.000 g bei 4°C zentrifugiert. Der Überstand wurde dekantiert und das Membransediment mit eiskaltem 50 mmol/l TRIS-Aoetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat wurden jeweils 100 µl je 1 ml-Ansatz im Rezeptorbindungstest eingesetzt (0,1 mg Protein/ml im Endansatz).

Im Bindungstest wurde als Puffer 50 mmol/l TRIS-Aoetatpuffer pH 7,0 sowie als radioaktiver Ligand 1 nmol/l ($^3$H)-MDL 105.519 (B.M. Baron et al. 1996) eingesetzt. Der Anteil an unspezfischer Bindung wurde in Anwesenheit von 1 mmol/l Glycin bestimmt.

In weiteren Ansätzen wurden die erfindungsgemäßen Verbindungen in Konzentrationsreihen zugegeben und die Verdrängung des radioaktiven Uganden aus seiner spezifischen Bindung an die Glycin-Bindungsstelle des NMDA-Rezeptorkanals ermittelt. Die jeweiligen Dreifachansätze wurden über 120 Minuten bei 4°C inkubiert und anschließend zur Bestimmung des an das Membranhomogenat gebundenen radioaktiven Liganden mittels Filtration durch Glasfaser-Filtermatten (GF/B) geerntet. Die auf den Glasfaser-Filtern zurückgehaltene Radioaktivität wurde nach Zugabe von Szintillator im β-Counter gemessen.

Die Affinität der erfindungsgemäßen Verbindungen zur Glycin-Bindungsstelle des NMDA-Rezeptorkanals wurde als $IC_{50}$ (Konzentration mit 50 % Verdrängung des radioaktiven Liganden aus seiner spezifischen Bindung) nach dem Massenwirkungsgesetz mittels nichtlinearer Regression berechnet und ist in Tabelle 1 nach Umrechnung (nach der Cheng-Prussoff-Beziehung) als Ki-Wert (Mittelwert von 3 unabhängigen Versuchen) angegeben oder als prozentualer Anteil des zuvor gebundenen radioaktiven Liganden s.o., der bei einer Konzentration von 10 µmol/l der zu testenden erfindungsgemäßen Substanz aus seiner spezifischen Bindung verdrängt wird.

Tabelle 1

| Beispiel | GlycinBindungsstelle des NMDA-Rezeptorkanals | |
|---|---|---|
| | Ki (µmol/l) | Verdrängung (%, 10µmol/l) |
| 2 | **0.3** | **100** |
| R **14** | - | **38** |
| R **15** | - | **75** |
| R **16** | - | **35** |
| R **17** | - | **21** |
| R **18** | - | **51** |
| R **19** | - | **26** |
| R **20** | - | **61** |
| R **21** | - | **26** |
| R **22** | - | **68** |
| R **26** | - | **43** |
| R **27** | - | **75** |
| R **28** | - | **35** |
| R **29** | - | **78** |
| R **30** | - | **32** |
| R **31** | - | **58** |
| R **32** | - | **92** |
| R **33** | - | **40** |
| R **34** | - | **32** |
| R **35** | - | **32** |
| R **36** | - | **46** |
| R **38** | - | **72** |
| R **40** | - | **70** |
| R **41** | - | **38** |
| R **42** | - | **21** |
| R **51** | - | **21** |
| R **52** | - | **22** |
| R **54** | - | **22** |
| **59** | | **14** |
| **60** | | **23** |
| **61** | | **15** |

Tabelle 1 (fortgesetzt)

| Beispiel | GlycinBindungsstelle des NMDA-Rezeptorkanals | |
|---|---|---|
| | Ki (μmol/l) | Verdrängung (%, 10μmol/l) |
| R **81** | | **66** |
| R **82** | | **96** |
| R **83** | | **93** |
| R **84** | | **79** |
| R **85** | | **61** |
| R **86** | | **33** |
| R **87** | | **37** |
| **88** | | **34** |
| R **89** | | **30** |
| R **90** | | **46** |
| R **91** | | **46** |
| R **92** | | **62** |
| R **93** | | **44** |
| R **94** | | **55** |
| R **95** | | **33** |
| R **96** | | **29** |
| R **97** | | **39** |
| R **98** | | **51** |
| R **99** | | **43** |
| **100** | | **80** |
| **101** | | **98** |
| R **102** | | **61** |
| R: REFERENZ-BEISPIEL | | |

**Beispiel 104**

NMDA/Glycin-induzierte Ionenströme an RNA-injizierten *Xenopus* Oocyten

Die Untersuchung zur Bestimmung von Funktionsänderungen des NMDA-Rezeptorkanals durch die erfindungsgemäße Verbindung der Formel I wurde an Oozyten des Südafrikanischen Krallenfrosches, *Xenopus laevis,* durchgeführt. Hierzu wurden neuronale NMDA-Rezeptorkanäle nach Injektion von RNA aus Rattenhim in Oozyten ausgebildet und durch Koapplikation von NMDA und Glycin ausgelöste Ionenströme gemessen.

*Xenopus* Oozyten der Stadien V und VI (Dumont, J.N., Journal of Morphology, Vol. 136, 1972) wurden mit Gesamt-RNA aus Himgewebe adulter Ratten mikro-injiziert (100-130 ng/Zelle) und bis zu 10 Tage in Kulturmedium (Zusammensetzung in mmol/l: 88.0 NaCl, 1.0 KCl, 1.5 $CaCl_2$, 0.8 $MgSO_4$, 2.4 $NaHCO_3$, 5 HEPES, 100 IU/ml Penicillin, 100 μg/ml Streptomycin, pH 7.4) bei 20 °C gehalten. Transmembranöse Ionenströme wurden mit Hilfe der konventionellen Zwei-Elektroden-Spannungsklemmtechnik bei einem Haltepotential von -70 mV registriert (P. Bloms-Funke P, M. Madeja, U. Mußhoff, E.-J. Speckmann, Neuroscience Letters, Vol. 205, S. 115, 1996). Zur Datenaufzeichnung und Steuerung der Versuchsapparatur wurden das OTC-Interface und die Software Cellworks verwendet (npi,FRG). Die erfindungsgemäßen Verbindungnen wurden einem nominal $Mg^{2+}$-freien Medium (Zusammensetzung in mmol/l: 89.0 NaCl, 1.0 KCl, 1.8 $CaCl_2$, 2.4 $NaHCO_3$, 5 HEPES, pH 7.4) zugesetzt und mit Hilfe einer Konzentrationsklemme systemisch appliziert (npi, FRG). Um Substanzeffekte zu testen, die über die Glycin B-Bindungsstelle des NMDA-Rezeptorkanals vermittelt sind, wurde die Glycin-Dosiswirkungskurve mit und ohne die jeweilige erfindungsgemäße Verbindung

aufgezeichnet. Dazu wurde NMDA in einer fixen Konzentration von 100 µmol/l mit Glycin in steigenden Konzentrationen (0-100 µmol/l) kumulativ koappliziert. Im Anschluß wurde das Experiment in gleicher Weise mit einer festen Konzentration der erfindungsgemäßen Verbindung wiederholt. Zur Abschätzung der Selektivität für NMDA- versus AMPA-Rezeptorkanäle wurden die Wirkungen der erfindungsgemäßen Verbindung (10 µmol/l) zusätzlich auf durch AMPA (100µmol/l) ausgelöste Ionenströme untersucht. Die Stromamplituden wurden auf die der Kontrollantwort auf Koapplikation von NMDA (100 µmol/l) mit Glycin (10 µmol/l) normiert. Die Analyse der Daten wurde mit der Software Igor-Pro (Version 3.1, WaveMetrics, USA) durchgeführt. Alle Ergebnisse wurden als Mittelwert ± Standardfehler (SEM) aus mindestens 3 Experimenten an verschiedenen Oozyten von mindestens zwei Fröschen angegeben. Die Signifikanz für ungepaarte Meßgrößen wird mit Hilfe des Mann-Whitney U-Test und für gepaarte Meßgrößen durch den Wiicoxon-Test ermittlet (Sysstat, SPSS Inc., USA). $EC_{50}$-Werte werden nach folgender Formel berechnet:

$$Y = Y_{min} + (Y_{max} - Y_{min}) / (1 + (X/EC_{50})^{-p})$$

(**$Y_{min}$** = minimaler Testwert, **$Y_{max}$** = maximaler Testwert, **Y**= relative Stromamplitude, **X** = Konzentration der Testsubstanz, **p** = Slope-Faktor). Bei Rechtsverschiebung der Glycin-Dosiswirkungskurve wurde anhand einer Schild-Regression der $pA_2$-Wert der erfindungsgemäßen Verbindung graphisch ermittelt. Konzentrationsverhältnisse wurden anhand der $EC_{50}$-Werte kalkuliert, die für jede Dosiswirkungskurve unabhängig errechnet wurden.

Für das Beispiel Nr. **2** wird die Rechtsverschiebung der Glycin-Dosiswirkungskurve in Abbildung **1** gezeigt (relative Amplitude: Stromamplitude, normiert auf die Antwort nach Gabe von NMDA/Glycin (100/10 µmol/l)). Ergebnisse ausgewählter erfindungsgemäßer Verbindungen bzgl. ihrer Wirkungen auf die Glycin-Dosiswirkungskurve sowie auf AMPA-induzierte lonenströme wurden in Tabelle 2 zusammengestellt.

Tabelle 2:

| Wirkungen der erfindungsgemäßen Verbindungen auf durch NMDA/Glycin und durch AMPA ausgelöste Ionenströme an RNA-injizierten Oozyten. | | |
|---|---|---|
| **Beisp. Nr.** | **NMDA/Glycin-Induzierte Ionenströme $pA_2$-Wert bzgl. der Glycin- Dosiswirkungskurve** | **AMPA-induzierte Ionenströme Hemmung bei 10µmol/l der erfindungsgemäßen Verbindungen** |
| 2 | 6.40 | 5.4 % (n=2) |

**Beispiel 105**

**Formalin-Test, Ratte**

Die Untersuchungen zur Bestimmung der antinociceptiven Wirkung der erfindungsgemäßen Verbindungen der Formel I wurden im Formalin-Test an männlichen Ratten (Sprague-Dawley, 150 - 170 g) durchgeführt.

Im Formalin-Test werden die erste (frühe) Phase (0 - 15 min nach Formalin-Injektion) und die zweite (späte) Phase (15 - 60 min nach Formalin-Injektion) unterschieden (D. Dubuisson, S.G. Dennis, Pain 4, 161 - 174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre, J. Katz, A.L. Vaccarino, R. Melzack, Pain, Vol. 52, S. 259, 1993).

Die erfindungsgemäßen Verbindungen wurden in der zweiten Phase des Formalin-Tests untersucht, um Aussagen über Substanzwirkungen im chronisch/entzündlichen Schmerz zu erhalten.

Durch eine einmalige subkutane Formalin-Injektion (50 µl, 5 %ig) in die dorsale Seite der rechten Hinterpfote wurde bei freibeweglichen Versuchstieren eine nociceptive Reaktion induziert, die sich in folgenden Verhaltensparametem darstellt: Heben und Halten der betroffenen Pfote (Score 1), Schütteln bzw. Zucken (Score 2), Lecken und Beißen (Score 3). Die aufgrund der Formalininjektion ausgelösten differierenden Verhaltensweisen wurden durch Beobachtung der Tiere in der späten Phase des Formalin-Tests kontinuierlich erfaßt und in einer Bewertung unterschiedlich gewichtet. Normalverhatten, bei dem das Tier alle vier Pfoten gleichmäßig belastet, wurde als Score 0 registriert. Abhängig von der Applikationsart der erfindungsgemäßen Verbindungen wurde der Applikationszeitpunkt vor der Formalin-Injektion gewählt (intraperitoneal: 15 min, intravenös: 5 min). Nach Injektion von Substanzen, die im Formalin-Test antinozizeptiv wirksam sind, sind die beschriebenen Verhaltensweisen (Score 1 - 3) der Tiere reduziert, evtl. sogar aufgehoben. Der Vergleich erfolgte mit Kontrolltieren, die Vehikel (Lösungsmittel) vor Formalinapplikation erhalten hatten. Das nozizeptive Verhalten wurde als sogenannte Schmerz-Rate (Pain-Rate, PR) berechnet. Die verschiedenen Verhaltensparameter erhielten eine unterschiedliche Gewichtung (Faktor 0, 1, 2, 3). Die Kalkulation erfolgte in Teilintervallen von 3 min nach folgender Formel:

$$PR = [(T_0 \times 0) + (T_1 \times 1) + (T_2 \times 2) + (T_3 \times 3)] / 180,$$

wobei $T_0$, $T_1$, $T_2$, und $T_3$ jeweils der Zeit in Sekunden entspricht, in der das Tier die Verhaltensweisen 0, 1, 2 oder 3 zeigte. Substanz- und Vehikelgruppen umfassen jeweils n = 10 Tiere. Basierend auf den PR-Berechnungen wurde die Substanzwirkung als Änderung gegen Kontrolle in Prozent ermittelt. Die $ED_{50}$-Berechnungen erfolgten mittels Regressionsanalyse.

Alle untersuchten erfindungsgemäßen Verbindungen zeigten eine mittelstarke bis starke Hemmung der durch Formalin induzierten Nociception.

Die Ergebnisse ausgewählter Untersuchungen im Formalin-Test Ratte sind in der nachfolgenden Tabelle zusammengefaßt.

Tabelle 3:

| Verbindung | Applikationsart | Dosierung [mg/kg] | % Hemmung der durch Formalin induzierten Nociception |
|---|---|---|---|
| **1** | i.p. | 21.5 | 88.3 |
| **2** | i.p. | 21.5 | 64.5 |
| **4** | i.v. | 21.5 | 67.1 |
| **26** R | i.p. | 21.5 | 82.6 |
| **33** R | i.p. | 21.5 | 69.0 |
| **34** R | i.p. | 21.5 | 53.1 |
| **35** R | i.p. | 21.5 | 21.9 |
| R: REFERENZ-BEISPIELE | | | |

**Beispiel 106: Parenterale Applikationsform**

38,5 g der Verbindung 2 werden in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte 1,2,3,4-Tetrahydrochinolin-2-carbonsäurederivate der allgemeinen Formel **I**

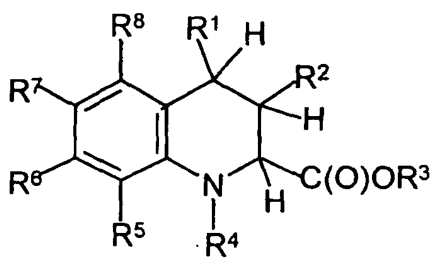

**I**

in Form ihrer Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren; **oder Salze von Basen,**

worin
**$R^1$** und **$R^2$** zusammen

$-(CH_2)_n-$ mit n= 3-10,
$-CH=CH-CH_2-$,
$-CH=CH-CH_2-CH_2-$,
$-CH_2-CH=CH-CH_2-$,
$-CH_2-CH=CH-CH_2-CH_2-$,
$-CH_2-CH_2-CH=CH-CH_2-CH_2-$,
$-O-CH_2-CH_2-$,
$-O-CH_2-CH_2-CH_2-$,
$-CH_2-O-CH_2-$,
$-CH_2-CH_2-O-CH_2-$,

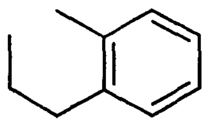

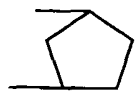

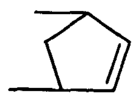

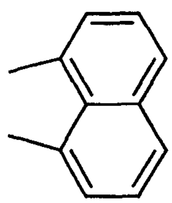

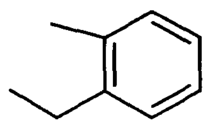

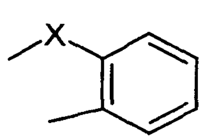

X= O, S.

bilden,
**$R^3$** ausgewählt ist aus

H; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder unge-

sättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch N, S oder O ersetzt ist; **Alkylaryl, Alkylheteroaryl, Aryl oder Heteroaryl, wobei das jeweilige Ringsystem von Alkylaryl, Alkylheteroaryl, Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist;**
**$R^4$** ausgewählt ist aus

$R^{4a}$ oder $ZR^{4a}$ mit Z = $C_1$-$C_6$-Alkyl, $C_2$-$C_6$-Alkenyl oder $C_2$-$C_6$-Alkinyl, jeweils verzweigt oder unverzweigt, einfach oder mehrfach **durch F, Cl, Br, I, $NH_2$, SH oder OH** substituiert oder unsubstituiert, mit $R^{4a}$ ausgewählt aus

H; $C_1$-$C_{12}$-Alkyl, $C_2$-$C_{12}$-Alkenyl oder $C_2$-$C_{12}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch S, O oder N ersetzt ist; **Aryl oder Heteroaryl, wobei das jeweilige Ringsystem von Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist;**

$C(O)R^9$, $C(O)OR^9$, $C(S)R^9$, $C(S)OR^9$ bzw. $S(O_2)R^9$ mit $R^9$ ausgewählt aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch S, O oder N ersetzt ist; **Alkylaryl, Alkylheteroaryl, Aryl oder Heteroaryl, wobei das jeweilige Ringsystem von Alkylaryl, Alkylheteroaryl, Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist,** insbesondere Phenethyl, 1-Adamantyl, 2-Adamantyl, 1-Naphthyl oder 2-Napthyl 2-,3- oder 4-Pyridyl; Thiazolyl;

$SR^{10}$ mit $R^{10}$ ausgewählt aus

**Aryl oder Heteroaryl, wobei das jeweilige Ringsystem von Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist;**

$C(O)NR^{11}R^{12}$, $C(O)NR^{11}NR^{12}R^{13}$, $C(NR^{11})NR^{12}R^{13}$, $C(S)NR^{11}R^{12}$ oder $C(S)NR^{11}NR^{12}R^{13}$, wobei $R^{11}$, $R^{12}$ und $R^{13}$ unabhängig voneinander ausgewählt sind aus

H; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch S, O oder N ersetzt ist, **Alkylaryl, Alkylheteroaryl, Aryl oder Heteroaryl, wobei das jeweilige Ringsystem von Alkylaryl, Alkylheteroaryl, Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist;**

**$R^5$, $R^6$, $R^7$** und **$R^8$** unabhängig voneinander ausgewählt sind aus

H, F, Cl, Br, I, CN, $NO_2$; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert;

$OR^{14}$, $OC(O)R^{14}$, $OC(S)R^{14}$, $C(O)R^{14}$, $C(O)OR^{14}$, $C(S)R^{14}$, $C(S)OR^{14}$, $SR^{14}$, $S(O)R^{14}$ bzw. $S(O_2)R^{14}$, wobei $R^{14}$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch S, O oder N ersetzt ist; **Alkylaryl, Alkylheteroaryl, Aryl oder Heteroaryl, wobei das jeweilige Ringsystem von Alkylaryl, Alkylheteroaryl, Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist;**

$NR^{15}R^{16}$, $NR^{15}C(O)R^{16}$, $C(NR^{15})NR^{16}R^{17}$ $NR^{15}C(S)R^{16}$, $C(S)NR^{15}R^{16}$ oder $C(S)NR^{15}NR^{16}R^{17}$ oder $S(O_2)$

$NR^{15}R^{16}$, wobei $R^{15}$, $R^{16}$ und $R^{17}$ unabhängig voneinander ausgewählt sind aus

H, O; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch S, O oder N ersetzt ist, **Alkylaryl, Alkylheteroaryl, Aryl oder Heteroaryl, wobei das jeweilige Ringsystem von Alkylaryl, Alkylheteroaryl, Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist;**

oder

$R^{15}$ und $R^{16}$ oder $R^{16}$ und $R^{17}$ zusammen ein $C_3$-$C_8$-Cycloalkyl, gesättigt oder ungesättigt, einfach oder mehrfach **durch F, Cl, Br, I, $NH_2$, SH oder OH** substituiert oder unsubstituiert, bilden, bzw. einen entsprechenden Heterocyclus, bei dem mindestens ein C-Atom im Ring durch S, O oder N ersetzt ist; oder

**$R^5$** und **$R^6$, $R^6$** und **$R^7$** oder **$R^7$** und **$R^8$** gemeinsam

$=CR^{18}-CH=CH-CH=$ oder $=CH-CR^{18}=CH-CH=$ bilden, mit $R^{18}$ ausgewählt aus

H, F, Cl, Br, I, OH oder $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert;

, mit der Maßgabe, daß,

wenn $R^1$ und $R^2$ zusammen $-CH=CH-CH_2-$ oder

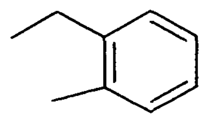

bilden und $R^3$ (-)p-Menthan-3-ol, insbesondere Menthol oder Borneol, entspricht, nicht gleichzeitig $R^7$ = Cl und $R^5$, $R^6$ und $R^8$ = H sind,

wenn $R^1$ und $R^2$ zusammen $-CH=CH-CH_2-$ bilden und $R^3$ $CH_3$ entspricht, nicht gleichzeitig $R^7$ = H, Cl oder $OCH_3$ und $R^5$, $R^6$ und $R^8$ = H sind,

wenn $R^{1b}$ und $R^{2a}$ zusammen $-CH=CH-CH_2-$ bilden und $R^3$ H entspricht, nicht gleichzeitig $R^7$ = $OCH_3$ oder $C(O)NH_2$ und $R^5$, $R^6$ und $R^8$ = H, $R^5$ und $R^7$ = $CH_3$ und $R^6$ und $R^8$ = H oder $R^5$ = $OCH_3$ und $R^6$, $R^7$ und $R^8$ = H sind,

wenn $R^{1b}$ und $R^{2a}$ zusammen

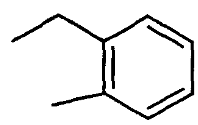

oder $-O-CH_2-CH_2-$ bilden und $R^3$ $C_2H_5$ entspricht, nicht gleichzeitig $R^7$ = H, Cl, $CH_3$, $OCH_3$ oder $NO_2$ und $R^5$, $R^6$ und $R^8$ = H oder $R^5$ = $NO_2$ und $R^6$, $R^7$ und $R^8$ = H sind, wobei

der Ausdruck "Aryl" bedeutet Phenyl, Naphthyl oder Anthracenyl,

der Ausdruck "Heteroaryl" bedeutet Thiophen, Furan, Pyrrol, Pyridin, Pyrimidin, Chinolin, Isochinolin, Phtlalazin oder Chinazolin und

der Ausdruck "Alkylaryl" bzw. "Alkylheteroaryl" bedeutet im Sinne dieser Erfindung mindestens mit $C_1$-$C_6$-Alkylen substituierte Aryle bzw. Heteroaryle, wobei die Ausdrücke Aryl, Heteroaryl und Alkyl die gleiche Bedeutung haben wie oben, in denen die Bindung über den Alkylrest erfolgt.

2. Substituierte 1,2,3,4-Tetrahydrochinolin-2-carbonsäurederivate gemäß Anspruch 1, mit der Maßgabe, daß,

wenn $R^1$ und $R^2$ zusammen $-CH=CH-CH_2-$ oder

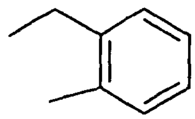

bilden und $R^3$ $C_3$-$C_8$ gesättigtem oder ungesättigtem Cycloalkyl entspricht, nicht gleichzeitig $R^7$ = Cl und $R^5$, $R^6$ und $R^8$ = H sind.

**3.** Substituierte 1,2,3,4-Tetrahydrochinolin-2-carbonsäurederivate gemäß einem der Ansprüche 1 oder 2, **dadurch gekennzeichnet, daß** $R^4$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert;

C(O)$R^9$ mit $R^9$ ausgewählt aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert; **Aryl oder Heteroaryl, wobei das jeweilige Ringsystem von Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist,** insbesondere Phenethyl, 1-Adamantyl, 2-Adamantyl, 1-Naphthyl oder 2-Napthyl 2-,3- oder 4-Pyridyl; Thiazolyl.

**4.** Substituierte 1,2,3,4-Tetrahydrochinolin-2-carbonsäurederivate gemäß einem der Ansprüche 1 bis 3, **dadurch gekennzeichnet, daß** $R^4$ ausgewählt ist aus

H; $C_1$-$C_{10}$- Alkyl, unsubstituiert oder einfach oder mehrfach **durch F, Cl, Br, I, $NH_2$, SH oder OH** substituiert; Phenyl, **wobei das Ringsystem von Phenyl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkonyl, $C_2$-$C_8$-Alkinyl, Phenyl oder Benzyl; oder Dioxolyl substituiert ist;** vorzugsweise H, $CH_3$ oder $C_2H_5$, insbesondere H.

**5.** Substituierte 1,2,3,4-Tetrahydrochinolin-2-carbonsäurederivate gemäß einem der Ansprüche 1 bis 4 **dadurch gekennzeichnet, daß** $R^3$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch N oder O ersetzt ist; **Alkylaryl, Aryl oder Heteroaryl, wobei das jeweilige Ringsystem von Alkylaryl, Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist.**

**6.** Substituierte 1,2,3,4-Tetrahydrochinolin-2-carbonsäurederivate gemäß einem der Ansprüche 1 bis 5 **dadurch gekennzeichnet, daß** $R^3$ ausgewählt ist aus

H; $C_1$-$C_4$-Alkyl, verzweigt oder unverzweigt, einfach oder mehrfach **durch F, Cl, Br, I, $NH_2$, SH oder** OH substituiert oder unsubstituiert; Phenyl, Benzyl, oder Phenethyl, **wobei das jeweilige Ringsystem von Phenyl, Benzyl oder Phenethyl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist,** vorzugsweise H, $CH_3$ oder $C_2H_5$, insbesondere H.

**7.** Substituierte 1,2,3,4-Tetrahydrochinolin-2-carbonsäurederivate gemäß einem der Ansprüche 1 bis 6, **dadurch gekennzeichnet, daß** $R^1$ und $R^2$ zusammen

$-O-CH_2-CH_2-$, $(-CH_2-)_n$ mit n=3-6, vorzugsweise 3 oder 6, $-CH=CH-CH_2-$, $-CH=CH-CH_2-CH_2-$,

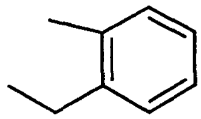 , 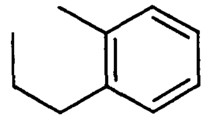 ,

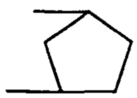 , 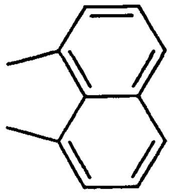 , 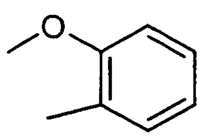 oder 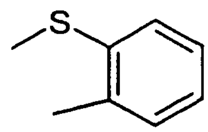 ,

vorzugsweise -CH=CH-$CH_2$- oder -CH=CH-$CH_2$-$CH_2$-, insbesondere -CH=CH-$CH_2$-, bilden.

**8.** Substituierte 1,2,3,4-Tetrahydrochinolin-2-carbonsäurederivate gemäß einem der Ansprüche 1 bis 8, **dadurch gekennzeichnet, daß** $R^5$, $R^6$, $R^7$ und $R^8$ unabhängig voneinander ausgewählt sind aus

H, F, Cl, Br, I, CN, $NO_2$; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert;

$OR^{14}$, $C(O)R^{14}$, $C(O)OR^{14}$ oder $SR^{14}$, wobei $R^{14}$ ausgewählt ist aus H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach durch F, **Cl, Br, I, $NH_2$, SH oder OH** substituiert oder unsubstituiert; $C_3$-$C_8$-Cycloalkyl, gesättigt oder ungesättigt, einfach oder mehrfach **durch F, Cl, Br, I, $NH_2$, SH oder OH** substituiert oder unsubstituiert, bzw. einem entsprechenden Heterocyclus, bei dem mindestens ein C-Atom im Ring durch S, O oder N ersetzt ist; **Alkylaryl, Alkylheteroaryl, Aryl oder Heteroaryl, wobei das jeweilige Ringsystem von Alkylaryl, Alkylheteroaryl, Aryl oder Heteroaryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist;**

$NR^{15}R^{16}$, $NR^{15}C(O)R^{16}$, wobei $R^{15}$ und $R^{16}$ unabhängig voneinander ausgewählt sind aus

H, O; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach **durch F, Cl, Br, I, $NH_2$, SH oder OH** substituiert oder unsubstituiert.

**9.** Substituierte 1,2,3,4-Tetrahydrochinolin-2-carbonsäurederivate gemäß einem der Ansprüche 1 bis 8, **dadurch gekennzeichnet, daß** $R^5$, $R^6$, $R^7$ und $R^8$ unabhängig voneinander ausgewählt sind aus

H, F, Cl, Br, I, CN, $NO_2$; $C_1$-$C_6$-Alkyl, $C_2$-$C_6$-Alkenyl oder $C_2$-$C_6$-Alkinyl, jeweils verzweigt oder unverzweigt, einfach oder mehrfach **durch F, Cl, Br, I, $NH_2$, SH oder OH** substituiert oder unsubstituiert;

$OR^{14}$, $C(O)R^{14}$, $C(O)OR^{14}$ oder $SR^{14}$, wobei $R^{14}$ ausgewählt ist aus

H; $C_1$-$C_4$-Alkyl, verzweigt oder unverzweigt, einfach oder mehrfach **durch F, Cl, Br, I, $NH_2$, SH oder OH** substituiert oder unsubstituiert; Aryl, **wobei das Ringsystem von Aryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist,**

vorzugsweise $R^5$, $R^6$, $R^7$ und $R^8$ unabhängig voneinander ausgewählt sind aus

H, F, Cl, Br, I, CN; $C_1$-$C_4$-Alkyl, verzweigt oder unverzweigt, einfach oder mehrfach **durch F, Cl, Br, I, $NH_2$, SH oder OH** substituiert oder unsubstituiert;

$OR^{14}$ oder $SR^{14}$, mit $R^{14}$ ausgewählt aus

$C_1$-$C_4$-Alkyl, verzweigt oder unverzweigt, einfach oder mehrfach **durch F, Cl, Br, I, $NH_2$, SH oder OH** substituiert oder unsubstituiert; Aryl, **wobei das jeweilige Ringsystem von Aryl unsubstituiert oder ein- oder mehrfach durch F; Cl; Br; I; $NH_2$; SH; OH; $CF_3$; =O; =S; unsubstituiertes $C_1$-$C_6$-Alkoxy; unsubstituiertes oder ein- oder mehrfach durch F, Cl, Br, I, $NH_2$, SH oder OH substituiertes $C_1$-$C_6$-Alkyl, $C_2$-$C_8$-Alkenyl, $C_2$-$C_8$-Alkinyl, Phenyl oder Benzyl; oder Dioxolyl substituiert ist,**

insbesondere $R^5$, $R^6$, $R^7$ und $R^8$ unabhängig voneinander ausgewählt sind aus

H, F, Cl, Br, I, CN; $CH_3$, $CF_3$, t-Butyl, i-Butyl, -$OCH_3$, -$OCF_3$, -$SCH_3$, -O-Phenyl.

**10.** Substituierte 1,2,3,4-Tetrahydrochinolin-2-carbonsäurederivate gemäß einem der Ansprüche 1 bis 9, **dadurch**

**gekennzeichnet, daß**
$R^5$, $R^6$ und $R^8$ H sowie $R^7$ Cl oder
$R^5$ und $R^7$ H sowie $R^6$ und $R^8$ Cl bedeuten.

**11.** Substituierte 1,2,3,4-Tetrahydrochinolin-2-carbonsäurederivate gemäß einem der Ansprüche 1 bis 9, **dadurch gekennzeichnet, daß**
$R^3$ und $R^4$ H entsprechen und
$R^5$, $R^6$ und $R^8$ H sowie $R^7$ Cl oder
$R^5$ und $R^7$ H sowie $R^6$ und $R^8$ Cl bedeuten.

**12.** Substituierte 1,2,3,4-Tetrahydrochinolin-2-carbonsäurederivate gemäß einem der Ansprüche 1 bis 11, **dadurch gekennzeichnet, daß** es sich um

- 7,9-Dichlor-3a,4,5,9b-tetrahydro-3H-cyclopenta[c]chinolin-4-carbonsäureethylester,
- 7,9-Dichlor-3a,4,5,9b-tetrahydro-3H-cyclopenta[c]chinolin-4-carbonsäure,
- 8-Chlor-3a,4,5,9b-tetrahydro-3H-cyclopenta[c]chinotin-4-carbonsäureethylester,
- 8-Chlor-3a,4,5,9b-tetrahydro-3H-cyclo-penta[c]chinolin-4-carbonsäure,
- 2-Phenoxy-5,6a,11,11a-tetrahydro-6H-inden[1,2-c]chinolin-6-carbonsäure-ethylester,
- 1,3-Dichlor-5,6,6a,7,8,12b-hexahydrobenzo[k]phenanthridin-6-carbonsäure-ethylester,
- 7,9-Dichlor-2,3,3a,4,5,9b-hexahydro-1*H*-cyclopenta[*c*]chinolin-4-carbonsäure-ethylester,
- 1,3-Dichlor-7,10-methano-5,6,6a,7,8,9,10,10a-octahydrophenanthridin-6-carbon-säureethylester,
- 5,6a,7,11b-Tetrahydro-6*H*-indeno-[2,1-*c*]chinolin-6-carbonsäureethylester
- 10,12-Dichlor-6b,7,8,12b-tetrahydro-8-azabenzo[*j*]fluoranthren-7-carbonsäureethyl-ester,
- 1,3-Dichlor-5,6,6a,11a-tetrahydro-11-oxa-5-aza-benzo[*a*]fluoren-6-carbonsäureethyl-ester,
- 1,3-Dichlor-5,6,6a,11a-tetrahydro-11-thia-5-aza-benzo[*a*]fluoren-6-carbonsäureethyl-ester,
- 6,8,9-Trichlor-2,3,3a,4,5,9b-hexahydro-furo[3,2-c]chinolin-4-carbonsäure
- 2-Trifluormethoxy-5,6,6a,7,8,9,10,11,12,12a-decahydro-5-aza-cycloocta[a]naphthalen-6-carbonsäure ethyl ester
- **2-Cyano-5,6a,7,11b-tetrahydro-6H-indeno[2,1-c]chinolin-6-carbonsäureethylester**
- **1,3-Dichlor-5,6,6a,7,8,12b-hexahydro-benzo[k]phenanthridine-6-carbonsäure**
- **1,3-Dichlor-5,6a,7,11b-tetrahydro-6H-indeno-[2,1-c]chinolin-6-carbonsäure**

vorzugsweise

- 7,9-Dichlor-3a,4,5,9b-tetrahydro-3H-cyclopenta[c]chinolin-4-carbonsäureethylester,
- 7,9-Dichlor-3a,4,5,9b-tetrahydro-3H-cyclopenta[c]chinolin-4-carbonsäure,
- 8-Chlor-3a,4,5,9b-tetrahydro-3H-cyclopenta[c]chinolin-4-carbonsäureethylester,
- 8-Chlor-3a,4,5,9b-tetrahydro-3H-cyclo-penta[c]chinolin-4-carbonsäure,

- 1,3-Dichlor-5,6,6a,7,8,12b-hexahydrobenzo[k]phenanthridin-6-carbonsäure-ethylester,
- 7,9-Dichlor-2,3,3a,4,5,9b-hexahydro-1*H*-cyclopenta[*c*]chinolin-4-carbonsäure-ethylester,
- 1,3-Dichlor-7,10-methano-5,6,6a,7,8,9,10,10a-octahydrophenanthridin-6-carbon-säureethylester,
- 5,6a,7,11b-Tetrahydro-6*H*-indeno-[2,1-*c*]chinolin-6-carbonsäureethylester
- 10,12-Dichlor-6b,7,8,12b-tetrahydro-8-azabenzo[*j*]fluoranthren-7-carbonsäureethyl-ester,
- 1,3-Dichlor-5,6,6a,11a-tetrahydro-11-oxa-5-aza-benzo[*a*]fluoren-6-carbonsäureethyl-ester,
- 1,3-Dichlor-5,6,6a,11a-tetrahydro-11-thia-5-aza-benzo[*a*]fluoren-6-carbonsäureethyl-ester,

insbesondere

- 7,9-Dichlor-3a,4,5,9b-tetrahydro-3H-cyclopenta[c]chinolin-4-carbonsäure,
- 8-Chlor-3a,4,5,9b-tetrahydro-3H-cyclo-penta[c]chinolin-4-carbonsäure,

handelt.

**13.** Substituierte 1,2,3,4-Tetrahydrochinolin-2-carbonsäurederivate gemäß einem der Ansprüche 1 bis 12 in Form ihrer Hydrochlorid-Salze.

**14.** Verfahren zur Herstellung substituierter 1,2,3,4-Tetrahydrochinolin-2-carbonsäurederivate gemäß Anspruch 1 mit $R^4$ = H, **dadurch gekennzeichnet, daß** Aniline gemäß Formel II, in denen $R^5$, $R^6$, $R^7$ und $R^8$ eine der in Anspruch 1 angegebenen Bedeutungen haben

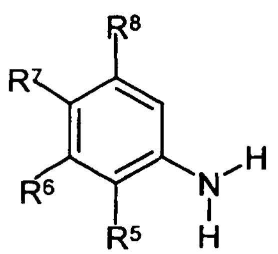
+
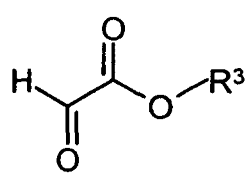
+
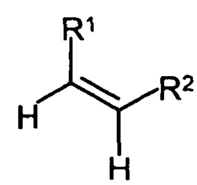

II III IV

mit Glyoxalsäureester gemäß Formel III und Olefinen gemäß IV, in denen $R^1$ und $R^2$ eine der in Anspruch 1 angegebenen Bedeutungen haben, mit Trifluoressigsäure zwischen 0°C und 100°C umgesetzt werden.

**15.** Verfahren nach Anspruch 14, **dadurch gekennzeichnet, daß** die Reaktionsdauer 0.25 - 12 Stunden, vorzugsweise maximal 2h, beträgt, die Reaktion bevorzugt bei Temperatur zwischen 20 und 40°C, vorzugsweise Raumtemperatur, erfolgt und/oder die Reaktion eine Eintopfreaktion ist.

**16.** Verfahren zur Herstellung substituierter 1,2,3,4-Tetrahydrochinolin-2-carbonsäurederivate gemäß Anspruch 1 mit $R^4 \neq$ H, **dadurch gekennzeichnet, daß** nach Ablauf der Reaktion nach Anspruch 13 das Reaktionsprodukt mit $R^4$ = H in einer Folgereaktion in bekannter Weise umgesetzt wird, so daß der Wasserstoff für $R^4$ entsprechend der übrigen Bedeutungen von $R^4$ in Anspruch 1 substituiert wird.

**17.** Verfahren gemäß einem der Ansprüche 14 bis 16, **dadurch gekennzeichnet, daß** in einem in den Verfahren eingesetzten Ausgangsprodukt mindestens eine OH-Gruppe durch eine $OSi(Ph)_2$tert-Butyl-Gruppe, mindestens

eine SH-Gruppe durch eine S-p-Methoxybenzylgruppe und/oder mindestens eine $NH_2$-Gruppe durch eine $NO_2$-Gruppe ersetzt wurde und vor der Aufreinigung des Endprodukts mindestens eine - vorzugsweise alle - OSi $(Ph)_2$tert-Butyl-Gruppe/n mit Tetrabutylammoniumflluorid in Tetrahydrofuran und/oder mindestens eine - vorzugsweise alle - p-Methoxybenzylgruppe/n mit einem Metallamid, bevorzugt Natriumamid, abgespalten und/oder mindestens eine - vorzugsweise alle - $NO_2$-Gruppe/n - vorzugsweise alle - zu $NH_2$ reduziert wird.

**18.** Verfahren nach einem der Ansprüche 14 bis 17, **dadurch gekennzeichnet, daß** vor der Aufreinigung des Endprodukts ein Verfahrensprodukt mit mindestens einer $C(O)OCH_3$- und/oder $C(S)OCH_3$-Gruppe mit KOH-Lösung bzw. NaOH-Lösung in Methanol bei 40°C - 60°C verseift wird.

**19.** Arzneimittel enthaltend als Wirkstoff mindestens ein substituiertes 1,2,3,4-Tetrahydrochinolin-2-carbonsäurederivat gemäß einem der Ansprüche 1 bis 13, in Form seiner Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren, insbesondere des Hydrochloridsalzes; oder Salze von Basen.

**20.** Arzneimittel gemäß Anspruch 19 enthaltend als Wirkstoff mindestens ein substituiertes 1,2,3,4-Tetrahydrochinolin-2-carbonsäurederivat gemäß Formel I, worin

$R^5$, $R^6$ und $R^8$ H sowie $R^7$ Cl oder

$R^5$ und $R^7$ H sowie $R^6$ und $R^8$ Cl bedeuten.

**21.** Verwendung mindestens eines substituierten 1,2,3,4-Tetrahydrochinolin-2-carbonsäurederivats gemäß einem der Ansprüche 1 bis 13 in Form seiner Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren, insbesondere des Hydrochloridsalzes; **oder Salze von Basen,** zur Herstellung eines Arzneimittels zur Behandlung von Schmerz, insbesondere von neuropathischem und/oder chronischem Schmerz und/oder zur Behandlung von Migräne.

**22.** Verwendung mindestens eines substituierten 1,2,3,4-Tetrahydrochinolin-2-carbonsäurederivats gemäß einem der Ansprüche 1 bis 13 in Form seiner Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren, insbesondere des Hydrochloridsalzes; **oder Salze von Basen,** zur Herstellung eines Arzneimittels zur Behandlung von Harninkontinenz, Juckreiz, Tinnitus aurium und/oder Diarrhoe.

**23.** Verwendung mindestens eines substituierten 1,2,3,4-Tetrahydrochinolin-2-carbonsäurederivats gemäß einem der Ansprüche 1 bis 13 in Form seiner Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren, insbesondere des Hydrochloridsalzes; **oder Salze von Basen,** zur Herstellung eines Arzneimittels zur Behandlung/Prophylaxe von/bei Epilepsie, Morbus Parkinson, Morbus Huntington, Glaukom, Osteoporose, Ototoxizität, Entzugserscheinungen bei Alkohol- und/oder Drogenmißbrauch, Schlaganfall, cerebralen Ischämien, cerebralen Infarkten, Hirnödem, Hypoxie, Anoxie und/oder zur Anxiolyse und/oder Anästhesie.

**24.** Verwendung mindestens eines substituierten 1,2,3,4-Tetrahydrochinolin-2-carbonsäurederivats gemäß einem der Ansprüche 1 bis 13 in Form seiner Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren, insbesondere des Hydrochloridsalzes; **oder Salze von Basen,** zur Herstellung eines Arzneimittels zur Behandlung/Prophylaxe von/bei Schizophrenie, Morbus Alzheimer, Psychosen bedingt durch erhöhten Aminosäurespiegel, AIDS-Demens, Encephalomyelitis, Tourette-Syndrom, perinataler Asphyxie inflammatorischen und allergischen Reaktionen, Depressionen, Drogen- und/oder Alkoholmißbrauch, Gastritis, Diabetes, cardiovaskulären Erkrankungen, Atemwegserkrankungen, Husten und/oder seelischen Erkrankungen.

## Claims

**1.** Substituted 1,2,3,4-tetrahydroquinoline-2-carboxylic acid derivatives of general formula I

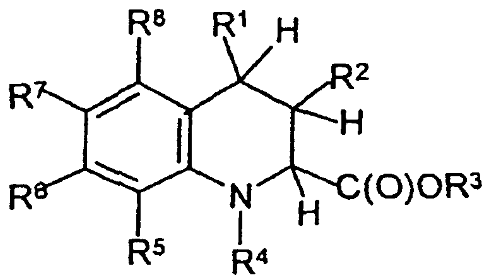

I

in the form of their racemates; enantiomers, diastereomers, in particular mixtures of their enantiomers or diastereomers or of an individual enantiomer or diastereomer; their bases and/or salts of physiologically acceptable acids; or salts of bases
wherein
**$R^1$** and **$R^2$** together form

$-(CH_2)_n-$ with n= 3 to 10,
$-CH=CH-CH_2-$,
$-CH=CH-CH_2-CH_2-$,
$-CH_2-CH=CH-CH_2-$,
$-CH_2-CH=CH-CH_2-CH_2-$,
$-CH_2-CH_2-CH=CH-CH_2-CH_2-$,
$-O-CH_2-CH_2-$,
$-O-CH_2-CH_2-CH_2-$,
$-CH_2-O-CH_2-$,
$-CH_2-CH_2-O-CH_2-$,

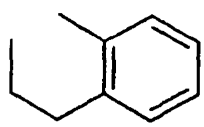

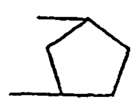

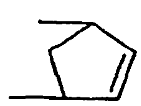

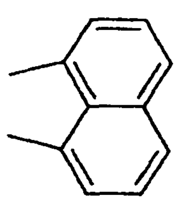

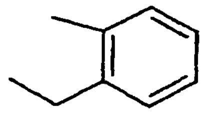

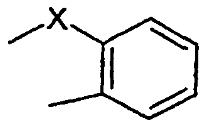

X= O, S.

X

**$R^3$** is selected from

H; respectively branched or unbranched $C_1$-$C_{18}$ alkyl, $C_2$-$C_{18}$ alkenyl or $C_2$-$C_{18}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH, or a corresponding heterocycle, in which at least one carbon atom in the ring is replaced by N, S or O; alkyl aryl, alkyl heteroaryl, aryl or heteroaryl, the respective alkyl aryl, alkyl heteroaryl, aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

**$R^4$** is selected from

$R^{4a}$ or $ZR^{4a}$ wherein Z = respectively branched or unbranched $C_1$-$C_6$ alkyl, $C_2$-$C_6$ alkenyl or $C_2$-$C_6$ alkynyl, unsubstituted or singly or multiply substituted by F, Cl, Br, I, $NH_2$, SH or OH, with $R^{4a}$ selected from

H; respectively branched or unbranched $C_1$-$C_{12}$ alkyl, $C_2$-$C_{12}$ alkenyl or $C_2$-$C_{12}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH, or a corresponding heterocycle, in which at least one carbon atom in the ring is replaced by S, O or N; aryl or heteroaryl, the respective aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

$C(O)R^9$, $C(O)OR^9$, $C(S)R^9$, $C(S)OR^9$ or $S(O_2)R^9$ with $R^9$ selected from

H; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH, or a corresponding heterocycle, in which at least one carbon atom in the ring is replaced by S, O or N; alkyl aryl, alkyl heteroaryl, aryl or heteroaryl, the respective alkyl aryl, alkyl heteroaryl, aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl, in particular phenethyl, 1-adamantyl, 2-adamantyl, 1-naphthyl or 2-naphthyl, 2-, 3- or 4-pyridyl; thiazolyl;

$SR^{10}$ with $R^{10}$ selected from

aryl or heteroaryl, the respective aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl,

$C(O)NR^{11}R^{12}$, $C(O)NR^{11}NR^{12}R^{13}$, $C(NR^{11})NR^{12}R^{13}$, $C(S)NR^{11}R^{12}$ or $C(S)NR^{11}NR^{12}R^{13}$, wherein $R^{11}$, $R^{12}$ and $R^{13}$, independently of one another, are selected from

H; respectively branched or unbranched $C_1$-$C_{18}$ alkyl, $C_2$-$C_{18}$ alkenyl or $C_2$-$C_{18}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH, or a corresponding heterocycle, in which at least one carbon atom in the ring is replaced by S, O or N, alkyl aryl, alkyl heteroaryl, aryl or heteroaryl, the respective alkyl aryl, alkyl heteroaryl, aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

**$R^5$, $R^6$, $R^7$** and **$R^8$**, independently of one another, are selected from

H, F, Cl, Br, I, CN, $NO_2$; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH;

$OR^{14}$, $OC(O)R^{14}$, $OC(S)R^{14}$, $C(O)R^{14}$, $C(O)OR^{14}$, $C(S)R^{14}$, $C(S)OR^{14}$, $SR^{14}$, $S(O)R^{14}$ or $S(O_2)R^{14}$, wherein $R^{14}$ is selected from

H; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH, or a corresponding heterocycle, in which at least one carbon atom in the ring is replaced by S, O or N; alkyl aryl, alkyl heteroaryl, aryl or heteroaryl, the respective alkyl aryl, alkyl heteroaryl, aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

$NR^{15}R^{16}$, $NR^{15}C(O)R^{16}$, $C(NR^{15})NR^{16}R^{17}$, $NR^{15}C(S)R^{16}$, $C(S)NR^{15}R^{16}$ or $C(S)NR^{15}NR^{16}R^{17}$ or $S(O_2)NR^{15}R^{16}$, wherein $R^{15}$, $R^{16}$ and $R^{17}$, independently of one another, are selected from

H, O; respectively branched or unbranched $C_1$-$C_{18}$ alkyl, $C_2$-$C_{18}$ alkenyl or $C_2$-$C_{18}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted F, Cl, Br, I, $NH_2$, SH or OH, or a corresponding heterocycle, in which at least one carbon atom in the ring is replaced by S, O or N, alkyl aryl, alkyl heteroaryl, aryl or heteroaryl, the respective alkyl aryl, alkyl heteroaryl, aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

or

$R^{15}$ and $R^{16}$ or $R^{16}$ and $R^{17}$ together form a saturated or unsaturated $C_3$-$C_8$ cycloalkyl, unsubstituted or singly or multiply substituted by F, Cl, Br, I, $NH_2$, SH or OH, or a corresponding heterocycle in which at least one carbon atom in the ring is replaced by S, O or N; or

**$R^5$** and **$R^6$, $R^6$** and **$R^7$** or **$R^7$** and **$R^8$** together form

$=CR^{18}$-CH=CH-CH= or =CH-$CR^{18}$=CH-CH=, with $R^{18}$ selected from

H, F, Cl, Br, I, OH or respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH;

provided that

if $R^1$ and $R^2$ together form -CH=CH-$CH_2$- or

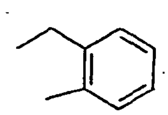

and $R^3$ corresponds to (-)p-menthan-3-ol, in particular menthol or borneol, $R^7$ = Cl and $R^5$, $R^6$ and $R^8$ = H do not simultaneously apply,

if $R^1$ and $R^2$ together form -CH=CH-$CH_2$- and $R^3$ corresponds to $CH_3$, $R^7$ = H, Cl or $OCH_3$ and $R^5$, $R^6$ and $R^8$ = H do not simultaneously apply,

if $R^{1b}$ and $R^{2a}$ together form -CH=CH-$CH_2$- and $R^3$ corresponds to H, $R^7$ = $OCH_3$ or $C(O)NH_2$ and $R^5$, $R^6$ and $R^8$ = H, $R^5$ and $R^7$ = $CH_3$ and $R^6$ and $R^8$ = H or $R^5$ = $OCH_3$ and $R^6$, $R^7$ and $R^8$ = H do not simultaneously apply,

if $R^{1b}$ and $R^{2a}$ together form

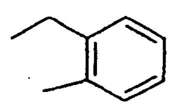

or -O-$CH_2$-$CH_2$- and $R^3$ corresponds to $C_2H_5$, $R^7$ = H, Cl, $CH_3$, $OCH_3$ or $NO_2$ and $R^5$, $R^6$ and $R^8$ = H or $R^5$ = $NO_2$ and $R^6$, $R^7$ and $R^8$ = H do not simultaneously apply, wherein the term "aryl" refers to phenyl, naphthyl or anthracenyl, the term "heteroaryl" refers to thiophene, furan, pyrrole, pyridine, pyrimidine, quinoline, isoquinoline, phtlalazine or quinazoline, and the term "alkylaryl" or "alkylheteroaryl" in the context of this invention refers to aryls or heteroaryls substituted at least with $C_1$-$C_6$ alkyls, the terms "aryl", "heteroaryl" and "alkyl" having the same meaning as above, in which the bond is produced via the alkyl radical.

2. Substituted 1,2,3,4-tetrahydroquinoline-2-carboxylic acid derivatives according to claim 1, provided that if $R^1$ and $R^2$ together form -CH=CH-$CH_2$- or

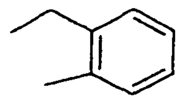

and $R^3$ corresponds to $C_3$-$C_8$ saturated or unsaturated cycloalkyl, $R^7$ = Cl and $R^5$, $R^6$ and $R^8$ = H do not simultaneously apply.

**3.** Substituted 1,2,3,4-tetrahydroquinoline-2-carboxylic acid derivatives according to either claim 1 or claim 2, **characterised in that** $R^4$ is selected from

H; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH;

C(O)$R^9$ with $R^9$ selected from

H; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH; aryl or heteroaryl, the respective aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl, in particular phenethyl, 1-adamantyl, 2-adamantyl, 1-naphthyl or 2-naphthyl 2-,3- or 4-pyridyl; thiazolyl.

**4.** Substituted 1,2,3,4-tetrahydroquinoline-2-carboxylic acid derivatives according to any one of claims 1 to 3, **characterised in that** $R^4$ is selected from

H; $C_1$-$C_{10}$ alkyl, unsubstituted or singly or multiply substituted by F, Cl, Br, I, $NH_2$, SH or OH; phenyl, the phenyl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl; preferably H, $CH_3$ or $C_2H_5$, in particular H.

**5.** Substituted 1,2,3,4-tetrahydroquinoline-2-carboxylic acid derivatives according to any one of claims 1 to 4, **characterised in that** $R^3$ is selected from

H; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH, or a corresponding heterocycle, in which at least one carbon atom in the ring is replaced by N or O; alkyl aryl, aryl or heteroaryl, the respective alkyl aryl, aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl.

**6.** Substituted 1,2,3,4-tetrahydroquinoline-2-carboxylic acid derivatives according to any one of claims 1 to 5, **characterised in that** $R^3$ is selected from

H; branched or unbranched $C_1$-$C_4$ alkyl, unsubstituted or singly or multiply substituted by F, Cl, Br, I, $NH_2$, SH or OH; phenyl, benzyl or phenethyl, the respective phenyl, benzyl or phenethyl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl; preferably H, $CH_3$ or $C_2H_5$, in particular H.

**7.** Substituted 1,2,3,4-tetrahydroquinoline-2-carboxylic acid derivatives according to any one of claims 1 to 6, **characterised in that** $R^1$ and $R^2$ together form

$-O-CH_2-CH_2-$, $(-CH_2-)_n$ with n = 3 to 6, preferably 3 or 6, $-CH=CH-CH_2-$ $-CH=CH-CH_2-CH_2-$,

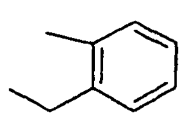 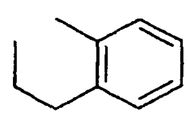 .

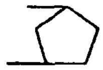, 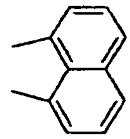, 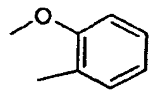 or 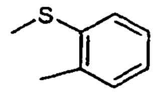.

preferably $-CH=CH-CH_2-$ or $-CH=CH-CH_2-CH_2-$, in particular $-CH=CH-CH_2-$.

**8.** Substituted 1,2,3,4-tetrahydroquinoline-2-carboxylic acid derivatives according to any one of claims 1 to 8, **characterised in that** $R^5$, $R^6$, $R^7$ and $R^8$, independently of one another, are selected from

H, F, Cl, Br, I, CN, $NO_2$; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH;

$OR^{14}$, $C(O)R^{14}$, $C(O)OR^{14}$ or $SR^{14}$, wherein $R^{14}$ is selected from H; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH; saturated or unsaturated $C_3$-$C_8$ cycloalkyl, unsubstituted or singly or multiply substituted by F, Cl, Br, I, $NH_2$, SH or OH, or a corresponding heterocycle, in which at least one carbon atom in the ring is replaced by S, O or N; alkyl aryl, alkyl heteroaryl, aryl or heteroaryl, the respective alkyl aryl, alkyl heteroaryl, aryl or heteroaryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

$NR^{15}R^{16}$, $NR^{15}C(O)R^{16}$, wherein $R^{15}$ and $R^{16}$, independently of one another, are selected from

H, O; respectively branched or unbranched $C_1$-$C_{10}$ alkyl, $C_2$-$C_{10}$ alkenyl or $C_2$-$C_{10}$ alkynyl, unsubstituted or singly or multiply substituted by F, Cl, Br, I, $NH_2$, SH or OH.

**9.** Substituted 1,2,3,4-tetrahydroquinoline-2-carboxylic acid derivatives according to any one of claims 1 to 8, **characterised in that** $R^5$, $R^6$, $R^7$ and $R^8$ independently of one another, are selected from

H, F, Cl, Br, I, CN, $NO_2$; respectively branched or unbranched $C_1$-$C_6$ alkyl, $C_2$-$C_6$ alkenyl or $C_2$-$C_6$ alkynyl, unsubstituted or singly or multiply substituted by F, Cl, Br, I, $NH_2$, SH or OH;

$OR^{14}$, $C(O)R^{14}$, $C(O)OR^{14}$ or $SR^{14}$, wherein $R^{14}$ is selected from H; branched or unbranched $C_1$-$C_4$ alkyl, unsubstituted or singly or multiply substituted by F, Cl, Br, I, $NH_2$, SH or OH; aryl, the aryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

$R^5$, $R^6$, $R^7$ and $R^8$, independently of one another, are preferably selected from

H, F, Cl, Br, I, CN; branched or unbranched $C_1$-$C_4$ alkyl, unsubstituted or singly or multiply substituted by F, Cl, Br, I, $NH_2$, SH or OH;

$OR^{14}$ or $SR^{14}$, wherein $R^{14}$ is selected from branched or unbranched $C_1$-$C_4$ alkyl, unsubstituted or singly or multiply substituted by F, Cl, Br, I, $NH_2$, SH or OH; aryl, the respective aryl ring system being unsubstituted or singly or multiply substituted by F; Cl; Br; I; $NH_2$; SH; OH; $CF_3$; =O; =S; unsubstituted $C_1$-$C_6$ alkoxy; $C_1$-$C_6$ alkyl, $C_2$-$C_8$ alkenyl, $C_2$-$C_8$ alkynyl, phenyl or benzyl, unsubstituted or singly or multiply substituted by F, Cl, Br, I, $NH_2$, SH or OH; or dioxolyl;

in particular $R^5$, $R^6$, $R^7$ and $R^8$, independently of one another, are selected from:

H, F, Cl, Br, I, CN; $CH_3$, $CF_3$, t-butyl, i-butyl, $-OCH_3$, $-OCF_3$, $-SCH_3$, -O-phenyl.

**10.** Substituted 1,2,3,4-tetrahydroquinoline-2-carboxylic acid derivatives according to any one of claims 1 to 9, **characterised in that**

$R^5$, $R^6$ and $R^8$ represent H and $R^7$ represents Cl or

$R^5$ and $R^7$ represent H and $R^6$ and $R^8$ represent Cl.

**11.** Substituted 1,2,3,4-tetrahydroquinoline-2-carboxylic acid derivatives according to any one of claims 1 to 9, **characterised in that**

$R^3$ and $R^4$ correspond to H and

$R^5$, $R^6$ and $R^8$ represent H and $R^7$ represents Cl or

$R^5$ and $R^7$ represent H and $R^6$ and $R^8$ represent Cl.

**12.** Substituted 1,2,3,4-tetrahydroquinoline-2-carboxylic acid derivatives according to any one of claims 1 to 11, **characterised in that** they are

- 7,9-dichloro-3a,4,5,9b-tetrahydro-3H-cyclopenta[c]quinoline-4-carboxylic acid ethyl ester,
- 7,9-dichloro-3a,4,5,9b-tetrahydro-3H-cyclopenta[c]quinoline-4-carboxylic acid,
- 8-chloro-3a,4,5,9b-tetrahydro-3H-cyclopenta[c]quinoline-4-carboxylic acid ethyl ester,
- 8-chloro-3a-4,5,9b-tetrahydro-3H-cyclo-penta[c]quinoline-4-carboxylic acid,
- 2-phenoxy-5,6a,11,11a-tetrahydro-6H-indene[1,2-c]quinoline-6-carboxylic acid ethyl ester,
- 1,3-dichloro-5,6,6a,7,8,12b-hexahydrobenzo[k]phenanthridine-6-carboxylic acid ethyl ester,
- 7,9-dichloro-2,3,3a,4,5,9b-hexahydro-1H-cyclopenta[c]quinoline-4-carboxylic acid ethyl ester,
- 1,3-dichloro-7,10-methano-5,6,6a,7,8,9,10,10a-octahydrophenanthridine-6-carboxylic acid ethyl ester,
- 5,6a,7,11b-tetrahydro-6H-indeno-[2,1-c]quinoline-6-carboxylic acid ethyl ester,
- 10,12-dichloro-6b,7,8,12b-tetrahydro-8-azabenzo[j]fluoranthrene-7-carboxylic acid ethyl ester,
- 1,3-dichloro-5,6,6a,11a-tetrahydro-11-oxa-5-aza-benzo[a]fluorene-6-carboxylic acid ethyl ester,
- 1,3-dichloro-5,6,6a, 11a-tetrahydro-11-thia-5-aza-benzo[a]fluorene-6-carboxylic acid ethyl ester,
- 6,8,9-trichloro-2,3,3a,4,5,9b-hexahydro-furo[3,2-c]quinoline-4-carboxylic acid
- 2-trifluoromethoxy-5,6,6a,7,8,9,10,11,12,12a-decahydro-5-aza-cycloocta[a]naphthalene-6-carboxylic acid ethyl ester
- 2-cyano-5,6a,7,11b-tetrahydro-6H-indeno[2,1-c]quinoline-6-carboxylic acid ethyl ester
- 1,3-dichloro-5,6,6a,7,8,12b-hexahydro-benzo[k]phenanthridine-6-carboxylic acid
- 1,3-dichloro-5,6a,7,11b-tetrahydro-6H-indeno-[2,1-c]quinoline-6-carboxylic acid

preferably

- 7,9-dichloro-3a,4,5,9b-tetrahydro-3H-cyclopenta[c]quinoline-4-carboxylic acid ethyl ester,
- 7,9-dichloro-3a,4,5,9b-tetrahydro-3H-cyclopenta[c]quinoline-4-carboxylic acid,
- 8-chloro-3a,4,5,9b-tetrahydro-3H-cyclopenta[c]quinoline-4-carboxylic acid ethyl ester,
- 8-chloro-3a-4,5,9b-tetrahydro-3H-cyclo-penta[c]quinoline-4-carboxylic acid,
- 1,3-dichloro-5,6,6a,7,8,12b-hexahydrobenzo[k]phenanthridine-6-carboxylic acid ethyl ester,
- 7,9-dichloro-2,3,3a,4,5,9b-hexahydro-1H-cyclopenta[c]quinoline-4-carboxylic acid ethyl ester,
- 1,3-dichloro-7,10-methano-5,6,6a,7,8,9,10,10a-octahydrophenanthridine-6-carboxylic acid ethyl ester,
- 5,6a,7,11b-tetrahydro-6H-indeno-[2,1-c]quinoline-6-carboxylic acid ethyl ester,
- 10,12-dichloro-6b,7,8,12b-tetrahydro-8-azabenzo[j]fluoranthrene-7-carboxylic acid ethyl ester,
- 1,3-dichloro-5,6,6a,11a-tetrahydro-11-oxa-5-aza-benzo[a]fluorene-6-carboxylic acid ethyl ester,
- 1,3-dichloro-5,6,6a,11a-tetrahydro-11-thia-5-aza-benzo[a]fluorene-6-carboxylic acid ethyl ester,

in particular

- 7,9-dichloro-3a,4,5,9b-tetrahydro-3H-cyclopenta[c]quinoline-4-carboxylic acid,
- 8-chloro-3a-4,5,9b-tetrahydro-3H-cyclo-penta[c]quinoline-4-carboxylic acid.

**13.** Substituted 1,2,3,4-tetrahydroquinoline-2-carboxylic acid derivatives according to any one of claims 1 to 12, in the form of the hydrochloride salts thereof.

**14.** Process for preparing substituted 1,2,3,4-tetrahydroquinoline-2-carboxylic acid derivatives according to claim 1, wherein $R^4$ = H, **characterised in that** anilines according to formula II, in which $R^5$, $R^6$, $R^7$ and $R^8$ have one of the meanings given in claim 1

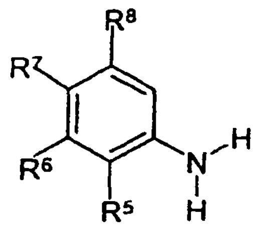

+

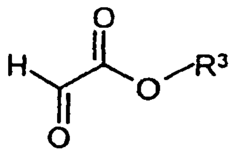

+

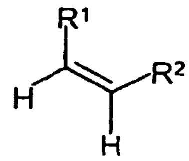

II III IV

are reacted with glyoxalic acid ester according to formula III and olefins according to IV, in which $R^1$ and $R^2$ have one of the meanings given in claim 1, with trifluoroacetic acid at between 0 and 100 °C.

**15.** Process according to claim 14, **characterised in that** the reaction period lasts 0.25 to 12 hours, preferably a maximum of 2 hours, the reaction preferably takes place at a temperature between 20 and 40 °C, preferably ambient temperature, and/or the reaction is a one-pack reaction.

**16.** Process for preparing substituted 1,2,3,4-tetrahydroquinoline-2-carboxylic acid derivatives according to claim 1, wherein $R^4 \neq H$, **characterised in that**, on completion of the reaction according to claim 13, the reaction product wherein $R^3 = H$ is reacted in a known manner in a subsequent reaction so the hydrogen is substituted for $R^4$ according to the other meanings of $R^4$ in claim 1.

**17.** Process according to any one of claims 14 to 16, **characterised in that**, in a starting product used in the process, at least one OH group has been replaced by a $OSi(Ph)_2$tert-butyl group, at least one SH group has been replaced by a S-p-methoxybenzyl group and/or at least one $NH_2$ group has been replaced by a $NO_2$ group and, prior to purification of the end product, at least one, preferably all, $OSi(Ph)_2$tert-butyl group(s) is eliminated with tetrabutyl ammonium fluoride in tetrahydrofuran and/or at least one, preferably all, p-methoxybenzyl group(s) is eliminated with a metal amide, preferably sodium amide, and/or at least one, preferably all, $NO_2$ group(s) is preferably all reduced to $NH_2$.

**18.** Process according to any one of claims 14 to 17, **characterised in that**, prior to purification of the end product, a product of the process with at least one $C(O)OCH_3$ and/or $C(S)OCH_3$ group is saponified with KOH solution or NaOH solution in methanol at 40 to 60 °C.

**19.** Pharmaceutical composition containing, as active ingredient, at least one substituted 1,2,3,4-tetrahydroquinoline-2-carboxylic acid derivative according to any one of claims 1 to 13, in the form of its racemates; enantiomers, diastereomers, in particular mixtures of their enantiomers or diastereomers or of an individual enantiomer or diastereomer; their bases and/or salts of physiologically acceptable acids, in particular the hydrochloride salt; or salts of bases.

**20.** Pharmaceutical composition according to claim 19, containing, as active ingredient, at least one substituted 1,2,3,4-tetrahydroquinoline-2-carboxylic acid derivative according to formula I, wherein
$R^5$, $R^6$ and $R^8$ represent H and $R^7$ represents Cl or
$R^5$ and $R^7$ represent H and $R^6$ and $R^8$ represent Cl.

**21.** Use of at least one substituted 1,2,3,4-tetrahydroquinoline-2-carboxylic acid derivative according to any one of claims 1 to 13, in the form of its racemates; enantiomers, diastereomers, in particular mixtures of their enantiomers or diastereomers or of an individual enantiomer or diastereomer; their bases and/or salts of physiologically acceptable acids, in particular the hydrochloride salt; or salts of bases, for preparing a pharmaceutical composition for the treatment of pain, in particular neuropathic and/or chronic pain and/or for the treatment of migraine.

**22.** Use of at least one substituted 1,2,3,4-tetrahydroquinoline-2-carboxylic acid derivative according to any one of claims 1 to 13, in the form of its racemates; enantiomers, diastereomers, in particular mixtures of their enantiomers

or diastereomers or of an individual enantiomer or diastereomer; their bases and/or salts of physiologically acceptable acids, in particular the hydrochloride salt; or salts of bases, for preparing a pharmaceutical composition for the treatment of urinary incontinence, itching, tinnitus aurium and/or diarrhoea.

**23.** Use of at least one substituted 1,2,3,4-tetrahydroquinoline-2-carboxylic acid derivative according to any one of claims 1 to 13, in the form of its racemates; enantiomers, diastereomers, in particular mixtures of their enantiomers or diastereomers or of an individual enantiomer or diastereomer; their bases and/or salts of physiologically acceptable acids, in particular the hydrochloride salt; or salts of bases, for preparing a pharmaceutical composition for the treatment/prophylaxis of/during epilepsy, Parkinson's disease, Huntington's chorea, glaucoma, osteoporosis, ototoxicity, withdrawal symptoms following alcohol and/or drug abuse, stroke, cerebral ischaemia, cerebral infarcts, cerebral oedema, hypoxia, anoxia and/or anxiolysis and/or anaesthesia.

**24.** Use of at least one substituted 1,2,3,4-tetrahydroquinoline-2-carboxylic acid derivative according to any one of claims 1 to 13, in the form of its racemates; enantiomers, diastereomers, in particular mixtures of their enantiomers or diastereomers or of an individual enantiomer or diastereomer; their bases and/or salts of physiologically acceptable acids, in particular the hydrochloride salt; or salts of bases, for preparing a pharmaceutical composition for the treatment/prophylaxis of/during schizophrenia, Alzheimer's disease, psychoses caused by a raised amino acid level, AIDS dementia, encephalomyelitis, Gilles de La Tourette's syndrome, perinatal asphyxia, inflammatory and allergic reactions, depression, drug and/or alcohol abuse, gastritis, diabetes, cardiovascular diseases, respiratory tract diseases, coughs and/or mental illnesses.

## Revendications

**1.** Dérivés substitués d'acide 1,2,3,4-tétrahydroquinoléine-2-carboxylique de formule générale I

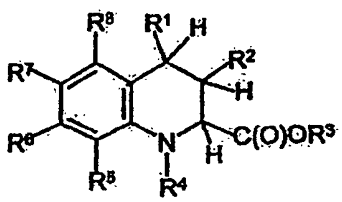

I

sous forme de leurs racémates ; de leurs énantiomères, leurs diastéréoisomères, en particulier sous forme de mélanges de leurs énantiomères ou diastéréoisomères, ou d'un énantiomère ou diastéréoisomère individuel ; de leurs bases et/ou de leurs sels d'acides acceptables du point de vue physiologique ; ou de leurs sels de bases, formule dans laquelle
$R^1$ et $R^2$ forment ensemble un groupe

$-(CH_2)_n-$ avec n = 3 à 10,
$-CH=CH-CH_2-$,
$-CH=CH-CH_2-CH_2-$,
$-CH_2-CH=CH-CH_2-$,
$-CH_2-CH=CH-CH_2-CH_2-$,
$-CH_2-CH_2-CH=CH-CH_2-CH_2-$,
$-O-CH_2-CH_2-$,
$-O-CH_2-CH_2-CH_2-$,
$-CH_2-O-CH_2-$,
$-CH_2-CH_2-O-CH_2-$,

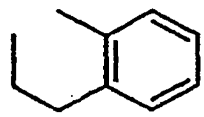

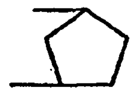

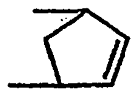

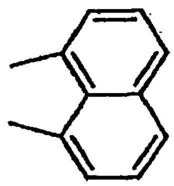

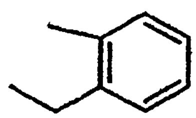

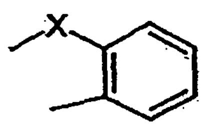

X= O, S.

$R^3$ est choisi entre

H ; un reste alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant dont au moins un atome de carbone du noyau est remplacé par N, S ou O ; un reste alkylaryle, alkylhétéroaryle, aryle ou hétéroaryle, chaque système cyclique du reste alkylaryle, alkylhétéroaryle, aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle ;

$R^4$ est choisi entre

$R^{4a}$ ou $ZR^{4a}$, avec Z = reste alkyle en $C_1$ à $C_6$, alcényle en $C_2$ à $C_6$ ou alcynyle en $C_2$ à $C_6$, chacun ramifié ou non ramifié, substitué une ou plusieurs fois par F, Cl, Br, I, $NH_2$, SH ou OH ou non substitué, $R^{4a}$ étant choisi entre

H ; un reste alkyle en $C_1$ à $C_{12}$, alcényle en $C_2$ à $C_{12}$ ou alcynyle en $C_2$ à $C_{12}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant, dont au moins un atome de carbone du noyau est remplacé par S, O ou N ; un reste aryle ou hétéroaryle, le système cyclique de chaque reste aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH

ou OH, un radical alcényle en $C_2$ à $C_8$, alcynyle en $C_2$ à $C_8$, phényle ou benzyle ; ou un radical dioxolyle ;

$C(O)R^9$, $C(O)OR^9$, $C(S)R^9$, $C(S)OR^9$ ou $S(O_2)R^9$, où $R^9$ est choisi entre

H ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant dont au moins un atome de carbone du noyau est remplacé par S, O ou N ; un reste alkylaryle, alkylhétéroaryle, aryle ou hétéroaryle, le système cyclique de chaque reste alkylaryle, alkylhétéroaryle, aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle ; en particulier phénéthyle, 1-adamantyle, 2-adamantyle, 1-naphtyle ou 2-naphtyle, 2-, 3- ou 4-pyridyle ; thiazolyle ;

$SR^{10}$, où $R^{10}$ est choisi entre

un reste aryle ou hétéroaryle, le système cyclique de chaque reste aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle ;

$C(O)NR^{11}R^{12}$, $C(O)NR^{11}NR^{12}R^{13}$, $C(NR^{11})NR^{12}R^{13}$, $C(S)NR^{11}R^{12}$ ou $C(S)NR^{11}NR^{12}R^{13}$, où $R^{11}$, $R^{12}$ et $R^{13}$ sont choisis indépendamment les uns des autres entre

H ; un reste alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant dont au moins un atome de carbone du noyau est remplacé par S, O ou N, un reste alkylaryle, alkylhétéroaryle, aryle ou hétéroaryle, le système cyclique de chaque reste alkylaryle, alkylhétéroaryle, aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle ;

$R^5$, $R^6$, $R^7$ et $R^8$ sont choisis indépendamment les uns des autres entre

H, F, Cl, Br, I, CN, $NO_2$ ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ;

$OR^{14}$, $OC(O)R^{14}$, $OC(S)R^{14}$, $C(O)R^{14}$, $C(O)OR^{14}$, $C(S)R^{14}$, $C(S)OR^{14}$, $SR^{14}$, $S(O)R^{14}$ ou $S(O_2)R^{14}$, $R^{14}$ étant choisi entre

H ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant, dont au moins un atome de carbone du noyau est remplacé par S, O ou N ; un reste alkylaryle, alkylhétéroaryle, aryle ou hétéroaryle, le système cyclique de chaque reste alkylaryle, alkylhétéroaryle, aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle ;

$NR^{15}R^{16}$, $NR^{15}C(O)R^{16}$, $C(NR^{15})NR^{16}R^{17}$, $NR^{15}C(S)R^{16}$, $C(S)NR^{15}R^{16}$ ou $C(S)NR^{15}R^{16}R^{17}$ ou $S(O_2)NR^{15}R^{16}$, $R^{15}$, $R^{16}$ et $R^{17}$ étant choisis indépendamment les uns des autres entre

H, O ; un reste alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant dont au moins un atome de carbone du noyau est remplacé par S, O ou N, un reste alkylaryle, alkylhétéroaryle, aryle ou hétéroaryle, le système cyclique de chaque reste alkylaryle, alkylhétéroaryle, aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle ;

ou bien

$R^{15}$ et $R^{16}$ ou $R^{16}$ et $R^{17}$ forment ensemble un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant dont au moins un atome de carbone du noyau est remplacé par S, O ou N ; ou bien

$R^5$ et $R^6$, $R^6$ et $R^7$ ou $R^7$ et $R^8$ forment ensemble un groupe

=$CR^{18}$-CH=CH-CH= ou =CH-$CR^{18}$=CH-CH=,

où $R^{18}$ est choisi entre

H, F, Cl, Br, I, OH ou un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ;

sous réserve que

lorsque $R^1$ et $R^2$ forment ensemble un groupe -CH=CH-$CH_2$- ou

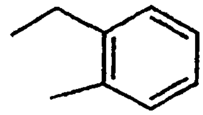

et $R^3$ est un groupe (-)p-menthane-3-ol, en particulier menthol ou bornéol, il n'y ait pas en même temps $R^7$ = Cl et $R^5$, $R^6$ et $R^8$ = H,

lorsque $R^1$ et $R^2$ forment ensemble un groupe -CH=CH-$CH_2$- et $R^3$ est un groupe $CH_3$, il n'y ait pas en même temps $R^7$ = H, Cl ou $OCH_3$ et $R^5$, $R^6$ et $R^8$ = H,

lorsque $R^{1b}$ et $R^{2a}$ forment ensemble un groupe -CH=CH-$CH_2$- et $R^3$ représente H, il n'y ait pas en même temps $R^7$ = $OCH_3$ ou C(O)$NH_2$ et $R^5$, $R^6$ et $R^8$ = H, $R^5$ et $R^7$ = $CH_3$ et $R^6$ et $R^8$ = H ou $R^5$ = $OCH_3$ et $R^6$, $R^7$ et $R^8$ = H,

lorsque $R^{1b}$ et $R^{2a}$ forment ensemble un groupe

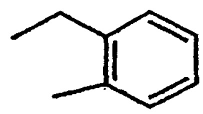

ou un groupe -O-$CH_2$-$CH_2$- et $R^3$ est un groupe $C_2H_5$, il n'y ait pas en même temps $R^7$ = H, Cl, $CH_3$, $OCH_3$ ou $NO_2$ et $R^5$, $R^6$ et $R^8$ = H ou bien $R^5$ = $NO_2$ et $R^6$, $R^7$ et $R^8$ = H,

le terme « aryle » désignant un reste phényle, naphtyle ou anthracényle,

le terme « hétéroaryle » désignant un reste thiophène, furanne, pyrrole, pyridine, pyrimidine, quinoléine, isoquinoléine, phtalazine ou quinazoline et

le terme « alkylaryle » ou « alkylhétéroaryle » désignant au sens de l'invention des restes aryle ou hétéroaryle au moins substitués avec des radicaux alkyle en $C_1$ à $C_6$, les termes aryle, hétéroaryle et alkyle ayant la même définition que ci-dessus et dans lesquels la liaison s'effectue par l'intermédiaire du reste alkyle.

**2.** Dérivés substitués d'acide 1,2,3,4-tétrahydroquinoléine-2-carboxylique suivant la revendication 1, sous réserve que

lorsque $R^1$ et $R^2$ forment ensemble un groupe -CH=CH-$CH_2$ - ou

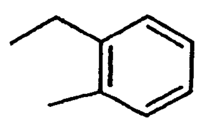

et $R^3$ est un reste cycloalkyle en $C_3$ à $C_8$ saturé ou non saturé, il n'y ait pas en même temps $R^7$ = Cl et $R^5$, $R^6$ et $R^8$ = H.

**3.** Dérivés substitués d'acide 1,2,3,4-tétrahydroquinoléine-2-carboxylique suivant l'une des revendications 1 ou 2, **caractérisés en ce que** $R^4$ est choisi entre

H ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué ;

C(O)$R^9$, où $R^9$ est choisi entre

H ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué ;

un reste aryle ou hétéroaryle, le système cyclique de chaque reste aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle, en particulier phénéthyle, 1-adamantyle, 2-adamantyle, 1-naphtyle ou 2-naphtyle, 2-, 3- ou 4-pyridyle ; thiazolyle.

**4.** Dérivés substitués d'acide 1,2,3,4-tétrahydroquinoléine-2-carboxylique suivant l'une des revendications 1 à 3, **caractérisés en ce que** $R^4$ est choisi entre

H ; un reste alkyle en $C_1$ à $C_{10}$, non substitué ou substitué une ou plusieurs fois par F, Cl, Br, I, $NH_2$, SH ou OH ; un reste phényle, le noyau du reste phényle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle, avantageusement H, $CH_3$ ou $C_2H_5$, notamment H.

**5.** Dérivés substitués d'acide 1,2,3,4-tétrahydroquinoléine-2-carboxylique suivant l'une des revendications 1 à 4, **caractérisés en ce que** $R^3$ est choisi entre

H ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ; un reste cycloalkyle en $C_3$ à $C_8$, saturé ou non saturé, substitué une ou plusieurs fois par F, Cl, Br, I, $NH_2$, SH ou OH ou non substitué, ou un hétérocycle correspondant dont au moins un atome de carbone du noyau est remplacé par N ou O ; un reste alkylaryle, aryle ou hétéroaryle, le système cyclique de chaque reste alkylaryle, aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle.

**6.** Dérivés substitués d'acide 1,2,3,4-tétrahydroquinoléine-2-carboxylique suivant l'une des revendications 1 à 5, **caractérisés en ce que** $R^3$ est choisi entre

H ; un reste alkyle en $C_1$ à $C_4$, ramifié ou non ramifié, substitué une ou plusieurs fois par F, Cl, Br, I, $NH_2$, SH ou OH ou non substitué ; un reste phényle, benzyle ou phénéthyle, le système cyclique de chaque reste phényle, benzyle ou phénéthyle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NE_2$, SH ou OH, un radical alcényle en $C_2$ à $C_8$, alcynyle en $C_2$ à $C_8$, phényle ou benzyle ; ou un radical dioxolyle, avantageusement H, $CH_3$ ou $C_2H_5$, notamment H.

**7.** Dérivés substitués d'acide 1,2,3,4-tétrahydroquinoléine-2-carboxylique suivant l'une des revendications 1 à 6, **caractérisés en ce que** $R^1$ et $R^2$ forment ensemble un groupe

$-O-CH_2-CH_2-$, $(-CH_2-)_n$ avec n = 3 à 6, avantageusement 3 ou 6, $-CH=CH-CH_2-$, $-CH=CH-CH_2-CH_2-$,

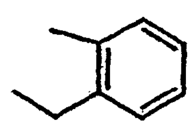
,

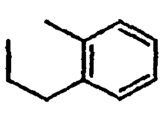
, 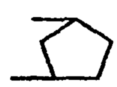
, 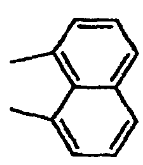
, 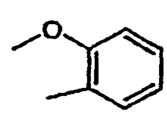
ou 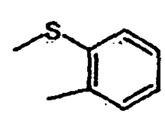
,

avantageusement $-CH=CH-CH_2-$ ou $-CH=CH-CH_2-CH_2-$, notamment $-CH=CH-CH_2-$.

**8.** Dérivés substitués d'acide 1,2,3,4-tétrahydroquinoléine-2-carboxylique suivant l'une des revendications 1 à 8, **caractérisés en ce que** $R^5$, $R^6$, $R^7$ et $R^8$ sont choisis indépendamment les uns des autres entre H, F, Cl, Br, I, CN, $NO_2$ ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ;

un groupe $OR^{14}$, $C(O)R^{14}$, $C(O)OR^{14}$ ou $SR^{14}$, $R^{14}$ étant choisi entre

H ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué ; un reste cycloalkyle en $C_3$ à Ce, saturé ou non saturé, substitué une ou plusieurs fois par F, Cl, Br, I, $NH_2$, SH ou OH ou non substitué, ou un hétérocycle correspondant, dont au moins un atome de carbone du noyau est remplacé par S, O ou N ; un reste alkylaryle, alkylhétéroaryle, aryle ou hétéroaryle, le système cyclique de chaque reste alkylaryle, alkylhétéroaryle, aryle ou hétéroaryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle ;

un groupe $NR^{15}R^{16}$, $NR^{15}C(O)R^{16}$, où $R^{15}$ et $R^{16}$ sont choisis indépendamment l'un de l'autre entre

H, O ; un reste alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH ou non substitué.

**9.** Dérivés substitués d'acide 1,2,3,4-tétrahydroquinoléine-2-carboxylique suivant l'une des revendications 1 à 8, **caractérisés en ce que** $R^5$, $R^6$, $R^7$ et $R^8$ sont choisis indépendamment les uns des autres entre

H, F, Cl, Br, I, CN, $NO_2$ ; un reste alkyle en $C_1$ à $C_6$, alcényle en $C_2$ à $C_6$ ou alcynyle en $C_2$ à $C_6$, chacun ramifié ou non ramifié, substitué une ou plusieurs fois par F, Cl, Br, I, $NH_2$, SH ou OH ou non substitué ;

un groupe $OR^{14}$, C (O)$R^{14}$, $C(O)OR^{14}$ ou $SR^{14}$, $R^{14}$ étant choisi entre

H ; un reste alkyle en $C_1$ à $C_4$, ramifié ou non ramifié, substitué une ou plusieurs fois par F, Cl, Br, I, $NH_2$, SH ou OH ou non substitué ; un reste aryle, le noyau du reste aryle étant non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle,

$R^5$, $R^6$, $R^7$ et $R^8$ étant avantageusement choisis indépendamment les uns des autres, entre

H, F, Cl, Br, I, CN ; un reste alkyle en $C_1$ à $C_4$, ramifié ou non ramifié, substitué une ou plusieurs fois par F, Cl, Br, I, $NH_2$, SH ou OH ou non substitué ;

un groupe $OR^{14}$ ou $SR^{14}$, $R^{14}$ étant choisi entre

un reste alkyle en $C_1$ à $C_4$, ramifié ou non ramifié, substitué une ou plusieurs fois par F, Cl, Br, I, $NH_2$, SH ou OH ou non substitué ; un reste aryle, le noyau du reste aryle étant dans chaque cas non substitué ou substitué une ou plusieurs fois par F ; Cl ; Br ; I ; $NH_2$ ; SH ; OH ; $CF_3$ ; =O ; =S ; un radical alkoxy en $C_1$ à $C_6$ non substitué ; un radical alkyle en $C_1$ à $C_6$ non substitué ou substitué une ou plusieurs fois par F, Cl, Br, I, $NH_2$, SH ou OH, un radical alcényle en $C_2$ à $C_8$, alcynyle en $C_2$ à $C_8$, phényle ou benzyle ; ou un radical dioxolyle,

$R^5$, $R^6$, $R^7$ et $R^8$ étant choisis notamment, indépendamment les uns des autres, entre

H, F, Cl, Br, I, CN ; $CH_3$, $CF_3$, tertiobutyle, isobutyle, $-OCH_3$, $-OCF_3$, $-SCH_3$, -O-phényle.

**10.** Dérivés substitués d'acide 1,2,3,4-tétrahydroquinoléine-2-carboxylique suivant l'une des revendications 1 à 9, **caractérisés en ce que**

$R^5$, $R^6$ et $R^8$ représentent H et $R^7$ représente Cl, ou bien

$R^5$ et $R^7$ représentent H et $R^6$ et $R^8$ représentent Cl.

**11.** Dérivés substitués d'acide 1,2,3,4-tétrahydroquinoléine-2-carboxylique suivant l'une des revendications 1 à 9, **caractérisés en ce que**

$R^3$ et $R^4$ représentent H et

$R^5$, $R^6$ et $R^8$ représentent H et $R^7$ représente Cl, ou bien

$R^5$ et $R^7$ représentent H et $R^6$ et $R^8$ représentent Cl.

**12.** Dérivés substitués d'acide 1,2,3,4-tétrahydroquinoléine-2-carboxylique suivant l'une des revendications 1 à 11, **caractérisés en ce qu'**il s'agit de :

- l'ester éthylique d'acide 7,9-dichloro-3a,4,5,9b-tétrahydro-3H-cyclopenta[c]quinoléine-4-carboxylique,
- l'acide 7,9-dichloro-3a,4,5,9b-tétrahydro-3H-cyclopenta[c]quinoléine-4-carboxylique,
- l'ester éthylique d'acide 8-chloro-3a,4,5,9b-tétrahydro-3H-cyclopenta[c]quinoléine-4-carboxylique,
- l'acide 8-chloro-3a,4,5,9b-tétrahydro-3H-cyclopenta[c]quinoléine-4-carboxylique,
- l'ester éthylique d'acide 2-phénoxy-5,6a,11,11a-tétra-hydro-6H-indène[1,2-c]quinoléine-6-carboxylique,
- l'ester éthylique d'acide 1,3-dichloro-5,6,6a,7,8,12b-hexahydrobenzo[k]phénanthridine-6-carboxylique,
- l'ester éthylique d'acide 7,9-dichloro-2,3,3a,4,5,9b-hexahydro-1*H*-cyclopenta[*c*]quinoléine-4-carboxylique,
- l'ester éthylique d'acide 1,3-dichloro-7,10-méthano-5,6,6a,7,8,9,10,10a-octahydrophénanthridine-6-carboxylique,

- l'ester éthylique d'acide 5,6a,7,11b-tétrahydro-6*H*-indéno-[2,1-*c*]quinoléine-6-carboxylique,
- l'ester éthylique d'acide 10,12-dichloro-6b,7,8,12b-tétrahydro-8-azabenzo[*j*]fluoranthrène-7-carboxylique,
- l'ester éthylique d'acide 1,3-dichloro-5,6,6a,11a-tétrahydro-11-oxa-5-azabenzo [*a*] fluorène-6-carboxylique,
- l'ester éthylique d'acide 1,3-dichloro-5,6,6a,11a-tétrahydro-11-thia-5-aza-benzo[*a*]fluorène-6-carboxylique,
- l'acide 6,8,9-trichloro-2,3,3a,4,5,9b-hexahydrofuro[3,2-c]quinoléine-4-carboxylique,
- l'ester éthylique d'acide 2-trifluorométhoxy-5,6,6a,7,8,9,10,11,12,12a-décahydro-5-aza-cycloocta-[a]naphtalène-6-carboxylique,
- l'ester éthylique d'acide 2-cyano-5,6a,7,11b-tétrahydro-6H-indéno[2,1-c]quinoléine-6-carboxylique,
- l'acide 1,3-dichloro-5,6,6a,7,8,12b-hexahydrobenzo[k]-phénanthridine-6-carboxylique,
- l'acide 1,3-dichloro-5,6a,7,11b-tétrahydro-6H-indéno-[2,1-c]quinoléine-6-carboxylique,

avantageusement

- l'ester éthylique d'acide 7,9-dichloro-3a,4,5,9b-tétrahydro-3H-cyclopenta[c]quinoléine-4-carboxylique,
- l'acide 7,9-dichloro-3a,4,5,9b-tétrahydro-3H-cyclopenta[c]quinoléine-4-carboxylique,
- l'ester éthylique d'acide 8-chloro-3a,4,5,9b-tétrahydro-3H-cyclopenta[c]quinoléine-4-carboxylique,
- l'acide 8-chloro-3a,4,5,9b-tétrahydro-3H-cyclopenta[c]quinoléine-4-carboxylique,
- l'acide 1,3-dichloro-5,6,6a,7,8,12b-hexahydrobenzo[k]phénanthridine-6-carboxylique,
- l'ester éthylique d'acide 7,9-dichloro-2,3,3a,4,5,9b-hexahydro-1*H*-cyclopenta[*c*]quinoléine-4-carboxylique,
- l'ester éthylique d'acide 1,3-dichloro-7,10-méthano-5,6,6a,7,8,9,10,10a-octahydrophénanthridine-6-carboxylique,
- l'ester éthylique d'acide 5,6a,7,11b-tétrahydro-6*H*-indéno-[2,1-*c*]quinoléine-6-carboxylique,
- l'ester éthylique d'acide 10,12-dichloro-6b,7,8,12b-tétrahydro-8-azabenzo[*j*]fluoranthrène-7-carboxylique,
- l'ester éthylique d'acide 1,3-dichloro-5,6,6a,11a-tétrahydro-11-oxa-5-azabenzo[*a*]fluorène-6-carboxylique,
- l'ester éthylique d'acide 1,3-dichloro-5,6,6a,11a-tétrahydro-11-thia-5-aza-benzo[*a*]fluorène-6-carboxylique,

notamment

- l'acide 7,9-dichloro-3a,4,5,9b-tétrahydro-3H-cyclopenta[c]quinoléine-4-carboxylique,
- l'acide 8-chloro-3a,4,5,9b-tétrahydro-3H-cyclopenta[c]quinoléine-4-carboxylique.

**13.** Dérivés substitués d'acide 1,2,3,4-tétrahydroquinoléine-2-carboxylique suivant l'une des revendications 1 à 12, sous forme de leurs chlorhydrates.

**14.** Procédé de production de dérivés substitués d'acide 1,2,3,4-tétrahydroquinoléine-2-carboxylique suivant la revendication 1, dans lesquels $R^4$ représente H, **caractérisé en ce que** l'on fait réagir des anilines de formule II, dans lesquelles $R^5$, $R^6$, $R^7$ et $R^8$ ont l'une des définitions indiquées dans la revendication 1,

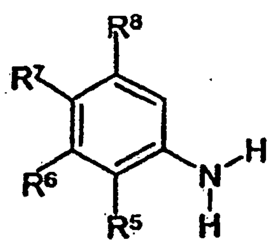

+

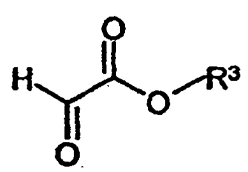

+

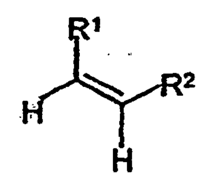

II III IV

avec un ester d'acide glyoxalique de formule III et des oléfines de formule IV, dans lesquelles $R^1$ et $R^2$ ont l'une des définitions indiquées dans la revendication 1, avec l'acide trifluoracétique entre 0 °C et 100 °C.

**15.** Procédé suivant la revendication 14, **caractérisé en ce que** la réaction a une durée de 0,25 à 12 h, avantageusement d'un maximum de 2 h, la réaction est avantageusement conduite à une température comprise entre 20 et

40 °C, avantageusement à la température ambiante, et/ou la réaction est une réaction en récipient unique.

**16.** Procédé de production de dérivés substitués d'acide 1,2,3,4-tétrahydroquinoléine-2-carboxylique suivant la revendication 1, dans lequel $R^4$ est différent de H, **caractérisé en ce que** lorsque la réaction selon la revendication 13 est terminée, le produit réactionnel dans lequel $R^4$ représente H est amené à réagir de manière connue dans une réaction ultérieure, de manière que l'hydrogène représentant $R^4$ soit substitué conformément aux autres définitions données pour $R^4$ dans la revendication 1.

**17.** Procédé suivant l'une des revendications 14 à 16, **caractérisé en ce que** dans l'un des produits de départ utilisés dans le procédé, au moins un groupe OH a été remplacé par un groupe $OSi(Ph)_2$tertiobutyle, au moins un groupe SH a été remplacé par un groupe S-p-méthoxybenzyle et/ou au moins un groupe $NH_2$ a été remplacé par un groupe $NO_2$ et avant la purification du produit final, au moins l'un, avantageusement la totalité, des groupes OSi $(Ph)_2$tertiobutyle est éliminé avec le fluorure de tétrabutylammonium dans le tétrahydrofuranne et/ou au moins l'un, avantageusement la totalité, des groupes p-méthoxybenzyle est éliminé avec un amidure métallique, avantageusement l'amidure de sodium et/ou au moins l'un, avantageusement la totalité, des groupes $NO_2$ est réduit en $NH_2$.

**18.** Procédé suivant l'une des revendications 14 à 17, **caractérisé en ce qu'**un produit du procédé portant au moins un groupe $C(O)OCH_3$- et/ou $C(S)OCH_3$- est saponifié avant la purification du produit final avec une solution de KOH ou une solution de NaOH dans le méthanol à une température de 40 °C à 60 °C.

**19.** Médicament contenant comme substance active au moins un dérivé substitué d'acide 1,2,3,4-tétrahydroquinoléine-2-carboxylique suivant l'une des revendications 1 à 13, sous forme de ses racémates ; de ses énantiomères, ses diastéréoisomères, en particulier des mélanges de ses énantiomères ou diastéréoisomères, ou sous forme d'un énantiomère ou d'un diastéréoisomère individuel ; de ses bases et/ou de ses sels d'acides acceptables du point de vue physiologique, en particulier sous forme du chlorhydrate ; ou de sels de bases.

**20.** Médicament suivant la revendication 19, contenant comme substance active au moins un dérivé substitué d'acide 1,2,3,4-tétrahydroquinoléine-2-carboxylique de formule I dans laquelle

$R^5$, $R^6$ et $R^8$ représentent H et $R^7$ représente Cl, ou bien

$R^5$ et $R^7$ représentent H et $R^6$ et $R^8$ représentent Cl.

**21.** Utilisation d'au moins un dérivé substitué d'acide 1,2,3,4-tétrahydroquinoléine-2-carboxylique suivant l'une des revendications 1 à 13, sous forme de ses racémates ; de ses énantiomères, diastéréoisomères, en particulier sous forme de mélanges de ses énantiomères ou diastéréoisomères, ou d'un énantiomère ou diastéréoisomère individuel ; de ses bases et/ou de ses sels d'acides acceptables du point de vue physiologique, en particulier du chlorhydrate ; ou de sels de bases, pour la préparation d'un médicament destiné au traitement de la douleur, en particulier d'une douleur neuropathique et/ou chronique et/ou pour le traitement de la migraine.

**22.** Utilisation d'au moins un dérivé substitué d'acide 1,2,3,4-tétrahydroquinoléine-2-carboxylique suivant l'une des revendications 1 à 13, sous forme de ses racémates ; de ses énantiomères, diastéréoisomères, en particulier sous forme de mélanges de ses énantiomères ou diastéréoisomères, ou d'un énantiomère ou diastéréoisomère individuel ; sous forme de ses bases et/ou de ses sels d'acides acceptables du point de vue physiologique, en particulier du chlorhydrate ; ou de ses sels de bases, pour la préparation d'un médicament destiné au traitement de l'incontinence d'urine, du prurit, des acouphènes et/ou de la diarrhée.

**23.** Utilisation d'au moins un dérivé substitué d'acide 1,2,3,4-tétrahydroquinoléine-2-carboxylique suivant l'une des revendications 1 à 13, sous forme de ses racémates ; de ses énantiomères, diastéréoisomères, en particulier de mélanges de ses énantiomères ou diastéréoisomères, ou sous forme d'un énantiomère ou d'un diastéréoisomère individuel ; sous forme de ses bases et/ou de ses sels d'acides acceptables du point de vue physiologique, en particulier du chlorhydrate ; ou sous forme de sels de bases, pour la préparation d'un médicament destiné au traitement/à la prophylaxie de l'épilepsie, de la maladie de Parkinson, de la maladie de Huntington, du glaucome, de l'ostéoporose, de la neurotoxicité du nerf auditif, de manifestations de privation en cas d'abus d'alcool et/ou de drogue, de l'apoplexie cérébrale, d'ischémies cérébrales, d'infarctus cérébraux, de l'oedème cérébral, de l'hypoxie, de l'anoxie et/ou pour l'anxiolyse et/ou pour l'anesthésie.

**24.** Utilisation d'au moins un dérivé substitué d'acide 1,2,3,4-tétrahydroquinoléine-2-carboxylique suivant l'une des revendications 1 à 13, sous forme de ses racémates ; de ses énantiomères, diastéréoisomères, en particulier de

mélanges de ses énantiomères ou diastéréoisomères, ou sous forme d'un énantiomère ou d'un diastéréoisomère individuel ; sous forme de ses bases et/ou de ses sels d'acides acceptables du point de vue physiologique, en particulier du chlorhydrate ; ou de ses sels de bases, pour la préparation d'un médicament destiné au traitement/à la prophylaxie de la schizophrénie, de la maladie d'Alzheimer, de psychoses dues à des taux élevés d'aminoacides, de la démence liée au SIDA, de l'encéphalomyélite, du syndrome de Tourette, de l'asphyxie périnatale, de réactions inflammatoires et allergiques, de dépressions, d'abus de drogue et/ou d'alcool, de la gastrite, du diabète, de maladies cardiovasculaires, de maladies des voies respiratoires, de la toux et/ou de maladies psychiques.

Fig. 1)

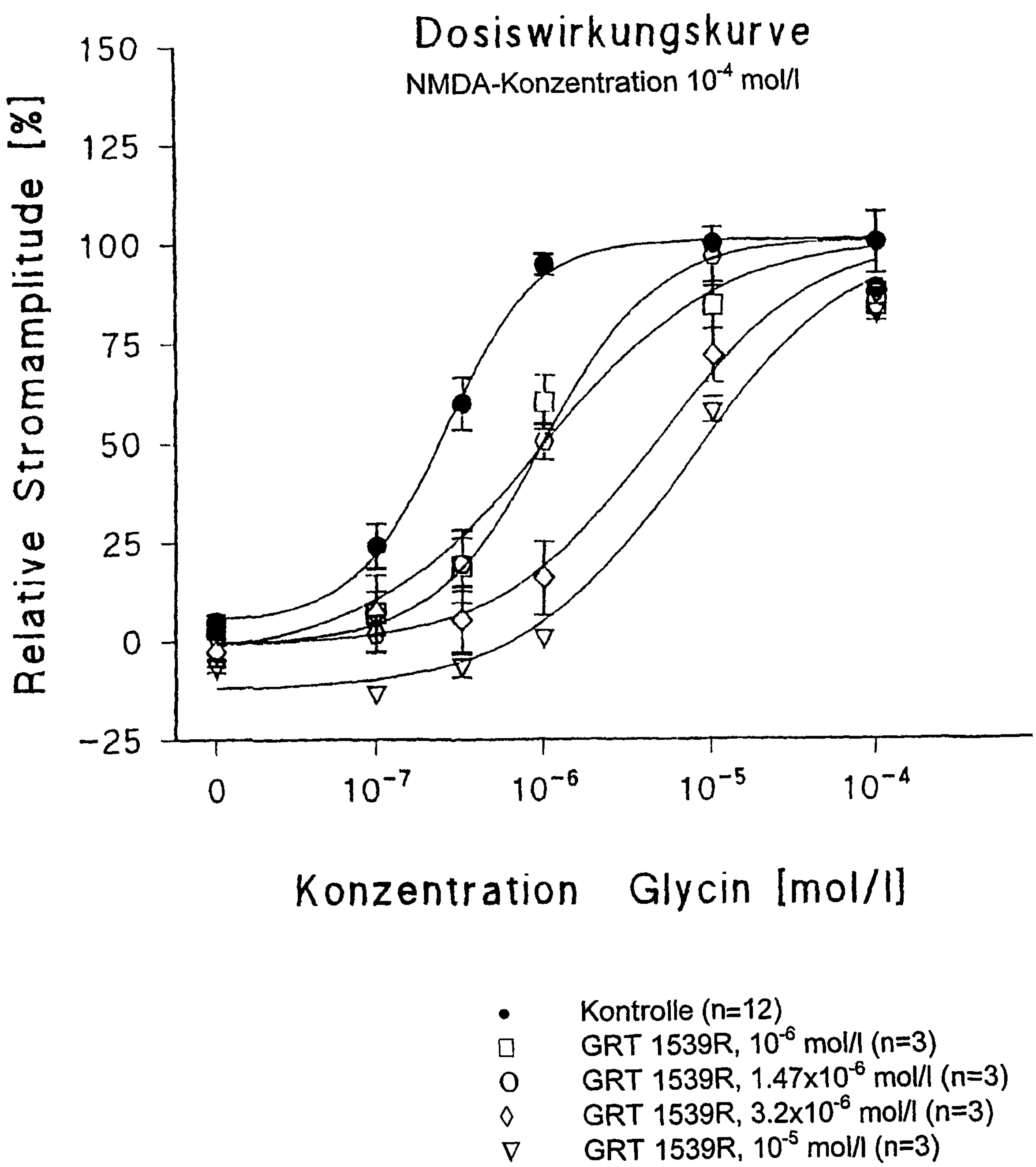


ERSATZBLATT (REGEL 26)